(19) Europäisches Patentamt
European Patent Office
Office européen des brevets

(11) EP 2 746 396 A1

(12) **EUROPEAN PATENT APPLICATION**

(43) Date of publication:
**25.06.2014 Bulletin 2014/26**

(21) Application number: **12075141.7**

(22) Date of filing: **20.12.2012**

(51) Int Cl.:
***C12N 15/113*** *(2010.01)*
***A61K 39/35*** *(2006.01)*
***A61P 37/00*** *(2006.01)*
***A61K 31/7125*** *(2006.01)*
***A61K 9/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **PLS-Design GmbH
20255 Hamburg (DE)**
- **Klinikum rechts der Isar der Technischen Universität München
81675 München (DE)**
- **Helmholtz Zentrum München Forschungszentrum für Gesundheit und Umwelt GmbH
85764 Neuherberg (DE)**

(72) Inventors:
- **Bredehorst, Reinhard
20255 Hamburg (DE)**
- **Grunwald, Thomas
22459 Hamburg (DE)**
- **Ollert, Markus
82131 Gauting (DE)**
- **Schmidt-Weber, Carsten
81249 München (DE)**
- **Spillner, Edzard
22767 Hamburg (DE)**

(74) Representative: **Jungblut, Bernhard Jakob et al
Jungblut & Seuss
Patentanwälte
Max-Dohrn-Strasse 10
10589 Berlin (DE)**

(54) **Selective local inhibition of TNFR1-mediated functions at the site of antigen/allergen presentation**

(57) The invention relates to a pharmaceutical composition for the modulation of T cell and B cell responses made of one or more prepatations and comprising a therapeutically effective dose of at least one inhibitor of TNFR1-mediated functions and of at least one antigen or allergen.

EP 2 746 396 A1

## Description

## INTRODUCTION

Allergen- or antigen-specific immunotherapy offers the promise of restoring lasting immunological tolerance to allergens or autoantigens and is associated with the re-induction of allergen- or antigen specific Treg cells. For the treatment of allergy, specific immunotherapy is efficient when patients are mono-sensitized against seasonal allergens, but can be less or not efficient if the patient is multi-sensitized or atopic or if the patient reacts to perennial allergens. Therefore, there is a need to design more effective allergen-tolerogenic therapies. For the treatment of asthma and autoimmune diseases including type I diabetes, rheumatoid arthritis, and multiple sclerosis, specific immunotherapy has shown some efficacy, but currently used treatment protocols need to be combined with immune modulatory techniques to restore lasting clinical tolerance.

*Regulatory T cells (Tregs).*

The activation, proliferation and effector functions of a large spectrum of immunocompetent cells such as CD4+ cells, CD8+ cells, NK cells, NKT cells, dendritic cells, macrophages, and B cells are susceptible to suppression mediated by regulatrory T cells (Tregs). The induction of Treg-suppressive activity is specific and requires antigenic or allergenic stimulation through the T cell receptor (TCR). The suppressive activity of Tregs, however, is not antigen/allergen-specific. Therefore, a wide range of immune responses can be inhibited by Tregs via 'bystander' suppression. The exact mechanisms of Treg-mediated suppression remain to be elucidated, but cell-to-cell contacts and several molecules such as IL-10, TGF-beta, CTLA-4 (cytotoxic T lymphocyte antigen 4; CD152) and granzyme/perforin are reported to contribute to the suppressive activity of Tregs (for a review, see Chen and Oppenheim, 2010).

There are two Treg populations, the naturally occurring Tregs (nTregs) and the induced or adaptive Tregs (iTregs). nTregs develop in the thymus and are exported to the periphery, whereas iTregs are converted from naive CD4+ T cells by TGF-beta in conjunction with TCR stimulation in the periphery. Tregs are preferentially self-reactive since their TCRs have higher affinity for self-antigens and are similar to the TCR of self-reactive pathogenic effector T cells (Teffs). However, Tregs are also capable of attenuating host defense responses against pathogens and foreign antigens.

One population of Tregs are CD4+ T cells expressing CD25 and the transcription factor FoxP3 (forkhead box P3), a master regulator of Treg development and function. The characteristic phenotype of these Tregs is regulated by FoxP3 and includes high expression of CD25 (alpha-chain of the IL-2 receptor), CTLA-4, GITR (glucocorticoid-induced TNFR family related protein, TNFRSF 18) and low expression of CD127 (alpha-chain of the IL-7 receptor). However, highly suppressive CD4+FoxP3+ Tregs which do not express CD25 also exist and are increased at inflammatory sites (Chen et al., 2010). CD4+FoxP3+ Tregs comprise approximately 10% of peripheral CD4+ cells and play a crucial role in maintaining immune system homeostasis, preventing autoimmunity, and limiting chronic inflammation.

In addition to FoxP3+ Tregs, there are other types of Tregs that can be induced from naive CD4+ cells in the periphery including IL-10- and TGF-beta-producing Tr1 cells and TGF-beta-producing Th3 cells, or other possible overlapping cell types. These FoxP3-non-expressing T cells also play significant parts in the maintenance of peripheral tolerance and immune suppression. For example, Tr1 cells specific for common environmental allergens represent the dominant subset in individuals such as bee keepers which developed tolerance after exposure to relatively high doses of allergen. Increased numbers of local Tr1 and FoxP3+ cells have been associated with elevated levels of serum IgG4 capable of blocking allergen presentation by IgE (for a review, see Akdis and Akdis, 2011). Furthermore, specific allergen immunotherapy (SIT) is associated with the induction of both Th1 and Tr1 responses (for a review, see Larche, 2006). Following SIT, IL-10 production has been reported in T cells, B cells, and monocytes/macrophages. Thus, SIT appears to modify both antigen-presenting cell and T cell responses through the induction of IL-10 and, in some reports, TGF-beta. Peripheral blood T cells isolated after SIT were able to suppress allergen-specific proliferation of pretreatment T cells in an IL-10-dependent and TGF-beta-dependent manner since neutralizing antibodies to these cytokines abrogated suppression (for a review, see Larche et al., 2006).

*Role of Tregs in allergic and autoimmune diseases.*

It is well established that the breakdown of immune tolerance maintained by Tregs can cause organ-specific autoimmune responses. Therefore, either low Treg numbers with normal function or normal Treg numbers with compromised suppressive function have been made responsible for allergic and autoimmune diseases. Clinical data, however, provide a more complex scenario. For example, the frequency of Tregs in rheumatoid arthritis (RA) with an activated phenotype and enhanced suppressive potential in the synovial fluid of inflamed joints is increased in comparison to the periphery. Similarly, activated Tregs also accumulate in the inflamed joint of other arthropathies such as juvenile idiopathic arthritis and spondyloarthropathies. Apparently, Tregs are often increased at the site of autoimmune inflammation, presumably resulting from active recruitment and in situ proliferative expansion. Detailed analyses have demonstrated that peripheral blood CD4+CD25+ T cells of RA patients are fully competent in inhibiting the proliferation of responder CD4+CD25- T cells but they are unable to suppress the production of proinflammatory cytokines by activated T cells and monocytes (Ehrenstein et al., 2004). This failure of Tregs to inhibit cytokine production could be restored by anti-TNF therapy (Ehrenstein et al., 2004). However, subsequent analyses demonstrated that in RA patients anti-TNF therapy induced Tr1 and/or Th3 suppressor cells, but did not expand or restore the function of pre-existing nTregs (Nadkarni et al., 2007). As summarized in a recent review, current published evidence does not lend support to the notion that anti-TNF therapy can simply restore or promote Treg activity in RA patients (Chen and Oppenheim, 2010). Apparently, therapeutic approaches need to be developed capable of inducing sustainable expansion of pre-existing Tregs and counter potentially insufficient drainage from tissue or retainment and enhancing their suppressive effect on effector T cells.

In contrast to the situation in RA patients, the main principle of specific immunotherapy in atopic patients is to increase the number of Tregs capable of controlling allergen-specific effector T cells. Based on current knowledge, allergen tolerance is mediated by peripherally induced regulatory T cells as evidenced by a deficit of allergen-specific IL-10 producing T cells in the peripheral blood of allergic patients (for reviews, see Schmidt-Weber et al., 2005; Schmidt-Weber et al., 2006).

In patients with allergic asthma the situation is more complicated. Several studies have shown that the number and function of Tregs in patients with allergic asthma is impaired or altered compared with healthy individuals (for a review, see Thorburn and Hansbro, 2010). However, analysis of the number of Tregs in patients with allergic asthma revealed variability between children and adults and between peripheral blood and airway tissue (for a review, see Langier et al., 2012). In paediatric patients with allergic asthma, fewer CD4+CD25+ T cells were found in blood and broncheoalveloar lavage (BAL) samples than in healthy control subjects (Lee et al., 2007; Hartl et al., 2007), whereas in the blood of paediatric patients with persistent severe allergic asthma higher numbers of CD4+CD25+ T cells were observed (Lee et al., 2007). In the blood of adult patients with moderate to severe allergic asthma low numbers of Tregs were reported, but patients with mild disease had higher numbers of Tregs than the other two groups (Abdulamir et al., 2009). In contrast, in the BAL of adult patients with moderate to severe allergic asthma an increase in the number of CD4+FoxP3+ Tregs was observed as compared to patients with mild disease and to healthy controls (Smyth et al., 2010). The interpretation of these data, however, requires caution since Tregs characterized by CD4 and CD25 positivity alone in some of the studies may also represent activated effector T cells. Furthermore, assessment of Treg numbers in blood may not represent Treg numbers that have migrated to the site of inflammation. Despite these controversial observations, however, there is convincing evidence that supports the requirement for Tregs in the control of asthma. For example, glucocorticoid treatment of asthma is effective in suppressing inflammation and symptoms. These therapeutics induce a short-term up-regulation of FoxP3 expression and Tregs in patients with asthma (Karagiannidis et al., 2004). Further support is provided by mouse models of allergic airways disease. Adoptive transfer of CD4+CD25+ Tregs into sensitized mice before antigen challenge has been demonstrated to suppress the development of allergic disease (Kearley et al., 2005; Strickland et al., 2006). Furthermore, adoptive transfer of Tregs after onset of the disease attenuates established inflammation (Kearley et al., 2008).

There is also evidence that human patients with diabetes type 1 or multiple sclerosis have a reduced frequency or functionality of CD4+ CD25+ regulatory T cells (Kukreja et al., 2002; Viglietta et al., 2004). The necessity for increasing the number of CD4+ $CD25^-$ regulatory T cells in patients with type I diabetes is supported by the observation that several therapeutic approaches which protect from diabetes, are correlated with an increase in CD4+ CD25+ regulatory T cells (for a review, see Juedes and von Herrath, 2004). For example, increased numbers of circulating CD4+ CD25+ regulatory T cells have been reported in patients receiving successful islet transplants and were treated with the anti-CD3 mAb hOKT3$\gamma$1(Ala-Ala) (Hering et al., 2004).

Taken together, the role of Tregs in allergic and autoimmune diseases indicates that control of the development, survival, and function of Tregs is instrumental for effective control of immune responses. Over the years, several new therapeutic approaches for modulating Treg activity in allergic and autoimmune diseases have been identified, among which are approaches targeting members of the tumor necrosis factor receptor (TNFR) superfamily and their respective ligands including TNF-alpha.

*TNF-alpha and TNFR2-mediatiated functions.*

TNF-alpha is a pleiotropic cytokine with biphasic pro-inflammatory and anti-inflammatory effects. Upon activation of pro-inflammatory signals, TNF-alpha is produced by macrophages, dendritic cells (DCs), B cells, T cells, and by other types of somatic cells such as endothelial cells, mast cells, and neuronal tissue cells. TNF-alpha is first produced as a transmembrane protein (mTNF-alpha) that can subsequently be cleaved by the TNF-alpha converting enzyme (TACE, which releases a soluble form (sTNF-alpha). Both mTNF-alpha and sTNF-alpha are biologically active.

TNF-alpha acts by binding to two receptors, TNFR1 (CD120a, or p55) and TNFR2 (CD120b, or p75). TNFR1 is ubiquitously expressed by a broad array of cell types, contains a death domain in its intracellular portion and can induce apoptosis via a caspase 3-dependent pathway. TNFR2 does not contain a death domain and binds preferentially to mTNF-alpha. Induction of the suppressive effect of human antigen-specific regulatory T cells by tolerogenic dendritic cells has been demonstrated to involve mTNF-alpha but not sTNF-alpha (Kleijwegt et al., 2010). In this study, anti-TNF-alpha antibodies (blocking both mTNF-alpha and sTNF-alpha) prevented Tregs from acquiring a suppressive effect on the proliferation of effector T cells (Teff), whereas soluble TNFR2 (blocking only sTNF-alpha) had no effect on the induction of a suppressive effect.

TNFR2 is expressed by cells of the immune system, predominantly on regulatory T cells (Tregs). All human thymic CD4+CD25+ Tregs and immunosuppressive human CD4-CD8+CD25+ T cells constitutively express TNFR2, while thymic CD4+CD25-cells do not express this receptor. In human peripheral blood cells, CD4+CD25+ T cells express the highest level of TNFR2. Only a fraction of CD4+CD25- T cells (20-30%) express TNFR2 at a low level. After TCR stimulation, surface expression of TNFR2 is up-regulated on both CD4+CD25+ and CD4+CD25- T cells, but activated CD4+CD25+ T cells still express considerably higher levels of TNFR2. TNFR1 was not detected by FACS on either CD4+CD25+ or CD4+CD25- T cells (for a review, see Chen and Oppenheim, 2010).

In the presence of interleukin 2 (IL-2), TNF-alpha-mediated activation of Tregs results in proliferation, up-regulation of FoxP3 expression and increase of their suppressive activity (for reviews, see Chen and Oppenheim, 2010; Biton et al., 2012). TNF-alpha has been shown to selectively up-regulate CD25 (alpha-chain of the IL-2 receptor) expression on Tregs (Chen et al., 2007), while IL-2 preferentially up-regulates TNFR2 expression on Tregs (Chen and Oppenheim, 2010). Thereby, TNF-alpha and IL-2 generate a powerful mechanism for receptor amplification and synergistically up-regulate Treg suppressive activity. Tregs are also capable to shed TNFR2, resulting in neutralization of sTNF-alpha (van Mierlo et al., 2008). Furthermore, in concert with a common gamma-chain cytokine (IL-2, IL-7, or IL-15) TNF-alpha also up-regulates the expression of other co-stimulatory members of the TNFR superfamily on Tregs including 4-1BB (CD137; TNFRSF9) and OX40 (CD134; TNFRSF4) (Hamano et al., 2011). Thus, TNF-alpha enhances multiple TNFRSF pathways by up-regulating a number of receptors that can cooperate to limit excessive inflammation and prevent self-destructive tissue damage.

A very recent study showed different effects of TNF-alpha on nTregs and iTregs. According to this study, nTregs may require TNF-alpha to function optimally, whereas the effects of iTregs may also occur independently of TNF-alpha, since TNFR2-negative iTregs were also able to control the development of colitis in a murine model (Housley et al., 2011).

*Selective inhibition of TNFR1.*

The TNF signaling pathway represents an important target in the therapy of autoimmune diseases, and anti-TNF therapeutics are used successfully for the treatment of diseases such as rheumatoid arthritis. By their ability to interfere with inflammatory processes at multiple levels, anti-TNF therapeutics are invaluable tools for inhibition of inflammation-induced tissue damage. However, this therapy has also serious drawbacks including increased incidence of infection, malignancy, and the onset of new autoimmune diseases such as lupus (for a review, see Chen and Oppenheim, 2010). In some cases anti-TNF therapy may also promote inflammation, contradictory to its well-known anti-inflammatory action. For example, neutralization of TNF in the heart of healthy baboon resulted in myocarditis (McTiernan et al., 2007), which may be caused by elimination of Treg activity via TNF-TNFR2 interactions. Since most of these adverse effects appear to be a result of unwanted abrogation of beneficial TNF signaling, there is a need for more specific targeting of pathological TNF-induced signaling in order to increase efficacy and safety of anti-TNF therapy.

Promising approaches for specific targeting of pathological TNF-induced signaling include a) specific inhibition of sTNF so that the mTNF-TNFR1 and mTNF-TNFR2 interactions remain intact, and b) specific inhibition of TNFR1 while leaving TNFR2 signaling intact (for a review, see Van Hauwermeiren et al., 2011). For the first approach the dominant-negative sTNF mutant XPro 1595 has been developed (Steed et al., 2003). TNFR1-sTNF interactions are effectively inhibited by this sTNF mutant, but potentially pathological mTNF-induced inflammatory processes via TNFR1 are still possible. For specific inhibition of TNFR1 a variety of compounds have been developed including monoclonal antibodies (Zettlitz et al., 2010) and antagonistic TNF mutants with specificity for TNFR1 (Shibata et al., 2009). However, due to their effective inhibition of sTNF-TNFR1 and mTNF-TNFR1 interactions, these therapeutics are associated with an increased risk for infections.

In addition to these inhibitory strategies, RNAi and antisense oligonucleotides have been used successfully to downregulate TNFR1 in mouse models. The study of Huang and coworkers (Huang et al., 2006) demonstrates that repeated intraperitoneal injections of 2'-O-(2-methoxy)ethyl-modified antisense oligonucleotides specific for TNFR1 are capable of protecting the liver of mice during radiotherapy for intrahepatic cancer. However, the limited and varying cellular uptake of injected oligonucleotides is a serious problem which still needs to be solved. Furthermore, systemic application of antisense oligonucleotides with specificity for TNFR1 is likely to induce an increased risk for infections. In another study cell-specific RNA interference (RNAi) based on adenoviral delivery of a short hairpin RNA-expressing construct, has been used for local silencing of TNFR1 (Arntz et al., 2010). Intra-articular delivery of this adenoviral vector into mice suffering from collagen-induced arthritis effected TNFR1 silencing in synovial lining cells and reduction of macroscopic arthritis severity. While this approach provides a promising treatment modality, long-term safety aspects of adenoviral vector systems for human applications need to be analyzed more in detail.

Taken together, several therapeutics and developmental drugs are available for selective inhibition of TNFR1, but there is a need in the field to minimize potential adverse side effects of these molecules including the risk for infections. Furthermore, there is a need for methods capable of maintaining high local concentrations of TNFR1 inhibitors at the site of antigen or allergen presentation over a prolonged period of time. High local TNFR1 inhibitor concentrations are required to effectively inhibit inflammatory processes at the site of antigen or allergen presentation and, thereby, to favor TNFR2-mediated activation of Tregs. High local TNFR1 inhibitor concentrations over a prolonged period of time are necessary, since the induction of Treg-suppressive activity most likely requires antigenic or allergenic stimulation through the T cell receptor (TCR) for at least 1-2 days. Differentiation of T cells and thus the development of immunologic memory has been reported to require the engagement of the T cell receptor (TCR) over 12-48 hours and long lasting memory may even require repetitive exposure. Accordingly, inhibition of TNFR1-mediated effects needs to be in effect at least for the same period of time, most likely as long as antigens or allergens are released from the injected antigen/allergen-adjuvant complex.

The present invention solves these problems by disclosing methods for sustained local delivery of high concentrations of TNFR1 inhibitors over a prolonged period of time at the site of antigen or allergen presentation. Due to the locally restricted delivery of TNFR1 inhibitors, potential adverse side effects induced by systemic application of TNFR1 inhibitors are minimized.

**SUMMARY OF THE INVENTION**

Control of the development, survival, and function of regulatory T cells (Tregs) is essential for therapeutic treatment of allergic and autoimmune diseases. Successful therapeutic approaches for modulating Treg activity include allergen- or antigen-specific immunotherapy and targeting of members of the tumor necrosis factor receptor (TNFR) superfamily and their respective ligands including TNF-alpha. With a few exceptions, however, the therapeutic efficacy of current protocols for allergen- or antigen-specific immunotherapy need to be optimized, and currently applied anti-TNF therapeutics are associated with potentially serious side effects including increased incidence of infections, malignancy, and the onset of new autoimmune diseases.

In the present invention methods are disclosed for allergen-or antigen-specific immunotherapy in combination with a sustained, locally restricted anti-TNFR1-mediated stimulation of Tregs at the site of allergen or antigen presentation. Thereby, the therapeutic efficacy of allergen- or antigen-specific immunotherapy is significantly increased, and due to the locally restricted delivery of ant-TNFR1 therapeutics at the site of allergen or antigen presentation, potential adverse side effects induced by systemic application of TNFR1 inhibitors are minimized.

In one embodiment the present invention discloses inhibitors of TNFR1 or TNFR1-mediated functions suitable for local delivery at the site of antigen or allergen presentation. Preferred inhibitors of TNFR1 include molecules capable of specific inhibition of sTNF so that the mTNF-TNFR1 and mTNF-TNFR2 interactions remain intact (e.g., dominant-negative sTNF mutants), and molecules capable of specific inhibition of TNFR1 while leaving TNFR2 signaling intact (e.g., TNFR1-specific monoclonal antibodies, aptamers with specificity for TNFR1, antagonistic TNF mutants with specificity for TNFR1, antisense oligonucleotides with specificity for TNFR1, and TNFR1 silencing by RNAi)). Preferred inhibitors of TNFR1-mediated functions include S-methylglutathione, pro-glutathione drugs (e.g., N-acetyl-L-cysteine and alpha-lipoic acid), and salicylates )e.g., aspirin and sodium salicylate).

In another embodiment, the present invention discloses methods for restricting high local concentrations of inhibitors of TNFR1 and TNFR1-mediated functions mainly to the site of allergen presentation to reduce adverse effects due to interaction of the inhibitors with targets distal from the site of allergen or antigen presentation. In one specific embodiment, inhibitors are used which provide a relatively short serum half-life that is sufficient for the inhibitors to be locally active upon their release from a depot at the site of allergen presentation, and which allows fast removal from circulation upon diffusion and transport away from the site of allergen presentation. Preferred inhibitors providing such characteristics include but are not limited to a) low molecular weight inhibitors of TNFR1-mediated functions such as but not limited to N-acetyl-L-cysteine and S-methylglutathione, b) antisense oligonucleotides with specificity for TNFR1, c) small size aptamers capable of inhibiting TNFR1, and d) TNF-alpha mutants. In a specific embodiment, TNFR1-specific inhibitors are used which are susceptible to inactivation by endogeneous enzymes after their release from a depot at the site of allergen or antigen presentation and which can be derivatized to adjust their susceptibility to enzymatic inactivation according to the needs of the method of the present invention. Preferred inhibitors providing such characteristics include but are not limited to aptamers the susceptibility of which to degradation by endogenous nucleases can be adjusted by chemical modification of the bases and the backbone structure.

In another embodiment the present invention discloses suitable matrices for sustained local delivery of inhibitors of TNFR1 or TNFR1-mediated functions at the site of allergen presentation along with a prolonged presentation of allergens or antigens. To achieve effective and prolonged inhibition of TNFR1 or TNFR1-mediated functions at the site of allergen or antigen presentation, the present invention discloses methods for a sustained release of such inhibitors or combinations of such inhibitors from a depot at the site of allergen presentation. Preferred matrices include but are not limited to biodegradable polymers which are suitable as depot for substantial quantities of such inhibitors or inhibitor combinations, which allow the release of sufficient quantities of the inhibitor or inhibitor combinations for efficient local inhibition of TNFR1 or TNFR1-mediated functions over a prolonged period of time, and which are chemically and physically compatible with the adjuvant and allergen (s) or antigen(s) used for the induction of tolerance according to the method of the present invention. In a preferred specific embodiment, injectable in situ-forming gel systems which are biodegradable, are used for controlled delivery of inhibitors of TNFR1 or TNFR1-mediated functions. Such in situ-forming gel systems (hydrogels) undergo a sol-gel-sol transition, which is free flowing sol at room temperature and a non-flowing gel at body temperature. Compared to other biodegradable polymers, the injectable thermogelling polymers possess several advantages including easy preparation, high encapsulation efficiency of bioactive molecules such as inhibitors of TNFR1 or TNFR1-mediated functions, and free of harmful organic solvents in the formulation process.

In another embodiment, the present invention discloses methods for combining sustained local delivery of TNFR1 inhibitors at the site of antigen or allergen presentation with approaches for sustained local depletion of complement component C3 (patent application EP 12075059.1) and/or sustained local inhibition of IL-4/IL-13 (patent application EP 11075261.5). Both techniques have the potential to improve the development of tolerance by several mechanisms including the generation of Tregs.

In another embodiment, the present invention discloses adjuvants for allergen or antigen presentation according to the method of the present invention. In a preferred embodiment, adjuvants are used which provide a depot effect for the allergens or antigens to be administered. If IL-4/IL-13 inhibitors are included in the treatment protocols, adjuvants are preferred which elicit Th2-type immune responses. Although adjuvants eliciting primarily a Th2-type immune response are potentially interfering with the induction of Tregs, in the presence of inhibitors of Il-4- and IL-13-mediated pathways such adjuvants are better suited for the therapeutic aims of the present invention than those elicting primarily a Th1-type immune response. Preferred adjuvants eliciting Th2-type immune responses include but are not limited to aluminum salts, Montanide emulsions (squalene-based water-in-oil emulsions) and polyphosphazenes. Adjuvants eliciting a combined Th1- and Th2-type immune resonse may also be used for the method of the present invention if such adjuvants provide advantageous properties for a particular composition of the present invention. Adjuvants eliciting a combined Th1-type and Th2-type immune response include but are not limited to squalene-based oil-in-water emulsions, granulocyte-macrophage colony stimulating factor (GM-CSF), and adjuvants based on inulin and virosomes.

In another embodiment, the present invention discloses the application of sustained local delivery of inhibitors of TNFR1 or TNFR1-mediated functions in combination with antigen- or allergen-specific immunotherapy for the treatment of patients from allergy, allergic asthma or various autoimmune diseases including but not limited to diabetes type 1, rheumatoid arthritis, juvenile idiopathic arthritis, and multiple sclerosis. Preferably, the methods of the present invention are applied for the treatment of patients having a TNF-alpha-related condition or are at risk developing a TNF-alpha-related condition, and for which enhanced mTNF-TNFR2 signaling by specific inhibition of TNFR1-mediated signaling is beneficial.

In another embodiment, the present invention discloses compositions comprising one or more inhibitors of TNFR1 or TNFR1-mediated functions, and one or more matrices mediating sustained delivery of the components. In one specific embodiment, the composition comprises a biodegradable thermogelling polymer solution containing one or more soluble inhibitors of TNFR1 or TNFR1-mediated functions. Upon injection, this fluid formulation spontaneously gels as the temperature of the formulation rises to body temperature. Thereby, the injected gel forms a non-flowing depot from which one or more soluble inhibitors of TNFR1 or TNFR1-mediated functions are released slowly and continuously for several days. The quantity of inhibitors in the composition is balanced in a way that upon gelation of the polymer composit at body temperature, the amount of released inhibitors is sufficient for the therapeutic aims of the method of the present invention. For prolonged allergen or antigen presentation at the site of the gelled polymer composit, one or more allergens or antigens are adsorbed onto aluminium salts or emulsified to form a squalene-based water-in-oil emulsion (Montanide emulsion), and injected in close proximity of the gelled polymer composit.

In another embodiment, the present invention discloses compositions comprising one or more inhibitors of TNFR1 or TNFR1-mediated functions, one or more antigens or allergens, and one or more matrices mediating sustained delivery of the components. In one specific embodiment, the composition comprises a biodegradable thermogelling polymer solution containing one or more soluble inhibitors of TNFR1 or TNFR1-mediated functions, and one or more allergens or antigens either adsorbed onto aluminium salts, or emulsified to form a squalene-based water-in-oil emulsion (Montanide emulsion), or premixed with a water-soluble polyphosphazenes polymer adjuvant. The quantity of each component in the composition is balanced in a way that a) upon injection into the body the biodegradable thermogelling polymer forms a non-flowing gel in which the other components are embedded, and b) upon gelation of the polymer composit at body temperature the amount of released components is sufficient for the therapeutic aims of the method of the present invention.

In another embodiment the present invention discloses compositions comprising one or more inhibitors of TNFR1 or TNFR1-mediated functions, one or more antigens or allergens, one or more adjuvant molecules supporting the induction of tolerance, and one or more matrices mediating sustained delivery of the components. Preferred adjuvant molecules supporting the induction of tolerance include but are not limited to recombinant human C3 derivatives for local depletion of complement component C3 (patent application EP 12075059.1) and IL-4/Il-13 inhibitors for local inhibition of IL-4/IL-13-mediated effects (patent application EP 11075261.5). In one specific embodiment, the composition comprises a biodegradable thermogelling polymer solution containing one or more allergens or antigens adsorbed onto a depot-mediating support, one or more soluble inhibitors of TNFR1 or TNFR1-mediated functions, and one or more adjuvant molecules supporting the induction of tolerance. The quantity of each component in the composition is balanced in a way that a) upon injection into the body the biodegradable thermogelling polymer forms a non-flowing gel in which the other components are embedded, and b) upon gelation of the polymer composit at body temperature the amount of released components is sufficient for the therapeutic aims of the method of the present invention.

In another embodiment, the present invention discloses methods for incorporating vaccine compositions into pharmaceutical formulations suitable for administration.

In yet another embodiment, the present invention discloses therapeutic methods including therapeutic applications of suitable compositions, the determination of therapeutically effective doses, and modes of administration for the induction of allergen or autoantigen tolerance using the compositions of the present invention.

Specific preferred embodiments of the present invention will become evident from the following more detailed description and the claims.

## BRIEF DESCRIPTION OF THE DRAWINGS

Figure 1 shows the *in vitro* release of a model protein (anti-lysozyme antibody) from gelled polymer at 37°C. The polymers were synthesized according to Example 2.1. Two gels at 25% concentration in PBS pH 7.4 were compared. One $\mu$l of the antibody was added to 200 $\mu$l of the gel solution and incubated at 37°C for gelation. Gels were covered with 2 ml PBS and incubated at 37°C. At indicated times samples of the supernatant of the gels were taken and analyzed in ELISA for the amount of released antibody protein binding to lysozyme. The amount of protein released is given as percentage of total release in comparison to a sample with 1 $\mu$l antibody in 2.2 ml PBS without gel.

Figure 2 shows the *in vitro* release of N-acetyl-L-csyteine (NAC) from gelled polymer at 37°C. The polymers (30% concentration in water) were synthesized according to Example 2.1. 20 $\mu$l 10x PBS (pH 7.4), 3 $\mu$l water and 10$\mu$l of NAC (800 mM) were added to 167 $\mu$l gel solution and incubated at 37°C for gelation. Gels were covered with 1.8 ml PBS and incubated at 37°C. At indicated times samples of the supernatant of the gels were taken and analysed for NAC using the reaction of Fe(II)-TPTZ with thiols, which gives the corresponding photometric adsorbance at 590 nm. Controls were prepared, corresponding to complete release values (100%), and dilutions thereof to provide a calibration curve. Release of NAC is depicted as percentage of total release over time.

Figure 3 shows the *in vitro* release of reduced glutathione (GSH) from gelled polymer at 37°C. The polymers (30% in water) were synthesized according to Example 2.1. 20 ml 10 xPBS (pH 7.4), 3 $\mu$l water and 10 $\mu$l GSH in water, were added to 167 $\mu$l gel solution and incubated at 37°C for gelation. Gels were covered with 1.8 ml PBS and incubated at 37°C. At indicated times samples of the supernatant of the gels were taken and analysed using the reaction of Fe(II)-TPTZ with thiols, which gives the corresponding photometric adsorbance at 590 nm. Controls were prepared, corresponding to complete release values (100%), and dilutions thereof to provide a calibration curve. Release of GSH is depicted as percentage of total release over time.

Figure 4 shows the *in vitro* release of sodium salicylate (SA) from gelled polymer at 37°C. The polymers (30% concentration in water) were synthesized according to Example 2.1. 20 $\mu$l 10xPBS (pH 7.4), 3 $\mu$l water, and 10$\mu$l SA in water, were added to 167 $\mu$l gel solution and incubated at 37°C for gelation. Gels were covered with 1.8 ml PBS and incubated at 37°C. At indicated times samples of the supernatant of the gels were taken and analysed for SA by derivatization with Fe(III) and quantification of the violet coloured tetraaquosalicylatroiron (III) complex at 530 nm. Controls were prepared, corresponding to complete release values (100%), and dilutions thereof to provide a calibration curve. Release of SA is depicted as percentage of total release over time.

Figure 5 shows the *in vitro* release of oligodeoxynucleotides (ODNs) complexed with cationic liposomes (Cellfectin II) from gelled polymer at 37°C. The polymers (30% concentration in water) were synthesized according to Example

2.1. 20 μl of 10xPBS (pH 7.4) and 13 μl of ODN/Cellfection complexes prepared with a 22-mer ODN according to Example 3.5.2., were added to 167 μl gel solution and incubated at 37°C for gelation. Gels were covered with 1.8 ml PBS and incubated at 37°C. At indicated times samples of the supernatant of the gels were taken and analysed for released ODNs by absorption at 260 nm and densitometric analysis of UV-iluminated ethidium bromide stained agarose gel. Controls were prepared, corresponding to complete release values (100%), and dilutions thereof to provide a calibration curve. Release of ODN is depicted as percentage of total release over time.

Figure 6 is a schematic representation of the rhC3-derivative H5 encoding a hybrid protein of approx. 91% identity with human C3. **A:** Chain structures of C3 and pro-CVF. **B:** Structure of the cDNA of H5. The dark part of the nucleic acid sequence from the 5' end up to the BglII cleavage site represents part of the molecule comprising a C3 nucleic acid sequence. the white part downstream of the BglII site comprises a CVF nucleic acid sequence (for details, see Example 4.2.).

Figure 7 shows the complement-consuming activity of natural cobra venom factor (nCVF), rhC3-derivative H5 and human C3 (hC3). The assay was performed as described in Example 4.2. Natural CVF is used as control for a stable C3 convertase with half-life of 7 hours and hC3 as control for a C3 convertase with a short half-life of 1.5 min (for details, see Example 4.2.). The figure shows the mean values ± standard deviation of at least three independent experiments.

Figure 8 shows the treatment schedule of a vespid venom-allergic patient under concomitant anti-TNF-alpha therapy and venom-specific immunotherapy.

Figure 9 shows the experimental schedule for sensitization of mice with OVA (black arrows), followed by s.c. immunotherapy (SIT) with OVA (brick-filled arrows) in the absence or presence of anti-TNF-alpha moAb (light grey arrows), and evaluation of SIT efficacy after inhalative OVA challenge (cross-filled grey arrows) by BAL analysis (dark grey arrow).

Figure 10 shows the effect of anti-TNF-alpha therapy during immunotherapy (SIT) with OVA on airway eosinophilia as determined by bronchoalveolar lavage (BAL) analysis (for details, see Example 9). Bars show eosinophils as percentage of total cells BAL samples.

Figure 11 shows the effect of anti-TNF-alpha therapy during immunotherapy (SIT) with OVA on allergen-specific IgG1 response (for details, see Example 9). S, sensitization with OVA; aTNF; treatment with anti-TNF-alpha moAb.

Figure 12 shows the DNA sequence coding for human TNF-alpha receptor 1 (TNFR1; Database no.: NM_001065.3; mRNA with 5'-and 3'-untranslated regions; 2258 base pairs; CDS: 304-1671, Signal peptide: 304-366, Mature peptide: 367-1668).
The underlined sequence marks position of TNFR1-specific 22mer antisense pPT ODN (Ojwang and Rando 1999). Preferred TNFR1-specific anti-sense oligodeoxynucleotides (anti-TNFR1-ODNs) are 15-mer to 25-mer ODNs selected randomly from the TNFR1-coding sequence. The sequence is also given in SEQ ID 1.

Figure 13 shows the amino acid sequence of human TNF-alpha.
The depicted protein consists of 157 amino acids.
In underlined and bold the positions of mutations in WO2005066206 (A1) are shown. In underlined and italics the positions of mutations in EP1354893 (B1) are shown.
Positions 84-89 are mutated in R1antTNF to STTHNQ (see Shibata et al 2008). The sequence is also given in SEQ ID 2.

Figure 14 shows the amino acid sequence of human interleukin-4 (IL-4).

A) The DNA sequence (without signal peptide sequence; 390 base pairs; Stop codon marked in bold) (SEQ ID 3) and protein sequence (without signal peptide; 129 amino acids; Asterix is marking stop codon position) of the native form (SEQ ID 4).
B) The DNA sequence (codon optimized for E. coli expression; 453 base pairs; N-terminal 6xHis- and protease cleavage-Tag; Stop codon marked in bold) (SEQ ID 5) and protein sequence (150 amino acids, approx. 17.2 kDa; N-terminal tag underlined; Asterix is marking stop codon position) of the human IL-4 RY mutant. The introduced mutations are R142D and Y145D (SEQ ID 6).

Figure 15 shows the amino acid sequence of murine interleukin-4 (IL-4).

A) The DNA sequence (without signal peptide sequence; 363 base pairs; Stop codon marked in bold) (SEQ ID 7) and protein sequence (without signal peptide; 120 amino acids; Asterix is marking stop codon position) of the native form (SEQ ID 8).
B) The DNA sequence (codon optimized for E. coli expression; 429 base pairs; N-terminal 6xHis- and protease cleavage-Tag; Stop codon marked in bold) (SEQ ID 9) and protein sequence (141 amino acids, approx. 15.8 kDa; N-terminal tag underlined; Asterix is marking stop codon position) of the murine IL-4 QY mutant. The introduced mutations are Q137D and Y140D (SEQ ID 10).

Figure 16 shows the amino acid sequence of human C3-derivative H5 (rhC3-H5).
Shown is the protein, as processed mature form (1637 amino acids). The construct contains 279-271 continuous amino acids identical to CVF. The marked in black sequence part derives from human C3 (SEQ ID 11).

## DETAILED DESCRIPTION OF THE INVENTION

The functions of Tregs in allergic and autoimmune diseases indicates that control of the development, survival, and function of Tregs is instrumental for effective control of immune responses. In the presence of IL-2, TNF-alpha-mediated activation of Tregs via interaction with TNFR2 results in proliferation, up-regulation of FoxP3 expression and increase of their suppressive activity. In order to favor TNFR2-mediated activation of Tregs, the present invention discloses methods for local delivery of high concentrations of TNFR1 inhibitors over a prolonged period of time at the site of antigen or allergen presentation. By locally sustained delivery of TNFR1 inhibitors in the presence of antigens or allergens, TCR-mediated activation of Tregs can be optimally amplified by locally available TNF-alpha via TNFR2/IL-2-mediated up-regulation of their proliferation and suppressive activity. Furthermore, potential adverse side effects such as increased risk for infections are minimized since systemically effective TNFR1 inhibitor concentrations are not established by local delivery of TNFR1 inhibitors.

### I. SELECTIVE TNFR1 INHIBITION

Preferred strategies for inhibition of TNFR1 or TNFR1-mediated functions include those that allow selective inhibition of TNFR1 or TNFR1-mediated functions (for a review, see Van Hauwermeiren et al., 2011). In one embodiment of the present invention, inhibitors are used which allow specific inhibition of sTNF such as dominant-negative sTNF mutants, so that the mTNF-TNFR1 and mTNF-TNFR2 interactions remain intact. In another embodiment of the present invention, inhibitors are used which allow specific inhibition of TNFR1 such as anti-TNFR1 antibodies or TNFR1-specific antisense oligonucleotides, while leaving TNFR2 signaling intact. In still another embodiment of the present invention, inhibitors are used which allow selective inhibition of TNFR1-mediated functions such as N-acetyl-L-cysteine (NAC) or glutathione (GSH). Preferred are small inhibitor molecules which can readily access sites that are difficult to target with large molecules such as the immunological synapse formed between the antigen presenting cells and the T cells. Preferred are also inhibitors with low toxicity such as NAC.

#### I.1. Inhibition of TNFR1-mediated effects by N-acetylcysteine, glutathione and derivatives thereof

In vivo, N-acetyl-L-cysteine (NAC, ACC) is readily deacetylated to form cysteine which efficiently supports intracellular glutathione (GSH) biosynthesis. In vitro, NAC and GSH have been shown to act on T cells by increasing interleukin-2 (IL-2) production, synthesis and turnover of IL-2 receptors (Liang et al., 1989), proliferation (Eylar et al., 1993), cytotoxic properties (Liang et al., 1991), and resistance to oxidative stress-mediated apoptosis (Sandstrom et al., 1994). Subsequent analyses revealed also a decrease of human IL-4 production by stimulated peripheral blood T cells and T helper cells after treatment with NAC and GSH in a dose-dependent manner, associated with a decrease of IL-4-induced IgE and IgG4 production by human peripheral blood mononuclear cells (Jeannin et al., 1995). In mice, oral administration of NAC (water containing 0.5-2.0 g/l NAC) for 8 days decreased both IgE and IgG1 antibody responses to intraperitoneally injected ovalbumin (1 μg) adsorbed onto aluminium hydroxide (Jeannin et al., 1995).

Recent studies have reported that glutathione deficiency might be causally linked to exacerbated TNF-alpha activation in several chronic diseases including neurodegeneration (Andersen, 2004), rheumatoid arthritis (Feldmann and Maini, 2003), and chronic lung diseases (Tirouvanziam et al., 2006). The well being of the patients affected with those diseases has been improved by treatment with the glutathione precursor NAC (e.g., Demedts et al., 2005).

NAC has also been demonstrated to prevent the deleterious effects of TNF-alpha on calcium transients and contraction in rat cardiac myocytes (Cailleret et al., 2004), and to hinder the progression of cardiac injury in hypertensive rats (Bourraindeloup et al., 2004). TNF-alpha is not expressed in the normal heart, but is up-regulated in the failing heart and the cardiac glutathione status determines the severity of the adverse effects of TNF-alpha on heart contraction (Cailleret et al., 2004). Interaction of sTNF-alpha with TNFR1 mediates several processes including oxidative stress and activation of the Mg-dependent neutral sphingomyelinase (N-SMase) which hydrolyzes sphingomyelin into ceramide, thereby controlling the sphingolipid signaling cascade and mediating TNF-alpha apoptotic and negative inotropic effects in cardiomyocytes. Downstream ceramide generation, the N-SMase pathway comprises down-regulation of the pro-survival factor Bcl-2, resulting in a shift of the pro-apoptotic Bax/pro-survial Bcl-2 protein ratio which in turn elicits activation of caspase-3. Sphingosine, produced by downstream ceramide production after activation of N-SMase by arachidonic acid (Amadou et al., 2002), mediates the immediate negative inotropic effects of TNF-alpha in mammalian cardiac myocytes (Oral et al., 1997).

Both TNFR1-mediated pathways, oxidative stress and N-SMase activation, are inhibited by glutathione which serves as natural inhibitor of N-SMase (Liu and Hannun, 1997) and as scavenger of reactive oxygen species (ROS). In addition, high cellular glutathione concentrations inhibit also nuclear factor kappa B activation (Staal et al., 1990), thereby down-regulating TNF-alpha expression (Von Haehling et al., 2004). NAC has also been reported to selectively block TNF-alpha-induced signaling by lowering the affinity of TNFR1 to TNF-alpha (Hayakawa et al., 2003). However, in cardiac myocytes isolated from NAC-treated rats, TNF-alpha enhanced in the absence of NAC calcium transients and cell fractional shortening via TNFR2-dependent activation of several proteins including ERK, MSK1, cPLA2, and CaMKII (Defer et al., 2007). Consequently, direct inhibition of TNF-alpha binding to TNFR1 by NAC or direct antioxidant effect of NAC can be ruled out.

In vitro and in vivo experiments have demonstrated that glutathione and its precursor NAC selectively neutralize TNFR1-mediated effects of TNF-alpha while releasing TNFR2 pathways. For example, glutathione repletion by oral NAC treatment of post-myocardial infarction rats has been shown to induce cardiac function and tissue recovery (Adamy et al., 2007). TNFR2-dependent activation of the cytosolic Phospholipase A2 has been identified as mechanism for the positive effect of TNF-alpha on the cardiomyocytes (Defer et al., 2007). With regard to allergen-specific immunotherapy or vaccination with self-antigens, glutathione and its precursor NAC provide important signals for the induction of tolerance via activation and proliferation of Tregs. As shown by Liang et al. (1989), NAC and GSH act on T cells by increasing interleukin-2 (IL-2) production, synthesis and turnover of IL-2 receptors. Up-regulation of IL-2 and its receptor is important for the induction of tolerance since in the presence of IL-2 TNF-alpha-mediated activation of Tregs via interaction with TNFR2 results in proliferation, up-regulation of FoxP3 expression and increase of their suppressive activity. Furthermore, the decreased production of human IL-4 in stimulated peripheral blood T cells and T helper cells after treatment with NAC and GSH (Jeannin et al., 1995) is helpful since IL-4 plays a key role in the development of allergic inflammation.

Analysis of the pharmacokinetics, pharmacodynamics and toxicity of NAC in a Phase I clinical study (Pendyala and Creaven, 1995) revealed that in most of the subjects the highest non-toxic dose was 800 $mg/m^2$/day. NAC was readily absorbed (median $t_{max}$: 1.0 h) and the plasma decay proved to be monoexponential ($t_{1/2}$: 2.4 h). Pharmacokinetics were found to be linear with dose.

A potential problem of NAC-induced inhibition of TNFR1-mediated effects, however, is the requirement of an extended pre-treatment period with NAC to achieve the desired therapeutic efficacy. For example, experiments with post-myocardial infarction rats have demonstrated that oral NAC treatment for an extended period of time is required to benefit from this therapy. After 3-day NAC treatment (120 mg/kg/day) of 2-month post-myocardial infarction rats, the GSH content of the left ventricle was partly replenished to 75% of its control level in sham rats, N-SMase activation was blunted, the Bcl-2 content replenished and caspase-3 inhibited, but changes of the cardiac phenotype required 1-month NAC treatment (Adamy et al., 2007).

S-Methylglutathione (GSM) provides an attractive alternative to NAC, since a short incubation of isolated cardiomyocytes with GSM under in vitro conditions has been demonstrated to reproduce the effects of in vivo NAC treatment (Cailleret et al., 2004). Gutathione repletion and unmasking of TNFR2-mediated effects by TNF-alpha in the isolated cardiomyocytes was achieved by incubation for 2-3 hours in the presence of 50 μmol/L GSM. In contrast, incubation of the isolated cardiomyocytes under identical conditions with 50 μmol/L NAC or 10 mmol/L GSH could not reproduce the effects of in vivo NAC treatment.

Based on these data, S-methylglutathione is most preferred for the methods of the present invention. Although precursors of glutathione such as NAC, alpha-lipoic acid and S-adenosylmethionine (SAMe) require an extended period of pretreatment before antigen or allergen presentation, they are also useful for the methods of the present invention. Low concentrations of lipoate and its analogue lipoamide have been shown to increase GSH in Jurkat cells and mitogenically stimulated peripheral blood lymphocytes (Sen et al., 1997). Additional clinically relevant pro-glutathione drugs include but are not limited to L-2-oxothiazalidine-4-carboxylate (OTC), reduced cysteine and oxidized cystine disulfide (for a review, see Sen, 1997). GSH is also applicable although administered GSH is not effectively transported into cells (for a review, see Sen, 1997).

### I.2. Inhibition of TNFR1-mediated effects by salicylates

Salicylates including but not limited to Aspirin (acetylsalicylic acid; ASA) and salicylic acid (SA) represent also preferred inhibitors for the methods of the present invention. ASA is a widely used non-steroidal anti-inflammatory drug (NSAID). The anti-inflammatory actions are mediated by inhibition of the inducible COX (cyclo-oxygenase) isoform COX-2, whereas the detrimental effects on gastric mucosa viability and platelet function are due mostly to inhibition of COX-1. However, several studies have demonstrated that ASA and related salicylates have a spectrum of biochemical and pharmacological effects that are not related to COX inhibition. Lower concentrations of ASA and AS can enhance (by means of inhibition of monocyte prostaglandin generation) the expression of some Th1 cytokines and, thereby, counteract Th2 responses (e.g., Tsuboi et al., 1995). High doses of ASA and SA, however, have been shown to interfere with the activation of critical transcription factors such as NF-kappa B (Koop and Ghosh, 1994) and activator protein 1 (AP-1; Dong et al., 1997). NF-kappa B is found in the cytoplasm of cells in an inactive form in association with the inhibitor I-kappa B-alpha. Stimulation triggers the release of NF-kappa B from I-kappa B, resulting in the translocation of NF-kappa B from the cytoplasm to the nucleus where NF-kappa B binds to DNA and regulates transcription of specific genes. Most of the genes known to be activated by NF-kappa B are involved in the immune and inflammatory response. These include cytokines such as IL-1, IL-6, IL-8, interferon-beta, and TNF-alpha, and the cell adhesion molecules endothelial leukocyte adhesion molecule-1 (ELAM-1), intercellular adhesion molecule-1 (ICAM-1), and vascular cell adhesion molecule-1 (VCAM-1). The inhibitory effect of salicylates is caused by activation of the p38 mitogen-activated kinase which leads to inhibition of TNF-alpha-induced I-kappa B-alpha phosphorylation and degradation. As a result of NF-kappa B inhibition, high doses of salicylates can interfere with Th1 cell differentiation and effector responses (e.g., Mazzeo et al., 1998).

An important finding was the observation that TNFR1-mediated activation of NF-kappa B is inhibited by high concentrations of sodium salicylate, but not TNFR2-mediated activation of NF-kappa B (Thommesen and Laegreid, 2005). Apparently, TNFR2 signaling involved in NF-kappa B activation proceeds independently of salicylate-sensitive signaling components, indicating distinct signaling pathways not shared with TNFR1. Inhibition of TNFR1-mediated activation of NF-kappa B requires high doses of salicylate (10-20 mM), but the inhibitory effect is highly reproducible (Thommesen and Laegreid, 2005).

Another major finding was the discovery that ASA and AS reduce IL-4 secretion and RNA expression in human CD4+ T cells (Cianferoni et al., 2001). IL-4 inhibition is important for the induction of Tregs since GATA3 induced by IL-4 inhibits the expression of FOXP3, which is a requirement for inducible Treg differentiation (Mantel *et al.,* 2007). The inhibitory effect on IL-4 expression occurs at the transcriptional level and is due to interference with the binding of a calcium-inducible factor to a proximal IL-4 promoter element upstream of, but not overlapping, the NF-kappa B-binding P1 element. IL-4 gene inhibition by ASA is not associated with reduced NF-kappa B activation (Cianferoni et al., 2001). As important is the observation that salicylate-mediated inhibition of IL-4 expression does not affect IL-2 expression (Cianferoni et al., 2001), an essential cytokine for up-regulating TNFR2 expression on Tregs (Chen and Oppenheim, 2010). Since TNF-alpha has been shown to selectively up-regulate the expression of the IL-2 receptor alpha-chain (CD25) on Tregs (Chen et al., 2007), both TNF-alpha and IL-2 generate a powerful mechanism for receptor amplification and synergistically up-regulate Treg suppressive activity.

The various inhibitory effects of ASA and related salicylates are concentration-dependent. A 50% inhibition by ASA requires a concentration of approx. $2 \times 10^{-6}$ M for COX-1, appprox. $3 \times 10^{-4}$ M for COX-2, approx. $1 \times 10^{-3}$ M for IL-4 gene transcription, and approx. $3 \times 10^{-3}$ M for NF-kappa B translocation (Cianferoni et al., 2001). Selective inhibition of TNFR1-mediated activation of NF-kappa B requires even higher doses of salicylates in the range of 1-2 x $10^{-2}$ M (Thommesen and Laegreid, 2005). However, the therapeutic range for ASA and SA has been restricted to 0.8-1.7 $10^{-3}$ M, since salicylate-related toxicity (e.g., tinnitus) can occur with salicylate plasma levels as low as $1.2 \times 10^{-3}$ M (Furst et al., 1987). Therefore, exploitation of COX-independent effects of salicylates for the induction of regulatory T cells requires novel application techniques that allow for high local concentrations of ASA and related salicylates well above the therapeutic range of systemically administered salicylates without the risk of increased side effects.

The present invention solves this problem by disclosing suitable matrices for sustained local delivery of ASA and related salicylates. Thereby, selective inhibition of TNFR1-mediated activation of NF-kappa B and effective inhibition of IL-4 gene transcription can be achieved at the site of antigen or allergen presentation, while upon diffusion away from the delivery site the salicylate concentration is rapidly decreased to systemically tolerable levels.

It should be noted, however, that application of ASA for selective inhibition of TNFR1-mediated activation of NF-kappa B and inhibition of IL-4 gene transcription is contra-indicated for patients suffering from Aspirin-induced asthma (also known as Aspirin-triad or Aspirin-intolerant asthma, AIA). AIA refers to the development of acute bronchoconstriction, profuse rhinorrhea and skin flushing in asthmatic individuals following the ingestion of Aspirin. AIA is likely to be mediated by a deviation of the arachidonic acid metabolic pathway toward excessive leukotriene production. Both cyclo-oxygenase and lipoxygenase are involved in the arachidonic acid metabolic pathway. The prevalence of AIA is 4.3% in Poland, 8.8% in Finland and 10.5% in Australia (for a review, see Gohil et al., 2010). AIA appears after puberty and is present in 10-20% of adult asthma. Approximately 50% of the patients with AIA have chronic, severe, corticosteroid-dependent asthma, approximately 30% have moderate asthma that can be controlled with inhaled steroids, and approximately 20% have mild to intermittent asthma. Atopy is present in on third of the AIA patients (for a review, see Gohil et al., 2010). Challenge tests with Aspirin are the cornerstones of AIA diagnosis. The nasal challenge is quick and free of life-threatening systemic or bronchial side effects.

While ASA is contra-indicated for patients with ASA, these patients can safely take sodium salicylate, salicylamide and choline magnesium trisalisylate, since these drugs are weak inhibitors of COX. Therefore, sodium salicylate is most preferred for the methods of the present invention.

### I.3. Inhibition of TNFR1 by antisense oligonucleotides

The use of antisense oligonucleotides with specificity for TNFR1 represent another preferred inhibition strategy for the methods of the present invention. TNFR1-specific antisense oligonucleotides have been applied to prevent apoptotic signaling through TNFR1 after ionizing radiation treatment (Huang et al., 2006). Pretreatment of mice with 2'-O-(2-methoxy)ethyl-modified antisense oligonucleotides (25 mg/kg i.p. every other day for a total of four times) protected the mice successfully against radiation-induced liver damage. However, a major limitation to the use of TNFR1-specific antisense oligonucleotides is the limited cellular uptake of oligonucleotides. In order to overcome this problem cationic lipids have been used as one of the major components in making non-viral delivery systems such as liposomes (Ross et al., 1998). Since cationic lipids have positive charges, they can be complexed with negatively charged oligonucleotides and then formulated into cationic liposomes. Such liposomes have been demonstrated to enhance the transfection efficiency of plasmid DNA significantly (Helbling-Leclerc et al., 1999).

Another major limitation to the use of TNFR1-specific antisense oligonucleotides is the necessity for efficient local inhibition of TNFR1-mediated effects without the risk of severe systemic side effects. This problem is solved in the present invention by disclosing suitable matrices for sustained local delivery of cationic liposome-embedded TNFR1-specific antisense oligonucleotides at the site of antigen or allergen presentation. Suitable matrices are listed in section II. Different liposome-hydrogel formulations for topical application of liposomes are described in the literature (e.g., Nie et al., 2011). In a preferred embodiment, the present invention discloses liposome-doped hydrogels for sustained local delivery of cationic liposome-embedded TNFR1-specific antisense oligonucleotides. In a more preferred embodiment, thermosensitive hydrogels doped with cationic liposome-embedded TNFR1-specific antisense oligonucleotides are used.

### I.4. Antagonistic TNF mutants with specificity for TNFR1

In another preferred embodiment, TNFR1-selective antagonistic TNF mutants are used for the method of the present invention. Useful TNF mutants include but are not limited to R1antTNF developed by Shibata et al. (2008).

For systemic applications the TNFR1-selective antagonistic TNF mutant R1antTNF is not suitable due to its very short half-life (approx. 10 min) in plasma (Shibata et al., 2009), but for the method of the present invention TNFR1-selective antagonistic TNF mutants with a short plasma half-life are advantageous. Utilizing matrices for sustained local delivery of such mutants, high local concentrations of the TNF mutants can be achieved at the site of antigen or allergen presentation, whereas upon diffusion away from the delivery site the TNF mutant concentration is rapidly decreased to therapeutically ineffective levels. Thereby, potential systemic side effects are minimized. If necessary, however, the plasma half-life of TNFR1-selective antagonistic TNF mutants can be extended by site-specific PEGylation as described for the TNF mutant R1antTNF (Shibata et al., 2009).

### I.5. TNFR1-neutralizing aptamers

In another preferred embodiment of the invention, TNFR1-neutralizing aptamers are used for the method of the present invention. Aptamers are nucleic acid binding species generated by iterative rounds of in vitro selection (for reviews, see Famulok et al., 1999; Yan et al., 2005). Typically, selected aptamers bind very tightly (often in the low nanomolar range) and specifically to their targets. Aptamers can also discriminate between closely related protein targets. Aptamers have several key features that make them particularly well suited as reagents for the method of the present invention. First, aptamers are relatively small and can readily access sites which are difficult to target with large molecules such as the immunological synapse that is formed between the antigen presenting cells and the T cells. Another advantage for the present invention is the possibility to engineer the stability of aptamers towards degrading nucleases, thereby determining the period of their inhibitory activity. Unmodified RNA- and DNA-based aptamers can begin degrading in serum within minutes, whereas aptamers containing modified bases and phosphorothioate linkages display a significantly increased stability towards degrading nucleases. For example, 2'-aminopyrimidine-modified RNA has been shown to remain stable for days. Furthermore, modifications can also be applied to the ends of aptamers to confer greater stability. For example, a 3'-3' linkage can be added to prevent 3'-exonuclease degradation. Stable aptamers can be generated by the Spiegelmer-technology (for a review, see Famulok et al., 1999) or by utilizing peptide nucleic acids (EP 1 785 490 B1). Peptide nucleic acids (PNA) represents a class of nucleic acid analogues where the charged deoxyribose-phosphodiester backbone has been replaced by a peptide strand comprising N-(2-aminoethyl)glycine monomers with the nucleobases adenine, thymine, guanine and cytosine attached to their alpha-carbon. Due to its artificial character PNA provides complete resistance against nucleases and proteases.

In another preferred embodiment of the invention, aptamers are used which provide limited resistance against enzymatic degradation that is sufficient for effective inhibition of TNFR1 at the site of allergen presentation and in its close proximity, but guarantees rapid degradation of the aptamer while diffusing away from this site. In a specific embodiment, the stability of an inhibitory aptamer is adjusted according to the requirement of the method of the present invention by modification of its structure including but not limited to the introduction of phosphorothioate linkages, modification of bases (e.g., by modification with 2'-aminopyrimidine), and addition of a 3'-3' linkage to the end of the aptamer.

In another preferred embodiment of the invention, the physiological clearance of an inhibitory aptamer is adjusted according to the requirement of the method of the present invention. In cases of an extremely fast clearance, the plasma life-time of an inhibitory aptamer can be extended by attachment of lipids or high molecular weight compounds such as 40 kD polyethylene glycol moieties.

**I.6. Dominant-negative sTNF mutants**

In another preferred embodiment of the invention, variant TNF proteins are used for the method of the present invention which rapidly form inactive heterotrimers with native sTNF-alpha to give complexes that neither bind to nor stimulate signaling through TNF receptor. By sequestration of sTNF-alpha these variant TNF proteins selectively inhibit the pro-inflammatory action mediated by TNFR1. Useful variant TNF proteins with these characteristics, named dominant-negative TNF variants (DN-TNF), include but are not limited to those described by Steed et al. (2003). One of the most potent DN-TNF molecules, the TNF-alpha double mutant A145R/Y87H, has been demonstrated to efficiently protect mice from TNF-alpha-induced damage. Lethal doses of native TNF-alpha (30 μg/kg) mixed before injection with varying ratios of A145R/Y87H produced no TNF-induced damage. This protection was even observed at native:variant ratios as low as 1:1 and with a superlethal dosis of TNF-alpha (Steed et al., 2003). Furthermore, A145R/Y87H proved to be non-toxic. Dosed as high as 30 mg/kg of mouse body weight (along with 30 μg/kg native TNF-alpha) A145R/Y87H did not induce mortality or hepatotoxicity. In a murine model of collagen-induced arthritis, variant A145R/Y87H reduced joint swelling when dosed once daily at 2 mg/kg subcutaneously with an intravenous loading dose of 2 mg/kg on the first treatment day (Steed et al., 2003).

**I.7. RNAi-mediated inhibition of TNFR1**

In another embodiment, RNA interference (RNAi)-mediated silencing of TNFR1 is used for the method of the present invention. Proof-of-principle of this technology has been demonstrated by using adenoviral delivery (replication-deficient adenoviral vectors with E1/E3 deletion) of a short hairpin RNA expressing construct to the knee joint cavity of mice suffering from experimental arthritis (Arntz et al., 2010).

**I.8. TNFR1-neutralizing antibodies**

In still another embodiment, TNFR1-neutralizing antibodies are used for the method of the present invention. Useful TNFR1-neutralizing antibodies include but are not limited to ATROSAB, a humanized antagonistic TNFR1-specific murine monoclonal antibody (Zettlitz et al., 2010). The antibody has been converted into a full IgG1 antibody using a heavy chain with greatly reduced antibody-dependent cell-mediated cytotoxicity (ADCC) and complement-mediated cytotoxicity (CDC). ATROSAB has been demonstrated to inhibit typical TNF-mediated responses like apoptosis induction and activation of NF-kappa B-dependent gene expression such as IL-6 and IL-8 production. However, in mice ATROSAB showed a plasma half-life of 10.5 ± 2.8 days (Zettlitz et al., 2010) which could increase the risk for systemic side effects.

**II. SUSTAINED LOCAL DELIVERY OF TNFR1 INHIBITORS**

Effective therapeutic targeting of locally expressed TNFR1 or TNFR1-associated signaling components requires a sustained local delivery of TNRF1-specific inhibitors from a depot-mediating matrix for a period of time that is sufficient for the purpose of the particular medical treatment.

In a preferred embodiment, matrices are used for local delivery of suitable TNRF1-specific inhibitors that a) can serve as depot for substantial quantities of one or more suitable TNRF1-specific inhibitors, b) allow the release of sufficient quantities of the embedded TNRF1-specific inhibitors over a prolonged period of time (optimally for a few days), c) are biodegradable, and d) are chemically and physically compatible with the TNRF1-specific inhibitors and other compounds necessary for modulation of effector T cell responses according to the method of the present invention.

In one embodiment of the invention, biodegradable polymers are used for controlled delivery of suitable TNRF1-specific inhibitors. Preferred biodegradable polymers approved by FDA and used in a clinical trial, include but are not limited to poly(D,L-lactic acid), poly(lactic-co-glycolic acid) (PLGA), and copolymers of L-lactide and D,L-lactide. An important characteristic of such polymers is their ability to be applied locally. All FDA approved polymers have been studied extensively for their biocompatibility, toxicology, and degradation kinetics. Furthermore, these polymers have been shown to release embedded therapeutics for several hours up to 40 weeks in vitro and several weeks in vivo.

In a more preferred embodiment, injectable in situ-forming gel systems which are biodegradable, are used for controlled delivery of suitable TNRF1-specific inhibitors (for a review, see Ruel-Gariepy and Leroux, 2004). Preferred in situ-forming gel systems (hydrogels) undergo a sol-gel-sol transition, which is a free flowing sol at room temperature and a non-flowing gel at body temperature. Compared to other biodegradable polymers, the injectable thermogelling polymers possess several advantages including easy preparation, high encapsulation efficiency of bioactive molecules including therapeutic proteins, and free of harmful organic solvents in the formulation process (Qiao et al. 2005).

In one specific embodiment, biodegradable thermogelling block polymers are used which are based on monomethoxy poly(ethylene glycol) (MPEG) including but not limited to a) diblock copolymers consisting of MPEG and poly(ε-caprolactone) (PCL) (Hyun et al., 2007), b) MPEG-*b*-(PCL-ran-PLLA) diblock copolymers (Kang *et al.,* 2010), and c) diblock copolymers consisting of MPEG and PLGA (Peng et al., 2010). MPEG copolymers containing PCL provide the advantage that they do not create an acidic environment upon biodegradation in contrast to MPEG copolymers containing PLLA and PLGA (Hyun et al., 2007).

In another specific embodiment, biodegradable thermogelling triblock polymers are used including but not limited to a) PLGA-PEG-PLGA (Qiao et al., 2005), b) PEG-PLGA-PEG (Zhang et al., 2006), and c) PEG-PCL-PEG (PECE) (Gong et al., 2009a). Various biodegradable thermogelling triblock polymers made up of PLGA and PEG are disclosed in patent application WO 99/18142. At lower temperatures, hydrogen bonding between hydrophilic PEG segments of the copolymer chains and water molecules dominate in aqueous solutions, resulting in the dissolution of these copolymers in water. As the temperature increases, the hydrogen bonding becomes weaker, while hydrophobic forces of the hydrophobic segments such as PLGA segments are getting stronger, leading to sol-gel transition. PEG, PLGA and PCL are well-known FDA-approved biodegradable and biocompatible materials which have been widely used in the biomedical field.

In another specific embodiment, biodegradable thermo-gelling diblock and triblock copolymers are used which consist of polyethylene oxide (PEO) and a biodegradable polyester such as poly-L-lactic acid (PLLA) (Jeong et al., 1997). These block copolymers, however, are a free flowing sol at a higher temperature and form a gel at a lower temperature. For example, a 23% aqueous solution of PEO-PLLA-PEO ($M_r$ 5,000-2,040-5,000) is a sol at 45°C and becomes a gel at 37°C By changing the biodegradable block length, the sol-gel transition temperature can be manipulated, e.g., increasing the PLLA block length increases the aggregation tendency of a block copolymer in water, resulting in a steepening of the gel-sol transition curve slopes and the onset of gelation at lower concentrations. The sol-gel transition temperature is a function of concentration as well as composition of a block polymer.

In another specific embodiment, Poloxamers (trade name Pluronics) are used. Poloxamers are nonionic triblock copolymers composed of a central hydrophobic chain of poly(propylene oxide) (PPO) flanked by two hydrophilic chains of poly (ethylene oxide) (PEO) (Gilbert et al., 1987). Poloxamers exhibit also sol-gel transition behavior in aqueous solutions and have been used for sustained delivery of several therapeutic agents. However, Poloxamers are not biodegradable and can be accumulated in the body which may lead to toxic side effects. Thus, the application of Poloxamers in biomedical fields has been greatly restricted. In a recent study, Pluronic F127 (100-unit PEO chain surrounding one 65-unit PPO) has been used to form composite thermosensitive hydrogels with PECE (Gong et al., 2009b). Based on the results of this study Pluronic F127/PECE composite hydrogels are biocompatible with low cell cytotoxicity and, therefore, may also be suitable for the method of the present invention.

The various biodegradable thermogelling polymers provide different stability characteristics. For example, Poloxamer triblock polymers provide excellent thermosensitivity, but due to weak hydrophobicity of the PPO block such copolymers form fast eroding gels which have been reported to persist in vivo a few hours at most. Exposure of Poloxamer gels to phosphate-buffered saline under in vitro conditions demonstrated gel erosion within 2 days (Hyun et al., 2007). Similar results were observed when the polymer solutions were subcutaneously injected into rats. Poloxamer gels could not be observed after 2 days (Hyun et al., 2007). Different results were obtained with MPEG-PCL gels. Under in vitro conditions MPEG-PCL gels maintained their structural integrity for more than 28 days and after subcutaneous injection into rats MPEG-PCL gels maintained their structural integrity longer than 30 days. The stability of MPEG-PCL gels, however, may also create problems since the rate of degradation of PCL *in vivo* is rather slow (2-3 years) compared to that of PLA, PGA or PLGA (for a review, see Sinha et al., 2004). Thus, after serving the function in delivering a suitable TNFR1 inhibitor, MPEG-PCL copolymers may remain in the body under physiological conditions for an uncertain period. Therefore, most preferred biodegradable thermogelling polymers for the method of the present invention are those which maintain their structural integrity for a few days but do not remain in the body for more than a month.

In a preferred embodiment of the present invention, biodegradable thermogelling polymers are used which allow to modify their degradation kinetics. For example, PLLA segments can be incorporated into the PCL segment of MPEG-PCL copolymers, since PLLA provides better accessibility of water to the ester bonds of PLLA which enhances the hydrolytic degradation of the copolymer (Kang et al., 2010). The resulting MPEG-b-(PCL-ran-PLLA) diblock copolymers offer a therapeutic window that is adjustable from a few weeks to a few months by varying the amount of PLLA in the PCL segment (Kang et al., 2010). In another example, the rate of PLGA-PEG-PLGA hydrogel erosion can be modified by altering the molar ratio of DL-lactide/glycolide in the PLGA segment. The DL-lactide moiety is more hydrophobic than the glycolide moiety. Therefore, by increasing the molar ratio of DL-lactide/glycolide in the PLGA segment of PLGA-PEG-PLGA triblock copolymers, more stable hydrogels are formed due to stronger hydrophobic interactions among the copolymer molecules (Qiao et al. 2005).

Several of the biodegradable thermogelling polymers have been analyzed for their ability to mediate sustained release of proteins. Although different proteins are likely to affect the release behavior of each copolymer in individual ways, characterization of the release of model proteins such as bovine serum albumin (BSA) provides important information. Using composite hydrogels containing different percentages of PECE and Pluronic F127, the in vitro release behavior of BSA proved to be dependent on the hydrogel composition, initial BSA loading amount, and hydrogel concentration (Gong et al., 2009b). Sustained release of BSA above 15 days was achieved with a composite hydrogel containing 60% PECE and 40% Pluronic F127, loaded with 4 mg BSA in the presence of 30wt% hydrogel. Using MPEG-PCL copolymers, sustained in vitro release of BSA proved to be above 20 days, and under in vivo conditions (after subcutaneous injection into rats) sustained release lasted for more than 30 days (Hyun et al., 2007).

While for most clinical applications a sustained release of therapeutic drugs from biodegradable thermogelling polymers over a period of several weeks is desirable, the method of the present invention does not require such an extended sustained release of active substance since the development of immunologic memory requires the engagement of the T cell receptor (TCR) for a period of only 1 to 2 days. Therefore, preferred are biodegradable thermogelling polymers which deliver TNFR1 inhibitors for a limited period only which does not exceed a week.

PLGA-PEG-PLGA triblock copolymers represent one example of hydrogels providing advantageous degradation and release kinetics for the method of the present invention. As demonstrated in a recent study, the release of insulin from PLGA-PEG-PLGA triblock copolymers lasted for approximately 4 days with an initial burst effect during the first day (Choi et al, 2003). The initial burst effect may be advantageous for effective inhibition of TNFR1 in the initial phase. However, the release kinetics may be modified by reducing the solubility of TNFR1-specific inhibitors via complexation with zink as shown for zink-insulin complexes (Choi et al, 2003).

Although Poloxamer gels are not biodegradable, Poloxamer 407 (trade name Pluronic F127) gels represent also an example of thermogelling polymers providing advantageous degradation and release kinetics for the method of the present invention. Although under in vitro conditions the release of BSA from Poloxamer 407 gels proved to be almost complete within 1 day, the sustained release of BSA under in vivo conditions (after subcutaneous injection into rats) lasted for 3 days (Hyun et al., 2007).

In still another embodiment, thermal-sensitive hydrogels formulated on the basis of trimethylated chitosan derivatives (Wu et al., 2012) are used for the sustained delivery of suitable active substances and one or more antigens. In addition to its application as a vaccine delivery system, the cationic polysaccharide has shown promising results as an adjuvant. For example, application of a chitosan solution for subcutaneous vaccination has been demonstrated to enhance both humoral and cell-mediated immune responses (Zaharoff et al., 2007). However, the unfavorable pH-dependent solubility and charge density of chitosan is a limiting factor. In contrast, trimethylated chitosan is well soluble in aqueous solution at neutral pH and provides excellent biocompatibility and mucoadhesive nature. Trimethylated chitosan derivatives are best characterized by the degree of quarternization, the degree of O-methylation and the degree of acetylation (Hagenaars et al., 2010).

Trimethylated chitosan derivatives are especially suited for nasal delivery of vaccines and are frequently formulated into particles or spray powder (Alhalaweh et al., 2009). Very recently, however, the trimethylated chitosan derivative N[(2-hydroxy-3-trimethylammonium) propyl] chitosan chloride has also been formulated together with $\alpha$,$\beta$-glycerophosphate into a thermal-sensitive hydrogel (Wu et al., 2012). This hydrogel was shown a) to significantly prolong the antigen residence time in the nasal cavity, b) to enhance the transepithelial transport via the paracellular routes, and c) to induce in mice a high mucosal immunity (sIgA) and systemic immune response (IgG1 and IgG2a).

For sustained local delivery of liposome-embedded TNFR1-specific antisense oligonucleotides at the site of antigen or allergen presentation, different liposome-hydrogel formulations are suitable (e.g., Nie et al., 2011). In a preferred embodiment, hydrogels doped with cationic liposome-embedded TNFR1-specific antisense oligonucleotides are used. In a more preferred embodiment, thermosensitive hydrogels doped with cationic liposome-embedded TNFR1-specific antisense oligonucleotides are used.

### III. SAFETY ASPECTS OF PEG/PLGA-BASED HYDROGELS

Preferred biodegradable polymers include but are not limited to poly(ethylene glycol) (PEG), poly(D,L-lactic acid), copolymers of L-lactic acid and D,L-lactic acid, poly(glycolic acid), and poly(lactic-co-glycolic acid) (PLGA). The use of these polymers as sustained-release protein delivery systems has been studied extensively (for a review, see Pai et al., 2009) and they represent the most compelling biodegradable polymers for depot systems due their inclusion in the FDA's General Recognized as Safe (GRAS) list for use in medical devices and drug formulations (FDA, http://vm.cfsan.fda.gov/%7Edms/eafus.html. Accessed various dates).

**IV. Adjuvants**

The time of allergen or antigen exposure is extremely important since T cell differentiation and thus the development of immunologic memory requires the engagement of the T cell receptor (TCR) over 12-48 h and long lasting memory may even require repetitive exposure. Adjuvants such as oils and alum provide a depot effect that extends the lifetime of allergens and antigens and thus prolong antigen-stimulation of T cells. However, in addition to the depot effect currently available adjuvants trigger either Th1-type or Th2-type immune responses or both (for reviews, see Leroux-Roels, 2010; Nicholls et al., 2010). Based on the assumption that the generation of Tregs represents a default pathway which requires T-cell receptor activation but the absence of decision signals for effector T cells, currently available adjuvants are not optimal for the purpose of the present invention. For the method of the present invention adjuvants are needed that promote allergen uptake and prolonged stimulation of T cells without providing decision signals, thereby inducing Treg expansion. Unfortunately, adjuvants providing all of the desired properties are not available.

Liposomes, synthetic nanospheres consisting of lipid layers, represent a classic adjuvant that is not active per se. Their main mode of action is through allergen presentation. Liposomes, can encapsulate allergens and act as allergen delivery vehicles. However, liposomes are not suitable for a slow release of allergens, which appears to be essential for a prolonged immune stimulation.

In one embodiment of the present invention, local TNFR1 inhibition used as adjuvant therapy in support of antigen- or allergen-specific immunotherapy, is combined with local inhibition of IL-4/IL-13 (see patent application EP 11075261.5). Coincident blockade of IL-4/IL-13-mediated pathways provides an important benefit for the use of adjuvants eliciting Th2-type immune responses including but not limited to aluminum salts, Montanide emulsions (squalene-based water-in-oil emulsions) and polyphosphazenes. Although adjuvants eliciting primarily a Th2-type immune response are potentially interfering with the induction of Tregs, in the presence of inhibitors of Il-4- and IL-13-mediated pathways such adjuvants are better suited for the therapeutic aims of the present invention than those eliciting primarily a Th1-type immune response. In the presence of inhibitors of Il-4-and IL-13-mediated pathways the contra-productive activity of adjuvants eliciting Th2-type immune responses is significantly reduced or even abolished, whereas the activity of Th1-type promoting adjuvants cannot be influenced. As a result, Th1-type cytokines would interfere with the induction of Tregs.

In one preferred specific embodiment, aluminum-containing adjuvants, typically aluminum phosphate or aluminum hydroxide gels (generally referred to as alum) are used for the method of the present invention. One mechanism of action is due to its retention of immunogenic molecules at specific sites in the body, allowing for their slow release and consequently a prolonged immune response, the so-called depot effect. Alum also appears to exert its adjuvant activity by keeping antigens in a particulate (rather than soluble) form, thereby enhancing phagocytosis of antigens by APCs. Dendritic cells (DC) are capable of taking up both soluble antigens and antigens adsorbed onto aluminum-containing adjuvants. Furthermore, it has become evident that alum is capable of directly activating immune cells. Alum can induce maturation of monocytes/macrophages into DC-like cells. This process is dependent on the activation of NLRP3 (NOD-like receptor family, pyrin domain containing 3), part of the inflammasome which causes production of pro-inflammatory cytokines such as IL-1β and IL-18. Primarily, alum elicits a Th2 response including IL-4 and IL-5 production as well as IgG1 and IgE production by B cells (for a review, see Nicolls et al., 2010).

Upon incubation of allergens with aluminum-containing adjuvants, the antigens or allergens are partially or completely adsorbed onto the adjuvant via electrostatic interactions and exchange between phosphate and hydroxyl groups. Several studies have demonstrated that adsorption onto aluminum-containing adjuvants prevents rapid enzymatic degradation of antigens and allergens, but the adsorption process may also induce structural changes resulting in a decreased thermal stability (for a review, see Clapp et al., 2011). One approach to improving the thermal stability of vaccines is to use a combination of buffers to alter the surface chemistry of aluminum-containing adjuvants and to control the ionization state of the antigen's or allergen's amino acid side chains. Another approach is to identify conditions that stabilizes antigens and allergens in the solution state and to use this as guidance on minimizing the extent of adsorption-induced thermal destabilization of the allergen(s). For practical purposes, however, stabilization procedures may be omitted since the importance of antigen (including allergen) adsorption to aluminum-containing adjuvants for the immunological response has been questioned by recent studies (for a review, see Clapp et al., 2011). For example, the immune response elicited in rabbits against hen egg white lysozyme was irrespective of the fraction of antigen adsorbed onto the aluminum-containing adjuvant (Chang et al., 2001). For maximizing the immune response, however, some degree of adsorption or at least the proximity of the allergen and the adjuvant appears to be important.

In another preferred specific embodiment, polyphosphazenes (water-soluble polymers) are used for the method of the present invention. Poly [di(carboxylatophenoxy) phosphazene] (PCPP) has been shown to enhance antibody responses while being negligibly reactogenic (Nicolls et al., 2010).

In still another specific embodiment, squalene-based water-in-oil emulsions (Montanide emulsions) are used for the method of the present invention. Montanide emulsions are similar to incomplete Freund's adjuvant, but they are biodegradable and therefore significantly less toxic and have been used in several clinical trials (Nicolls et al., 2010).

In another embodiment, adjuvants eliciting a combined Th1- and Th2-type immune response are used for the method of the present invention. Although adjuvants eliciting a combined Th1- and Th2-type immune response are likely to interfere to a certain extent with the induction of Tregs, such adjuvants may provide advantageous properties for the composition, which may be beneficial for the method of the present invention. Adjuvants eliciting a combined Th1-type and Th2-type immune response include but are not limited to squalene-based oil-in-water emulsions, granulocyte-macrophage colony stimulating factor (GM-CSF), and adjuvants based on inulin and virosomes (for a review, see Nicholls et al., 2010).

V. FIELDS OF APPLICATION FOR SUSTAINED LOCAL DELIVERY OF TNFR1 INHIBITORS

Specific inhibition of TNFR1 or TNFR1-mediated signaling allows the development of more effective treatment strategies for diseases in which mTNF-TNFR2 signaling is beneficial, such as multiple sclerosis (MS) and rheumatoid arthritis (RA). Furthermore, patients suffering from other autoimmune diseases such as diabetes type 1 and allergic diseases (e.g., allergic conjunctivitis, allergic rhinitis, allergic asthma) will also benefit from specific inhibition of TNFR1-mediated signaling in combination with specific immunotherapy due to enhanced TNFR2-mediated activation of Tregs.

In one embodiment, the present invention discloses the application of sustained local delivery of TNFR1 inhibitors in combination with antigen- or allergen-specific immunotherapy for the treatment of patients suffering from allergy, allergic asthma or various autoimmune diseases including but not limited to diabetes type 1, RA and MS. It should be mentioned that the method of the present invention is not applicable for the treatment of diseases in which mTNF or TNFR2 contributes to the pathology, such as inflammatory bowel disease (IBD), sarcoidosis and Wegener's granulomatosis (WG) (for a review, see Van Hauwermeiren et al., 2011).

V.1. TREATMENT OF ALLERGY: LOCAL TNFR1 INHIBITION AS ADJUVANT THERAPY FOR ALLERGEN-SPECIFIC IMMUNOTHERAPY

Allergen-specific immunotherapy has been used for many decades as a desensitizing therapy for allergic diseases and represents the potentially curative method of treatment. The main principle of specific immunotherapy in atopic patients is to increase the number of Tregs capable of controlling allergen-specific effector T cells. Based on current knowledge, allergen tolerance is mediated by peripherally induced regulatory T cells as evidenced by a deficit of allergen-specific IL-10 producing T cells in the peripheral blood of allergic patients (for reviews, see Schmidt-Weber et al., 2005; Schmidt-Weber et al., 2006).

Subcutaneous immunotherapy (SCIT) involves the regular subcutaneous injection of allergen extracts or recombinant allergens using incremental regimes, with the induction of tolerance taking from several days to several months depending on the regime used. The usual approach is a build-up phase (consisting of weekly injections) followed by a maintenance phase (consisting of monthly injections). Once tolerance is induced it can last for several years without further treatment. The limiting factor in SCIT is anaphylactic side-effects, which vary in incidence from 0.1-5% of individuals depending on severity. Therefore, improved efficacy with decreased side-effects is the aim of new approaches to SCIT including T-cell-reactive peptide, hypoallergenic recombinant allergens and chemically modified allergens (allergoids). The present invention aims for immune intervention by targeting of the molecular mechanisms of allergen tolerance and reciprocal regulation of effector and regulatory T cells.

V.1.1. *Local selective inhibition of TNFR1 or TNFR1-mediated functions.*

In one embodiment of the present invention, local inhibition of TNFR1 or TNFR1-mediated functions is used as adjuvant therapy at the site of allergen presentation in support of allergen-specific immunotherapy. The rationale for concomitant TNFR1 inhibition along with allergen presentation is based on several lines of evidence.

First, it has been demonstrated that antigen-specific activation of CD4+ effector T cells (Teff) strongly boosts the expansion of CD4+CD25+FoxP3+ Tregs and their suppressive function (Grinberg-Bleyer et al., 2010). TNF family members are involved in the Teff-mediated Treg boost, and the Treg boost has been demonstrated to be partially dependent on TNF-alpha via TNFR2-mediated signaling. Although this helper function of CD4+ T cells was observed in a murine model of type 1 diabetes (Grinberg-Bleyer et al., 2010), a Teff-mediated boost of Tregs represents most likely a general mechanism in immune diseases.

By inhibition of TNFR1-mediated pro-inflammatory effects at the site of allergen presentation, TNFR2-mediated up-regulation of CD25 (alpha-chain of the IL-2 receptor) expression on Tregs is favoured and, as a result of enhanced Il-2-mediated functions, up-regulation of TNFR2 expression on Tregs (for reviews, see Chen and Oppenheim, 2010; Biton et al., 2012). IL-2 has been shown to have a critical role in thymic development, expansion within peripheral blood, and the suppressive activity of CD4+CD25FoxP3+ Tregs. Furthermore, the selective availability of IL-2 has been demonstrated to represent a major determinant controlling the production of CD4+CD25+FoxP3+ T regulatory cells (Yu and Malek, 2006). While the present invention does not provide methods for increasing the local concentration of IL-2, the local synergistical up-regulation of CD25 and TNFR2 according to the method of the present invention favours a selective enhancement of IL-2-mediated functions on Tregs. Therefore, selective inhibition of TNFR1 represents an attractive adjuvant therapeutic approach for enhancing the proliferation, FoxP3 expression and the suppressive activity of Tregs in support of allergen-specific immunotherapy.

The important role of TNF-alpha for the induction of allergen tolerance by specific immunotherapy is further documented by the observation that anti-TNF-alpha therapy during specific immunotherapy prevents the induction of allergen tolerance. As described in detail in the Examples of the present invention.

V.1.2. *Combination of local TNFR1 inhibition with local inhibition of IL-4/IL-13 and/or local depletion of complement component C3.*

In another embodiment of the present invention, local TNFR1 inhibition used as adjuvant therapy in support of allergen-specific immunotherapy, is combined with other local adjuvant treatments including local inhibition of IL-4/IL-13 (see patent application EP 11075261.5) and/or local depletion of complement component C3 (see patent application EP 12075059.1).

Concomitant inhibition of IL-4-mediated functions represents a particularly promising therapeutic approach, since IL-4 inhibits the expression of FOXP3, which is a requirement for inducible Treg (iTreg) differentiation and it is known that IL-4 is released locally upon allergen injection due to the activation of Th2 memory T cells. IL-13 is also known to be associated with the induction of the IgE isotype switch, secretion of IgE by B lymphocytes, and enhancement of IgE-mediated responses. Furthermore, coincident blockade of IL-4/IL-13-mediated pathways provides an additional benefit for the use of alum-adsorbed allergens for specific immunotherapy (see section IV). Therefore, blockade of IL-13-mediated pathways along with allergen stimulation of T cells provides also important benefits for patients undergoing allergen-specific immunotherapy.

Preferred inhibitors of IL-4 and IL-13 are those which a) provide a small to moderate molecular size and high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects mediated by interaction of the inhibitor with targets away from the site of allergen presentation. In a preferred embodiment of the invention, interleukin variants capable of antagonizing the activity of IL-4 and IL-13 such as the double mutant of human IL-4 (R121D/Y124D) are used as inhibitors.

The impact of local C3 depletion on the development of tolerance to allergens is evident from experiments with C3-deficient mice (Yalcindag et al., 2006). In order to investigate the role of C3 in inciting allergic skin inflammation and systemic immune responses after epicutaneous or intraperitoneal sensitization with allergens, C3-deficient ($C3^{-/-}$) mice and wild-type mice were subjected to epicutaneous or intraperitoneal immunization with ovalbumin (OVA) adsorbed onto alum. By comparison of the epicutaneous or intraperitoneal immunization procedure, the study addressed the local synthesis of C3 by keratinocytes and peritoneal macrophages, both of which are activated by the adjuvant alum. The study revealed that splenocytes from C3-deficient mice secreted less TH2-specific interleukins including IL-4, IL-5 and IL-13, and less TH1-specific IFN-$\gamma$ in response to OVA stimulation than splenocytes from wild-type control mice. C3-deficient mice had impaired IgG1, IgG2a, and IgE antibody responses after both epicutaneous and intraperitoneal immunization. It is important to note that the defect was corrected by the addition of purified C3 protein (Yalcindag et al., 2006). Another study has demonstrated that $C3^{+/+}$ and $C3^{-/-}$ dendritic cells (DCs) have different abilities to elicit the development of regulatory T cells (Peng et al., 2006). Co-culturing of CD4+ T cells with allogeneic C3 deficient ($C3^{-/-}$) dendritic cells (DCs) for 9 days yielded a 4-fold higher level in T cells expressing the *foxp3* gene as compared to those stimulated with $C3^{+/+}$ DCs (Peng et al., 2006).

In conclusion, the combination of local inhibition of IL-4/IL-13-mediated effects and local C3 depletion along with local inhibition of TNFR1-mediated functions and allergen stimulation of T cells has the potential to improve the development of tolerance significantly and, thereby, to reverse the pathological processes in allergy.

### V.2. TREATMENT OF ALLERGIC ASTHMA: LOCAL TNFR1 INHIBITION AS ADJUVANT THERAPY FOR ALLERGEN-SPECIFIC IMMUNOTHERAPY

Combination inhalers are highly effective in asthma therapy and relatively inexpensive. However, even when taken regularly, inhaled corticosteroids (ICS) with or without long-acting beta-2-agonists (LABA) are not curative, as asthma symptoms and inflammation rapidly recur when the treatment is discontinued. In contrast, treatments that target the immune deviation in asthma might hold the prospect of long-term cure.

The majority of patients with asthma experience allergies, and treatments that target the underlying allergic inflammation are therefore a logical approach, particularly as such treatments might also treat associated allergic conditions. However, the efficacy and safety of SCIT in patients with allergic asthma is controversial. According to a review of 75 trials covering a total of 3,188 patients with asthma, SCIT led to a significant reduction in asthma symptom scores, medication use and airway hyper-responsiveness, with evidence of a dose-related effect (Abramson et al., 2003). Dose-response studies carried out for dog, cat, ragweed and grass allergies show consistent efficacy of SCIT using between 5 and 20 $\mu$g of the major allergen (for a review, see Holgate and Polosa, 2008). SCIT and SLIT (sublingual immunotherapy) also decrease the development of sensitization to new allergens and decrease the risk of new asthma in both adults and children with rhinitis. A recent retrospective cohort study of 322 subjects with allergic rhinitis showed that 53.1% of subjects who were not treated with SCIT developed asthma, whereas only 41.6% of subjects who received SCIT were diagnosed with asthma (for a review, see Holgate and Polosa, 2008).

Taken together, immunotherapy has been proven efficacious in treating mild asthma, as well as in preventing the progression to asthma in patients suffering from rhinoconjunctivitis (Moller et al., 2002; Jacobsen et al., 2007), but it is not yet recommended for the treatment of moderate to severe asthmatic patients (Rolland et al., 2009). Therefore, new strategies are required that are capable to reconstitute regulatory responses in combination with allergen-specific immunotherapy. Since Treg cells are key players in maintaining homeostasis within the lung, enhancement of the proliferation, FoxP3 expression and the suppressive activity of Tregs in support of allergen-specific immunotherapy by selective inhibition of TNFR1 represents an attractive adjuvant therapeutic approach.

#### V.2.1. *Local selective inhibition of TNFR1 or TNFR1-mediated functions.*

In one embodiment of the present invention, local TNFR1 inhibition is used as adjuvant therapy in support of allergen-specific immunotherapy for the treatment of allergic asthma. Several small studies have suggested that anti-TNF-alpha therapy using the TNF-alpha-blocking antibody Infliximab or the soluble TNFR2 Etanercept, might be useful in reducing symptoms, exacerbations and airway hyper-responsiveness in patients with severe asthma (for a review, see Barnes, 2010), although a recent large multicenter trial with the fully human anti-TNF-alpha antibody Golimumab which also affects both TNFR1- and TNFR2-mediated signaling, did not show beneficial effects on lung function, symptoms and exacerbations (Wenzel et al., 2009). It should be noted, however, that specific local targeting of TNFR1-mediated functions in combination with allergen-specific immunotherapy as disclosed in the present invention represents a fundamentally different approach, since specific TNFR1 inhibition releases and favours the desired TNFR2-mediated functions at the site of allergen presentation. Furthermore, the enhancement of TNFR2-mediated functions is focused to the site of allergen presentation and, thereby, the reported Golimumab-induced side effects such as pneumonia and cancer (Wenzel et al., 2009) are avoided.

Selective inhibition of TNFR1-mediated signaling will also enhance an effector T cell (Teff)-mediated boost of Tregs as described in section V.1.1. Although the strong boost of the expansion of CD4+CD25+FoxP3+ Tregs and their suppressive function by antigen-specific activation of CD4+ effector T cells (Teff) has been observed in a murine model of type 1 diabetes (Grinberg-Bleyer et al., 2010), this feed-back mechanism represents most likely a general process in immune responses. Most important, the Treg boost has been demonstrated to be partially dependent on TNF-alpha via TNFR2-mediated signaling. Therefore, selective inhibition of TNFR1-mediated signaling is a promising approach to support the induction of tolerance.

#### V.2.2. *Combination of local TNFR1 inhibition with local inhibition of IL-4/IL-13 and/or local depletion of complement component C3.*

In another embodiment of the present invention, local TNFR1 inhibition used as adjuvant therapy in support of allergen-specific immunotherapy for treatment of allergic asthma, is combined with other local adjuvant treatments such as local inhibition of IL-4/IL-13 (see patent application EP 11075261.5) and/or local depletion of complement component C3 (see patent application EP 12075059.1) The combination of local inhibition of IL-4- and IL-13-mediated effects and local C3 depletion along with local inhibition of TNFR1-mediated functions and allergen stimulation of T cells represents a particularly promising therapeutic approach, since the combined approach allows also modulation of the functions of macrophages and DCs in a manner that favours the development of regulatory T cells with the concomitant reduction or inhibition of TH1 and TH2 immune responses. Furthermore, coincident blockade of IL-4/IL-13-mediated pathways provides an additional benefit for the use of alum-adsorbed allergens for specific immunotherapy (see section IV).

Preferred inhibitors of IL-4 and IL-13 are those which a) provide a small to moderate molecular size and high solubility in aqueous environments for fast diffusion into the immunological synapse, b) can be embedded in substantial quantities in matrices suitable for continuous release of the inhibitor over a prolonged period of time, c) are releasable from a depot-forming matrix in sufficient quantities to provide high local concentrations at the site of allergen presentation, and d) have a relatively short plasma half-life to reduce potential adverse effects mediated by interaction of the inhibitor with targets away from the site of allergen presentation. In a preferred embodiment of the invention, interleukin variants capable of antagonizing the activity of IL-4 and IL-13 are used as inhibitors. For example, the double mutant of human IL-4 (R121D/Y124D) has been shown to protect allergic cynomolgus monkeys from allergen-induced airways hyper-responsiveness when administered either subcutaneously or via nebulization. Furthermore, subcutaneous administration of this double mutant in monkeys for 6 weeks or more also reduced the cutaneous wheal response and circulating concentrations of allergen-specific IgE. The effect of the double mutant of IL-4 (R121D/Y124D) on late phase asthmatic response to allergen challenge in asthmatic patients has been evaluated recently in clinical studies (Wenzel et *al.,* 2007). The studies demonstrate that dual inhibition of IL-4 and Il-13 can positively affect the course of late asthmatic response after experimental allergen challenge. Treatment with the double mutant was associated with few adverse events, whether administered by subcutaneous injection (up to 30 mg for up to 13 weeks) or by inhalation (up to 60 mg for up to 4 weeks) in participants with atopic asthma or atopic eczema.

Local depletion of complement component C3 prior and along with allergen stimulation of T cells represents also a promising adjuvant approach for the therapy of allergic asthma. For example, using a mixture of *Aspergillus fumigatus* and OVA in a murine model of pulmonary allergy, C3 deficient mice were protected from airway reduction due to reduced airways responsiveness to inhaled methacholine (Drouin et al., 2001). Furthermore, in this study reduced levels of IL-4, antigen-specific IgE and IgG, and reduced eosinophil infiltration were observed. In another study, using the OVA model of antigen-induced airway hyper-responsiveness, near complete protection from the development of hyper-responsiveness to aerosolized methacholine was observed in C3aR deficient mice (Humbles et al., 2000). The same observation was made in a strain of guinea pigs with a natural C3aR defect (Bautsch et al., 2000). Using an OVA sensitization and challenge model of asthma, significant protection from airway broncho-constriction following antigen challenge (in contrast to airway hyper-responsiveness to methacholine) was noticed. In the absence of C3a-C3aR interactions, bone marrow-derived DCs exhibit also a less activated phenotype with reduced capacity for antigen uptake/presentation and lowered ability to stimulate TH1 responses (i.e. IL-12 production, generation of IFN-γ producing T cells) *in vitro* and *in vivo* (for a review, see Zhou, 2012). As a result of the reduced ability of macrophages and DCs to stimulate TH1 responses in the absence of C3a-C3aR interactions, immune responses could shift potentially to TH2 responses as suggested by studies with C3aR deficient mice (Kawamoto et al., 2004). In C3aR deficient mice the immune response upon epicutaneous introduction of antigen has been demonstrated to shift towards TH2 responses, with significantly higher levels of serum IgG1 and lower levels of serum IgG2a, IgG3, and IgA compared with wild-type mice (Kawamoto et al., 2004). However, the potential shift towards TH2 responses under C3-depleted conditions is inhibited by two mechanisms, by C5a-C5aR interactions and by inhibition of IL-4- and IL-13-mediated effects along with allergen stimulation of T cells.

There is also evidence for an important role of C5a in the pathogenesis of allergic asthma. Using a rat model, a synthetic hexapeptide C5a antagonist as well as inhibition of complement activation by a soluble CR1-derivative blocked the inflammatory response and airway hyper-responsiveness to methacholine (Abe et al., 2001). Furthermore, deficiency of C5 or C5aR has been shown to inhibit TH2 immune responses in asthma, thus providing protection to antigen challenged mice (Drouin et al., 2006; Köhl et al., 2006). Coincident blockade of IL-4/IL-13-mediated pathways is important, since both TH2-related cytokines are known to play key roles in the pathogenesis of asthma and other allergic diseases.

Restoring clinical tolerance to allergen(s) is associated with the re-induction of allergen-specific Treg cells, and the generation of Tregs represents a default pathway that requires T-cell receptor activation as for any other T-cell differentiation, but the absence of decision signals for effector T cells. Therefore, reduction or inhibition of TH1 and TH2 immune responses in the absence of C3a-C3aR and C5a-C5aR interactions, the presence of an IL-4/IL-13 antagonist, and enhanced TNFR2/IL-2-mediated stimulation of the proliferation, FoxP3 expression and suppressive activity of Tregs is extremely promising. Furthermore, dendritic cells from $C3^{-/-}$ mice have been demonstrated to effect in vitro a significant increase of the number of Tregs (Peng et al., 2006).

In conclusion, the combination of local inhibition of IL-4/IL-13-mediated effects and local C3 depletion along with local inhibition of TNFR1-mediated functions and allergen stimulation of T cells has the potential to improve the development of tolerance significantly and, thereby, to reverse the pathological processes in asthma.

## V.3. TREATMENT OF DIABETES TYPE I: LOCAL TNFR1 INHIBITION AS ADJUVANT THERAPY FOR AUTOANTIGEN-SPECIFIC IMMUNOTHERAPY

Type 1 diabetes (previously known as juvenile diabetes) is a T cell-dependent autoimmune disease in which islet antigens from insulin-producing beta-cells of the pancreas are presented by APCs to autoreactive T cells, breaking self tolerance. Expansion of autoreactive CD4+ and CD8+ T cells, autoantibody-producing B cells and activation of the innate immune system cause beta-cell injury (Bluestone et al., 2010).

The appearance of diabetes-related autoantibodies has been shown to be able to predict the appearance of diabetes type 1 before any hyperglycemia arises, the main ones being islet cell autoantibodies, insulin autoantibodies, autoantibodies targeting the 65 kDa isoform of glutamic acid decarboxylase (GAD65) and autoantibodies targeting the phosphatase-related IA-2 molecule (Knip et al., 2005). The emergence of autoantibodies may be termed 'latent autoimmune diabetes', since not everyone with autoantibodies develops diabetes type 1. However, the risk increases with the number of autoantibody types and the presence of three to four antibody types is associated with a risk of progressing to type 1 diabetes of 60% to 100%. The time interval from emergence of autoantibodies to diabetes type 1 can be a few months in infants and young children, but in some people it may take years (Knip et al., 2005). Only after destruction of approximately 90% of the β-cells, type 1 diabetes becomes clinically manifest (for a review, see Homann and von Herrath, 2004). It has been proposed that diabetes Type 1 might be prevented at the latent autoimmune stage, before it starts destroying beta-cells (Bluestone et al., 2010).

Type 1 diabetes causes an estimated 5-10% of all diabetes cases or 11-22 million worldwide. The incidence of type 1 diabetes has been increasing by about 3% per year. In 2006, it affected 440,000 children under 14 years of age and was the primary cause of diabetes in those less than 10 years of age. However, the majority of new-onset type 1 diabetes is seen in adults. Adult-onset type 1 diabetes is two to three times more common than classic childhood-onset type 1 diabetes (http://en.wikipedia.org/wiki/Diabetes_mellitus_type_1).

Eventually, type 1 diabetes (autoimmune diabetes) is fatal unless continuously treated with insulin. Pancreatic islet cell transplantation is performed, but serious limitations are associated with this kind of treatment. Immunotherapy with anti-CD3 antibodies, including the humanized anti CD3 mAb Teplizumab (mutated to prevent binding to Fc receptors) and anti CD3 mAb Otelixizumab (similarly Fc-mutated), has provided evidence for preserved insulin production in newly diagnosed type 1 diabetes patients (Bluestone et al., 2010), but phase III studies with both antibodies failed to show clinical efficacy (Tolerx, 2011; MacroGenics, 2011), potentially due to an insufficient dosing schedule. The anti-CD20 antibody Retuximab inhibits B cells and has been shown to provoke C-peptide responses (evidence for insulin production) three months after diagnosis of type 1 diabetes, but long-term effects of this treatment have not been reported (Bluestone et al., 2010).

Adverse effects of the therapeutic approaches with broad immune-suppressive agents as well as the lack of permanent remission of disease with any agent tested to date have boosted interest in the development of antigen-specific interventions. Such an approach may require only a single beta-cell-derived antigen despite the polyclonal nature of the autoimmune response, reflected by the presence of multiple autoantibodies and multiple T cell epitopes that are recognized by peripheral blood cells. Immunological tolerance mechanisms, even by antigen-specific cells, are not restricted to a single antigen. Cytokines such as IL-10 and TGF-beta, produced by T cells in response to antigen or antigen-presenting cells, can modulate the function of effector T cells in the vicinity of the antigen. Thereby, activated regulatory T cells can exert their function at the site of the immune response without the need to recognize those antigen(s) recognized by effector T cells. In addition, CD4+ CD25+ FoxP3+ and other regulatory T cells are able to modulate the function of effector T cells through contact-dependent mechanisms. It has been demonstrated that polyclonal diabetogenic T cells can be inhibited by antigen-specific Treg cells in vitro and in an adoptive transfer model of diabetes (for a review, see Bluestone et al., 2010).

A promising approach is the vaccination with the 65 kDa isoform of glutamic acid decarboxylase (GAD65). Immunization with alum-GAD65 was shown to attenuate the loss of C-peptide in individuals treated within six months of diagnosis (Ludvigsson et al., 2008). Furthermore, oral insulin administration yielded also promising results in a subset of patients with high levels of insulin autoantibodies (Skyler et al., 2005), an observation that is now being studied in detail. These data support the concept that an antigen-specific intervention may have broad immunological effects, provided the selected antigen is directly involved in the disease pathogenesis and/or regulation. Phase III trials with alum-GAD65 are underway in the USA and in Europe (Diamyd, 2011a and 2011b). In addition, prevention studies are underway in which this vaccine is given to persons who have not yet developed type 1 diabetes (Diamyd, 2011c). The method of the present invention aims to support the efficacy of vaccination with self-antigens by targeting of the molecular mechanisms of self-antigen tolerance and reciprocal regulation of effector and regulatory T cells.

### V.3.1. *Local selective inhibition of TNFR1 or TNFR1-mediated functions.*

In one embodiment of the present invention, methods are disclosed which support the self-antigen vaccination approach by local selective inhibition of TNFR1 or TNFR1-mediated functions. It is known for a long time that prolonged administration of antigens in the absence of pro-inflammatory stimuli could lead to the induction of tolerance and that part of this in vivo tolerance induction is mediated by CD4+CD25+FoxP3+ Tregs being induced from naive T cells (for a review, see Jaeckel et al., 2008). With regard to type 1 diabetes development, compelling evidence indicates that the loss of control of the autoimmune response in by FoxP3+ Tregs is a major contributing factor (for a review, see Li et al., 2012). There is evidence that human patients with diabetes type 1 have a reduced frequency or functionality of CD4+ CD25+ regulatory T cells (Kukreja et al., 2002). The necessity for increasing the number of CD4+ $CD25^+$ regulatory T cells in patients with type I diabetes is supported by the observation that several therapeutic approaches which protect from diabetes, are correlated with an increase in CD4+ CD25+ regulatory T cells (for a review, see Juedes and von Herrath, 2004). For example, increased numbers of circulating CD4+ CD25+ regulatory T cells have been reported in patients receiving successful islet transplants and were treated with the anti-CD3 mAb hOKT3$\gamma$1(Ala-Ala) (Hering et al., 2004). Important for therapeutic approaches is the observation that human CD4+ CD25+ regulatory T cells may also be generated in the periphery after activation of CD4+ $CD25^-$ T cells (Akbar et al., 2003; Walker et al., 2003). It has also been reported that adaptive Tregs elicited from murine CD4+ $CD25^-$ T cells can persist as CD25- Treg cells despite expressing FoxP3 (Godebu et al., 2008).

Specific local inhibition of TNFR1 is likely to support the generation of CD4+ CD25+ regulatory T cells at the site of self-antigen presentation, since the resulting enhancement of TNFR2-mediated functions promotes Treg expansion (for reviews, see Chen and Oppenheim, 2010; Biton et al., 2012). This assumption is in agreement with the observation that mice deficient in TNF-alpha (Genetic Knock-Out (KO)) or treated with neutralizing antibody) developed various immune diseases including diabetes, whereas TNF-alpha administration has therapeutic effects in these diseases (Kassiotis and Kollias, 2001). The immunoregulatory role of TNF-alpha has been explained by its capacity to boost Treg activity.

Recently, it has been demonstrated that antigen-specific activation of CD4+ effector T cells (Teff) strongly boosts the expansion of CD4+CD25+FoxP3+ Tregs and their suppressive function (Grindberg-Bleyer et al., 2010). This helper function of CD4+ T cells was observed in the pancreas and draining lymph nodes in mouse recipients of islet-specific Teffs and Tregs. When islet-specific Teffs were transferred alone, they induced diabetes. When the same Teffs were co-transferred with islet-specific Tregs, they induced disease protection by boosting Treg expansion and suppressive function. Thus, diabetogenic Teffs help islet-specific Tregs to provide sustained protection from diabetes. RNA microarray analyses suggested that TNF family members are involved in the Teff-mediated Treg boost. In vivo experiments showed that this Treg boost is partially dependent on TNF-alpha but not on IL-2 (Grindberg-Bleyer et al., 2010). Again, the TNF-alpha-mediated effect has been explained by its capacity to boost Treg activity. In the Teff-mediated boost of Tregs, IL-2 was necessary to maintain Treg survival and was thus critical to preserving a high number of boosted Tregs, but was clearly not involved in the increased Treg proliferation mediated by Teffs. On the other hand, TNF-alpha is clearly involved, since blocking of TNF-alpha with soluble TNFR2 (Etanercept) or with anti-TNF-alpha monoclonal antibody XT3.11 significantly reduced the Teff-mediated Treg boost without inhibiting Teff activation (Grindberg-Bleyer et al., 2010).

Thus, during an autoimmune process such as type 1 diabetes, there is a local enrichment of both autoreactive Teffs and Tregs. Since the efficacy of Treg-mediated suppression depends on the equilibrium between activated Teffs and activated Tregs, any factor that could tip this balance to one side or the other could then determine the outcome of the autoimmune process. By specific inhibition of TNFR1-mediated pro-inflammatory effects at the site of allergen presentation, TNFR2-mediated up-regulation of CD25 (alpha-chain of the IL-2 receptor) expression on Tregs is favoured and, as a result of enhanced Il-2-mediated functions, up-regulation of TNFR2 expression on Tregs inducing a boost of Treg activity.

V.3.2. *Combination of local TNFR1 inhibition with local depletion of complement component C3 and/or local inhibition of IL-4/IL-13.*

In another embodiment of the present invention, local TNFR1 inhibition used as adjuvant therapy in support of self-antigen vaccination for treatment of type I diabetes, is combined with other local adjuvant treatments including but not limited to local depletion of complement component C3 (see patent application EP 12075059.1) and/or local inhibition of IL-4/IL-13 (see patent application EP 11075261.5).

Based on recent evidence, autoimmune processes such as diabetes type 1 are composed not only of autoaggressive T cell responses, but also of autoreactive regulatory components. Therefore, successful approaches for immunotherapy of type 1 diabetes obviously require enhancement of regulatory T cell responses and inhibition of autoreactive CD8+ T cells responses.

Animal studies have provided strong evidence that complement component C3 is required for priming and expansion of CD8+ T cells. For example, primed CD8+ T cell responses to allogeneic endothelial cells are controlled by local complement activation (Raedler et al., 2009), and activation as well as expansion of CD8+ T cells during a systemic viral infection was markedly reduced in C3-deficient mice (Suresh et al., 2003. Furthermore, priming of CD8+ T cells requires help from CD4+ T cells, and local complement has been shown to regulate the help of CD4+ cells for CD8+ T cells required for murine allograft rejection (Vieyra et al., 2011). Cognate interactions between CD4+ T cells and dendritic cells (DCs) induce C3a and C5a production and CD4-cell help is mediated, in part, by interaction of DC-derived C3a/C5a with CD8+ T cell-expressed C3aR/C5aR. This concept is supported by two observations. First, CD8+ T cells lacking C3aR and C5aR proliferate weakly to allogeneic DCs despite CD4 help. Second, augmented production of C3a/C5a by DCs bypasses the requirement for CD4- and CD40-dependent help to wild-type CD8+ T cells (Vieyra et al., 2011).

The impact of these observations for type I diabetes is evident from another recent animal study (Munegowda et al., 2011). The data of this study demonstrate that both CD4+TH17-cells and TH17-stimulated CD8+ cytotoxic T cells are involved in type I diabetes in mice, whereas induction of type I diabetes required only TH17-stimulated CD8+ T cells. This observation is in accordance with the recently described role of human islet-autoreactive CD8+ T cells in the pathogenesis of type I diabetes in humans (Coppieters et al., 2012). Although the role of local complement activation for TH17-mediated stimulation of CD8+ T cells has not been investigated in this study, the mechanisms of activation are most likely to be identical with those observed in other immune responses. In animal studies, immune cell-derived C3, C3a- and C5a-receptors have been demonstrated to be essential for the development of T cell-dependent type 1 diabetes induced by multiple low doses of streptozotocin (Lin et al., 2010). Therefore, local depletion of complement component C3 prior and along with self-antigen stimulation of T cells provides significant potential to support the induction of clinical tolerance to self-antigens.

Local inhibition of IL-4-mediated effects may contribute to the reduction of activated autoreactive CD8+ cytotoxic T cells via local C3-depletion at the site of self-antigen presentation, since IL-4 has been shown to be crucial for the priming of antigen-specific CD8+ T cells after vaccination with the Leishmania antigen HASPB-1 (Stäger et al., 2003). Further analyses revealed that immune complexes formed by reaction of natural antibodies with the Leishmania antigen causes complement activation, which in turn stimulates primary IL-4 secretion from CD11b+ CD11c+ phagocytes. In the presence of specific antibodies to ovalbumin (OVA), OVA injection induced IL-4 secretion comparable to that induced by the Leishmania antigen. Therefore, the authors concluded that immune complexes can, in general, provide a means of stimulating primary IL-4 secretion from CD11b+ CD11c+ phagocytes. Complement activation is essential for this process since in Clq-deficient mice neither IL-4 secretion nor priming of antigen-specific CD8+ T cell could be observed (Stäger et al., 2003).

Since in type 1 diabetes patients undergoing vaccination therapy, circulating auto-antibodies will also form immune complexes with injected self-antigens, local C3 depletion and inhibition of IL-4-mediated effects represents a promising approach to reduced priming of harmful autoreactive CD8+ cytotoxic T cells.

V.3.3. *Combination of self-antigen vaccination and local TNFR1 inhibition with anti-CD3 therapy.*

Several studies have provided evidence that Treg cells are selectively preserved by treatment with anti-CD3 monoclonal antibodies (for a review, see Bluestone et al., 2010). In vitro, anti-CD3 mAbs have been shown to induce FoxP3 and CD25 expression and regulatory function on $CD4+CD25^-$ T cells (Walker et al., 2003), and immunotherapy with anti-CD3 antibodies, including Teplizumab (Herold et al., 2002) and Otelixizumab (Keymeulen, 2005), has provided evidence for preserved insulin production in newly diagnosed type 1 diabetes patients.

Currently, anti-CD3 therapy is one of the most promising approaches for increasing the number of CD4+ $CD25^-$ regulatory T cells in patients with type I diabetes. However, supportive adjuvant therapeutic approaches are required to achieve a lasting remission. Coincident suppression of priming and expansion of islet-autoreactive CD8+ cytotoxic T cells appears to be important, since a very recent study has provided first time evidence for islet-autoreactive CD8+ T cells in insulitic lesions from recent-onset and long-term type 1 diabetes patients, pointing to the CD8+ T cells as being one of the most important cells in type I diabetes progression (Coppieters et al., 2012). Tolerization of these CD8+ T cell species may thus be a requirement to achieve long-lasting remission. At least for some patients, however, therapeutic tolerization will require a combination of treatment modalities, since the functionality of CD4+ CD25+ regulatory T cells can be impaired in human patients with diabetes type 1. As a result, the regulatory functions of Tregs may not be sufficient in some type I diabetes patients to efficiently suppress priming and expansion of autoreactive CD8+ cytotoxic T cells.

V.3.4. *Combination of self-antigen vaccination and local TNFR1 inhibition with depletion of complement component C3, local inhibition of IL-4/IL-13 and anti-CD3 therapy.*

In another embodiment of the present invention, local TNFR1 inhibition used as adjuvant therapy in support of self-antigen vaccination for treatment of type I diabetes, is combined with anti-CD3 therapy and additional local adjuvant treatments including but not limited to local depletion of complement component C3 (see patent application EP 12075059.1), local inhibition of IL-4/IL-13 (see patent application EP 11075261.5).

### V.4. TREATMENT OF RHEUMATOID ARTHRITIS: LOCAL TNFR1 INHIBITION AS ADJUVANT THERAPY FOR AUTOANTIGEN-SPECIFIC IMMUNOTHERAPY

Rheumatoid arthritis (RA) is characterized by inflammatory reactions in the synovial membranes and articular structures of joints. Approximately 1% of the general population is believed to be affected with peak incidence of onset during the $4^{th}$ and $5^{th}$ decades of life. Despite new treatment modalities and more intensive therapeutic strategies, in most cases RA remains a chronic and progressive disease. The challenge for novel treatments is to specifically inhibit immune cells initiating and perpetuating RA-related inflammation and tissue destruction and to change the disease without causing severe side effects and/or general immunosuppression.

Important evidence for the involvement of TNF-alpha in RA came from studies with mice over-expressing human TNF-alpha and spontaneously developing polyarthritis. Successful treatment of these mice with an anti-TNF-alpha antibody demonstrated the detrimental role of TNF-alpha in this model. Currently, different mouse models are used to study arthritis. One model of TNF-alpha-induced arthritis is the TNFdARE mouse. In this mouse, deletion of the ARE region in the 3'UTR of the TNF-alpha mRNA stabilizes the mRNA and, thereby, causes continuous production of TNF-alpha. These animals develop spontaneous poly-arthritis and small bowel inflammation resembling Crohn's disease. When crossed with TNFR1 deficient mice, TNFdARE mice do not develop arthritis, but crossing with TNFR2 KO mice leads to exacerbation of disease progression. This indicates that TNFR1-mediated signaling is pathologic, whereas TNFR2-mediated signaling induces anti-inflammatory effects.

In another arthritis model, collagen-induced arthritis (CIA), TNF-alpha plays an important but not a dominant role. However, anti-TNF-alpha therapy is also effective in these mice. Therapeutic inhibition of TNFR1 has shown some success in the CIA model. For example, specific inhibition of TNFR1 with the TNF-alpha mutant R1antTNF was effective in reducing the clinical symptoms (Shibata et al., 2009).

To understand the role of sTNF-alpha in arthritis, mice expressing an uncleavable transmembrane TNF-alpha (mTNT-alpha) were tested in a TNF-alpha-dependent model of arthritis (Alexopoulou et al., 2006). The mice expressing only mTNF-alpha were resistant to arthritis, indicating that sTNF-alpha is essential for the development of this disease. This observation was confirmed by specific therapeutic inhibition of sTNF-alpha in experimental arthritis by a dominant negative TNF-alpha mutant (Zalevsky et al., 2007).

Anti-TNF therapy has been shown to ameliorate clinical symptoms and laboratory parameters of inflammation in the majority of RA patients, although treatment with total TNF-alpha blockers (e.g., Infliximab, Adalimumab, Etanercept) attenuates TNFR1-mediated inflammatory responses and at the same time inhibits TNFR2-mediated anti-inflammatory effects. A recent study showed that anti-TNF-alpha therapy in experimental arthritis expanded pathogenic Th1 and Th17 cells in the lymph nodes, mediated by up-regulation IL-12/IL-23p40 expression. However, anti-TNF-alpha therapy blocked the accumulation of Th1/Th17 cells in the synovium, thereby providing an explanation for the paradox that anti-TNF-alpha therapy ameliorates experimental arthritis despite increasing the number of pathogenic T cells in lymph nodes (Notley et al., 2008).

Different effects of TNF-alpha on Tregs and Teffs may also contribute to the beneficial therapeutic effects of anti-TNF-alpha therapy. Tregs are often increased at the site of RA-related inflammation, presumably resulting from active recruitment and in situ proliferative expansion. High levels of TNFR2 expression by Tregs mediated by chronic inflammatory responses may enable Tregs to outcompete other TNFR2-expressing cells for TNF-alpha and, thereby, to accumulate. However, despite the increased presence of suppressive Tregs the inflammation in joints of RA patients is ongoing, possibly due to the interaction of TNF-alpha with TNFR2 expressed by effector T cells (Teffs). Expression of TNFR2 by Teffs is also up-regulated upon TCR stimulation (Chen et al., 2007), which may allow the TNF-alpha signal mediated by TNFR2 to effectively stimulate Teffs and, thereby, render Teffs more resistant to Treg-mediated inhibition. The observation that higher concentrations of TNF-alpha down-regulated Treg suppressive activity (Valencia et al., 2006) is likely to reflect a dose-dependent stimulating effect of TNF-alpha on Teffs. Thus, the increasing resistance of Teffs to Treg-mediated inhibition may be the reason for pro-inflammatory effects of TNF-alpha. In view of different effects of TNF-alpha on Tregs and Teffs, the therapeutic effect of anti-TNF-alpha therapy is probably achieved by eliminating the TNF-alpha/TNFR2 costimulation pathway on activated pathogenic Teffs which most likely express elevated levels of TNFR2.

It should be mentioned, however, that anti-TNF therapy of RA patients has also been demonstrated to result in the generation of a distinct population of suppressor cells derived from naive CD4+ cells. The suppressive activity of these induced cells which lack CD62L expression, proved to be dependent on IL-10 and TGF-beta (Nadkarni et al., 2007). Therefore, these induced suppressor cells are assumed to be Tr1 and/or Th3 cells.

However, despite the beneficial therapeutic effects of total TNF-alpha blockers for RA patients, long-term use of these inhibitors can cause serious side effects including an increased incidence of infections. For example, in a retrospective analysis of 709 patients treated with at least one TNF-alpha blocker, 34.5% of patients reported infectious complications during the treatment period, with 6.2% falling under the category of serious infections (for a review, see Ichim et al., 2008). The present invention avoids these risks by disclosing methods for selective inhibition of TNFR1-mediated functions and matrices for local delivery of TNFR1 specific inhibitors. Thereby, beneficial mTNF-alpha/TNFR2 interactions are retained and potential systemic side effects are minimized.

Since clinical inhibition of inflammatory cytokines does not lead to a long-term cure, various approaches for the induction of autoantigen-specific tolerance have been tested (for a review, see Ichim et al., 2008). Although immune suppressive epitopes of collagen II have been identified in animal models that induce protection from disease, the majority of clinical work has been performed targeting the autoantigen hsp 60. A clinical trial using hsp 60 peptides demonstrated clinical remission in patients with juvenile RA (Kamphuis et al., 2005). Recently, a multicentre, double-blind placebo-controlled autoantigen-specific immunotherapy study was performed using the human cartilage glycoprotein-39 (HC gp-39) (Landewe et al., 2010). HC gp-39 was administered via the intranasal route since the nasal mucosa is currently regarded as the most powerful route for induction of immunological tolerance (Higuchi et al., 2000). Although HC gp-39 has been identified as a potential key autoantigen in RA, the used treatment protocol did not result in more clinical improvement than in placebo-controlled patients.

Based on these examples it becomes clear that autoantigen-specific immunotherapy is feasible, but in its present state the antigen-specific suppression is too weak for widespread clinical implementation. In order to develop more potent protocols, there is a need for concomitant inhibition of effector T cells and the induction of regulatory T cells along with self-antigen presentation.

V.4.1. *Local selective inhibition of TNFR1 or TNFR1-mediated functions.*

In one embodiment of the present invention, methods are disclosed which support the self-antigen vaccination approach by sustained local selective inhibition of TNFR1-mediated functions at the site of self-antigen presentation.

Although synovial fibroblasts have been identified as the primary targets for TNF-alpha in the development of arthritis, the site for selective inhibition of TNFR1-mediated functions does not appear to be critical for the therapeutic effect. As demonstrated in a recent study, systemic treatment with an adenoviral vector containing a short hairpin RNA directed against TNFR1 ameliorated collagen-induced arthritis (CIA) in mice almost to the same extent as local treatment (Arntz et al., 2010). Furthermore, TNFR1 silencing in knee joints also protected the ipsilateral ankle joints in CIA mice. Based on these observations, local inhibition of TNFR1-mediated functions at the site of self-antigen presentation has the potential to induce systemic tolerance.

V.4.2. *Combination of local TNFR1 inhibition with local depletion of complement component C3 and/or local inhibition of IL-4/IL-13.*

In another embodiment of the present invention, sustained local TNFR1 inhibition used as adjuvant therapy in support of self-antigen vaccination, is combined with other sustained local adjuvant treatments including but not limited to sustained local depletion of complement component C3 (see patent application EP 12075059.1) and/or sustained local inhibition of IL-4/IL-13 (see patent application EP 11075261.5) at the site of self-antigen presentation.

Given the central role of dendritic cells (DCs) in stimulating naive T cell responses and also generating T cells with regulatory functions, means of manipulating DCs towards a tolerogenic phenotype appears to be a promising approach. Local depletion of complement component C3 provides a way to reduce the functions of immune stimulatory DCs and, thereby, to tip the balance more to a tolerogenic phenotype.

Several human dendritic cell subsets (e.g., monocyte derived, dermal, Langerhans, myeloid, plasmacytoid) express C3a and/or C5a receptors (C3aR; C5aR, CD88), and both C3a and C5a, which are present in the DC environment, can up-regulate TH1 responses by modulating DC activation and function (for a review, see Zhou, 2012). In the absence of C3a-C3aR interactions, the functions of bone marrow-derived DCs were impaired, resulting in a less activated phenotype with reduced capacity for antigen uptake/presentation and lowered ability to stimulate TH1 responses (i.e. IL-12 production, generation of IFN-$\gamma$ producing T cells) in vitro and in vivo. Accordingly, stimulation of C3aR with C3a increased the capacity of DCs for antigen presentation and enhanced their ability to stimulate allospecific T cell responses (Peng et al., 2008; Li et al., 2008). Similar observations have been made for C5a-C5aR interactions (for a review, see Zhou, 2012).

Furthermore, a recent study has demonstrated that the interaction of serum-derived C5a with immune cell-expressed C5aR is an essential mediator in an IL-17-dependent model of autoimmune arthritis (Hashimoto et al., 2010).

Collectively, these data demonstrate that activation of C3 and C5 plays also an important role in the regulation of autoreactive T cells (for a review, see Heeger and Kemper, 2012). Accordingly, complement deficiency or blockade effects attenuation of T cell-mediated autoimmunity (for a review, see Kwan et al., 2012). Local blockade of the complement system is achieved by local depletion of complement component C3, since in the presence of depleted C3 the local generation of C3 and C5 convertases is greatly reduced or even negligible, resulting in a significantly decreased concentration of activated C3- and C5-fragments at the site of self-antigen presentation.

Concomitant inhibition of IL-4-mediated functions may also provide benefits for RA patients, since IL-4 inhibits the expression of FOXP3, which is a requirement for inducible Treg (iTreg) differentiation.

## V.5.TREATMENT OF MULTIPLE SCLEROSIS (MS): LOCAL TNFR1 INHIBITION AS ADJUVANT THERAPY FOR AUTOANTIGEN-SPECIFIC IMMUNOTHERAPY

Worldwide, MS may affect 2.5 million individuals and it is the most common disease affecting young adults. MS is thought to occur in genetically predisposed individuals following exposure to an environmental trigger that activates myelin-specific T cells. To initiate central nervous system (CNS) inflammation, myelin-specific T cells must gain access to the CNS after activation in the periphery. Tight junctions between the endothelial cells of the blood-brain barrier and the epithelial cells of the blood-cerebrospinal fluid barrier limit access to the CNS by circulating cells. However, activated and memory T cells can carry out immune surveillance of the CNS because they express adhesion molecules, chemokine receptors and integrins that allow them to cross the barrier. Reactivation of the T cells by CNS-resident antigen presenting cells (APCs) that present myelin antigens, triggers the recruitment of innate immune cells, which have important roles in mediating demyelination and axonal damage.

MS has been widely studied using the animal model of experimental autoimmune encephalomyelitis (EAE) which is induced by immunization with myelin-derived antigens in adjuvant or by the adoptive transfer of activated myelin-specific T cells. The inflammatory infiltrates and demyelination seen in EAE have many similarities to the pathology of MS. Myelin basic protein (MBP) and the myelin-derived proteolipid protein (PLP) are the most abundant proteins in the myelin sheath in the CNS and were the first to be identified as CD4+ T cell targets in the initial subsets of mouse strains that exhibited susceptibility to EAE. Susceptibility to EAE requires not only the expression of an MHC class II molecule that can bind at least one peptide derived from a myelin antigen, but also that some myelin-specific T cells escape central tolerance so that they are available for activation in the periphery. Central tolerance is the process in which T cells that exhibit high avidity interactions with APCs presenting self-antigen-MHC complexes in the thymus are deleted. High avidity, however, is a function of both the affinity of the T cell receptor (TCR) for the self-antigen-MHC complex and the abundance of these complexes on the surface of the APC. The observation that many myelin proteins mediate central tolerance suggests that myelin-specific T cells which escape to the periphery are mostly T cells with low avidity for self-antigens (for a review, see Goverman, 2009). The low avidity of these T cells for self-antigen normally prevents them from engaging self-antigen in the periphery and allows them to circulate without inducing damage. However, the avidity of T cells for self-antigen can be increased under certain circumstances. For example, APCs responding to an immunogenic stimulus such as an infection increase their expression of co-stimulatory molecules and MHC complexes on the cell surface. These changes can result in an increase in the avidity of the interaction between APC and self-reactive T cells. Thereby, T cells with low avidity for self-antigens can now respond to the self-antigen that they previously ignored.

Several studies have described TCRs that recognize both myelin antigens and pathogen-derived epitopes (for a review, see Goverman, 2009). According to the molecular mimicry hypothesis, the more T cells with this type of degenerate recognition an individual has, the greater the chance that T cells activated in the response to a specific pathogen will initiate an autoimmune response against a myelin antigen. Support for this hypothesis is based on the observation that the incidence of spontaneous EAE which develops in one model of MBP-specific TCR-transgenic mice increases as the level of microbial exposure increases.

MS and its animal model EAE have long been regarded as primarily T helper cell type 1-mediated diseases. However, recent evidence suggests that CD4+Th17 cells may be even more pathogenic than CD4+Th1 cells. In the EAE model, this cell type is crucial for the recruitment of leukocytes into the CNS and for triggering parenchymal inflammation. In humans, Th17 cells are found in acutely active and on the borders of chronically active lesions. Furthermore, recent studies point also to the potential importance of CD8+ T cells in CND autoimmunity. One compelling reason for this is that depletion of CD4+ T cells in patients with MS resulted in no improvement in disease, whereas treatment with a monoclonal antibody which depletes several leukocyte populations including CD4+ and CD8+ T cells seemed to be beneficial. CD8+ T cells also outnumber CD4+ T cells in multiple sclerosis lesions (for a review, see Goverman, 2009).

The function of CD4+FoxP3+ Tregs in preventing inflammation within the CNS is controversial. In myelin-specific CD4+TCR transgenic mouse models, FoxP3+ regulatory T cells prevent spontaneous EAE by suppressing the activation of myelin-specific CD4+ T cells in the periphery. Furthermore, a correlation between the presence of IL-10-proucing FoxP3+ Treg cells in the CNS and disease recovery has been reported (McGeachy et al., 2005). However, Tregs seem ineffective in suppressing effector T cells in the CNS until the local levels of IL-6 and TNF-alpha decrease (for a review, see Goverman, 2009).

Regulatory CD8+ T cells have also been reported to be present in EAE and patients with MS (for a review, see Zozulya and Wiendl, 2008). Such regulatory CD8+ T cells include naturally occurring CD8+CD122+ T cells that inhibit CD8+ effector T cells by secreting IL-10, and induced regulatory CD8+ T cells that are thought to function in the periphery. CD8+ T cells were detected in patients with MS that lysed CD4+ myelin-specific T cells and, importantly, their numbers were decreased during exacerbation of the disease. Since there is also evidence that human patients with multiple sclerosis have a reduced frequency or functionality of CD4+ CD25+ regulatory T cells (Viglietta et al., 2004), strategies for enhancing CD8+ as well as CD4+ regulatory T cells in vivo may be beneficial of the treatment of patients with MS. The present invention discloses methods that support the enhancement of Treg activity in combination with self-antigen vaccination.

Several attempts have been made to apply self-antigen-specific immunotherapy in MS patients including DNA vaccines encoding myelin proteins (e.g., Garren et al., 2008), myelin proteins and myelin protein-derived peptides (for a review, see Steinman and Zamvil, 2010). Key autoantigens in MS include myelin basic protein (MBP), proteolipid protein (PLP) and myelin oligodendrocyte glycoprotein (MOG). In clinical trials, oral application of myelin proteins (Faria and Weiner, 2005) and intravenous application of MBP (Warren et al., 2006) showed no or only modest effects on MS disease course. However, a recent clinical trial in 30 relapsing-remitting MS patients with transdermally applied peptides derived from three myelin proteins (MBP. PLP, MOG) induced immune tolerogenic effects (Jurynczyk et al., 2010). The transdermally applied peptides activated dendritic Langerhans cells in the skin at the site of immunization, induced a unique population of granular dendritic cells in local lymph nodes, and generated type 1, IL-10-producing Tregs in the periphery. Repeated transdermal application of these peptides (weekly in the first month and then monthly for the remainder of the year) resulted in suppression of specific autoreactive proliferative responses and suppression of interferon-gamma and TGF-beta production.

Application of fusion proteins containing GM-CSF coupled to myelin auto-antigens for immunotherapy of EAE in mice (Abbott et al., 2011) have demonstrated that self-antigen-based vaccination approaches can be optimized by combining self-antigen presentation with immune response-modulating agents. GM-CSF is recognized as a potent regulatory cytokine able to ameliorate disease in several mouse models of autoimmunity. For example, GM-CSF drives differentiation of dendritic cells (DCs), including tolerogenic DC subsets that in turn facilitate Treg differentiation and antigen-specific tolerance. In mice with EAE, a fusion protein of GM-CSF and the encephalitogenic MOG35-55 peptide facilitated tolerance rather than immunity to dominant self-epitopes of myelin (Abbott et al., 2011). In patients, however, therapeutic applications of GM-CSF can be associated with serious side effects such as the vascular leak syndrome. Therefore, alternative approaches are needed that employ more suitable enhancers of self-antigen-specific immunotherapy. The present invention discloses methods for optimizing self-antigen-based vaccination approaches in patients with MS which are based on selective TNFR1 inhibition at the site of self-antigen presentation.

V.5.1. *Local selective inhibition of TNFR1 or TNFR1-mediated functions.*

In one embodiment of the present invention, methods are disclosed which support the self-antigen vaccination approach by local selective inhibition of TNFR1-mediated functions. Although TNF-alpha is involved in the initiation of MS, its presence in later stages is essential for resolving the inflammation and initiating repair. Mice deficient in TNF-alpha (KO or treated with neutralizing antibody) develop EAE, whereas TNF-alpha administration has been demonstrated to provide therapeutic effects (Kassiotis and Kollias, 2001). Also, TNF-alpha neutralization in patients with MS exacerbated the disease (The Lenercept Multiple Sclerosis Study Group and The University of British Columbia MS/MRI Analysis Group, 1999).

Of interest for therapeutic design is that the two opposing functions of TNF-alpha diverge at the level of TNF receptors. In the murine EAE model, TNFR1 was found to be responsible for the detrimental signals, whereas TNFR2 was found to be essential for resolving inflammation and initiating repair (Kassiotis and Kollias, 2001). Accordingly, EAE was exacerbated in TNFR2-KO mice and reduced in TNFR1-KO mice (Suvannavejh et al., 2000). Interestingly, mutant mice expressing uncleavable transmembrane TNF-alpha (mTNF-alpha) were protected against autoimmunity, indicating that sTNF-alpha/TNR1 interactions are responsible for the pathology (Alexopoulou et al., 2006). In agreement with these data, specific inhibition of TNFR1 by a pegylated mutant TNF molecule (PEG-R1antTNF) was shown to ameliorate the clinical symptoms in the EAE model (Nomura et al., 2011).

Evidence that the beneficial immune-regulatory function of TNF-alpha/TNFR2 interaction results from its capacity to boost Treg activity (Chen et al., 2007) is provided by the observation that spontaneous disease remission in EAE after high Treg expansion can be abolished by depleting Tregs (McGeachy et al., 2005). Accordingly, TNF-alpha/TNFR2 interactions are also essential for the recently described boost of the expansion of CD4+CD25+FoxP3+ Tregs and their suppressive function by CD4+ effector T cells (Teff) after antigen-specific activation (Grinberg-Bleyer et al., 2010). Although this helper function of CD4+ T cells was observed in a murine model of type 1 diabetes, a Teff-mediated boost of Tregs most likely occurs also in other autoimmune diseases including MS and EAE. Furthermore, the dynamic interaction between Teffs and Tregs may explain the relapsing/remitting status of autoimmune diseases such as MS and EAE. During EAE Teffs first colonize the CNS, where they extensively proliferate during the acute phase of the disease. This is followed by a secondary active Treg proliferation and the disease remission, due to the presence of Tregs (McGeachy et al., 2005; Korn et al., 2007; O'Connor et al., 2007). Possibly, the primary Teff activation has promoted a Teff-mediated Treg boost, favouring remission. Then, because of the low level of Teff activation during remission, the Teff-mediated Treg boost is weakened, leading to lower Treg suppressive activity, opening a window for disease relapse when a new wave of Teffs colonizes the target tissue (Grinberg-Bleyer et al., 2010).

Specific local inhibition of TNFR1 represents a promising approach for supporting the generation of regulatory T cells in the periphery at the site of self-antigen presentation, since the resulting enhancement of TNFR2-mediated functions promotes Treg expansion (for reviews, see Chen and Oppenheim, 2010; Biton et al., 2012). The sustained presence and close proximity of TNFR1 inhibitors and self-antigens creates a favourable environment for the expansion of Tregs and their suppressive function which in turn will decrease the number of activated myelin-specific T cells capable of gaining access to the CNS. Therefore, specific inhibition of TNFR1 at the site of self-antigen presentation allows more effective treatment strategies for MS in which mTNF/TNFR2 signaling is beneficial.

V.5.2. *Combination of local TNFR1 inhibition with local depletion of complement component C3 and/or local inhibition of IL-4/IL-13.*

In another embodiment of the present invention, local TNFR1 inhibition used as adjuvant therapy in support of self-antigen vaccination, is combined with other local adjuvant treatments including but not limited to local depletion of complement component C3 (see patent application EP 12075059.1) and/or local inhibition of IL-4/IL-13 (see patent application EP 11075261.5).

Using a murine model of experimental allergic encephalomyelitis (T cell-mediated autoimmunity that mimics aspects of human multiple sclerosis), DAF$^{-/-}$ mice developed more severe paralysis (associated with enhanced IL-17 production) upon immunization with myelin oligodendrocyte glycoprotein than wild-type mice (Liu et al., 2008). The stronger autoreactive T cell response in DAF$^{-/-}$ mice is C3aR-and C5aR-dependent since mice deficient in either or both of these receptors develop weaker autoreactive T cell responses and are resistant to the development of experimental allergic encephalomyelitis, regardless of DAF expression (Strainic et al., 2008).

As mentioned earlier, animal studies have also provided strong evidence that complement component C3 is required for priming and expansion of CD8+ T cells which play an important role in MS. Furthermore, priming of CD8+ T cells requires help from CD4+ T cells, and local complement has been shown to regulate the help of CD4+ cells for CD8+ T cells required for murine allograft rejection (Vieyra et al., 2011). Cognate interactions between CD4+ T cells and dendritic cells (DCs) induce C3a and C5a production and CD4-cell help is mediated, in part, by interaction of DC-derived C3a/C5a with CD8+ T cell-expressed C3aR/C5aR (Vieyra et al., 2011).

Collectively, these data demonstrate that local depletion of complement component C3 at the site of self-antigen presentation has the potential to support the development of tolerance.

Concomitant inhibition of IL-4-mediated functions represents also a potentially valuable therapeutic approach, since IL-4 inhibits the expression of FOXP3, which is a requirement for inducible Treg (iTreg) differentiation.

## VI. COMPOSITIONS FOR SUSTAINED DELIVERY OF ONE OR MORE TNFR1 INHIBOTORS IN COMBINATION WITH ALLERGEN- OR AUTOANTIGEN-SPECIFIC IMMUNOTHERAPY

In one embodiment, the present invention discloses compositions comprising one or more inhibitors of TNFR1 or TNFR1-mediated functions, and one or more matrices mediating sustained delivery of the components. In one specific embodiment, the composition comprises a biodegradable thermogelling polymer solution containing one or more soluble inhibitors of TNFR1 or TNFR1-mediated functions. Upon injection, this fluid formulation spontaneously gels as the temperature of the formulation rises to body temperature. Thereby, the injected gel forms a non-flowing depot from which one or more soluble inhibitors of TNFR1 or TNFR1-mediated functions are released slowly and continuously for several days. The quantity of inhibitors in the composition is balanced in a way that upon gelation of the polymer composit at body temperature, the amount of released inhibitors is sufficient for the therapeutic aims of the method of the present invention. For prolonged allergen or antigen presentation at the site of the gelled polymer composit, one or more allergens or antigens are adsorbed onto aluminium salts or emulsified to form a squalene-based water-in-oil emulsion (Montanide emulsion), and injected in close proximity of the gelled polymer composit.

In another embodiment, the present invention discloses compositions comprising one or more inhibitors of TNFR1 or TNFR1-mediated functions, one or more antigens or allergens, and one or more matrices mediating sustained delivery of the components. In one specific embodiment, the composition comprises a biodegradable thermogelling polymer solution containing one or more soluble inhibitors of TNFR1 or TNFR1-mediated functions, and one or more allergens or antigens either adsorbed onto aluminium salts, or emulsified to form a squalene-based water-in-oil emulsion (Montanide emulsion), or premixed with a water-soluble polyphosphazenes polymer adjuvant. The quantity of each component in the composition is balanced in a way that a) upon injection into the body the biodegradable thermogelling polymer forms a non-flowing gel in which the other components are embedded, and b) upon gelation of the polymer composit at body temperature the amount of released components is sufficient for the therapeutic aims of the method of the present invention.

## VII. COMPOSITIONS FOR SUSTAINED DELIVERY OF ONE OR MORE TNFR1 INHIBOTORS AND ADDITIONAL ADJUVANT THERAPEUTICS IN COMBINATION WITH ALLERGEN- OR AUTOANTIGEN-SPECIFIC IMMUNOTHERAPY

In one embodiment, the present invention discloses compositions comprising one or more inhibitors of TNFR1 or TNFR1-mediated functions, one or more additional adjuvant therapeutics, and one or more matrices mediating sustained delivery of the components. Preferred adjuvant therapeutics supporting the induction of tolerance include but are not limited to recombinant human C3 derivatives for local depletion of complement component C3 (patent application EP 12075059.1) and IL-4/Il-13 inhibitors for local inhibition of IL-4/IL-13-mediated effects (patent application EP 11075261.5).

In one specific embodiment, the composition comprises a biodegradable thermogelling polymer solution containing one or more soluble inhibitors of TNFR1 or TNFR1-mediated functions, and one or more additional adjuvant therapeutics supporting the induction of tolerance. Upon injection, this fluid formulation spontaneously gels as the temperature of the formulation rises to body temperature. Thereby, the injected gel forms a non-flowing depot from which one or more soluble inhibitors of TNFR1 or TNFR1-mediated functions and the additional adjuvant therapeutics are released slowly and continuously for several days. The quantity of inhibitors and additional adjuvant therapeutics in the composition is balanced in a way that upon gelation of the polymer composit at body temperature, the amount of released components is sufficient for the therapeutic aims of the method of the present invention. For prolonged allergen or antigen presentation at the site of the gelled polymer composit, one or more allergens or antigens are adsorbed onto aluminium salts or emulsified to form a squalene-based water-in-oil emulsion (Montanide emulsion), and injected in close proximity of the gelled polymer composit.

In another embodiment the present invention discloses compositions comprising one or more inhibitors of TNFR1 or TNFR1-mediated functions, one or more antigens or allergens, one or more additional adjuvant therapeutics supporting the induction of tolerance, and one or more matrices mediating sustained delivery of the components. Preferred adjuvant therapeutics supporting the induction of tolerance include but are not limited to recombinant human C3 derivatives for local depletion of complement component C3 (patent application EP 12075059.1) and IL-4/Il-13 inhibitors for local inhibition of IL-4/IL-13-mediated effects (patent application EP 11075261.5).

In one specific embodiment, the composition comprises a biodegradable thermogelling polymer solution containing one or more allergens or antigens adsorbed onto a depot-mediating support, one or more soluble inhibitors of TNFR1 or TNFR1-mediated functions, and one or more additional adjuvant therapeutics supporting the induction of tolerance. The quantity of each component in the composition is balanced in a way that a) upon injection into the body the biodegradable thermogelling polymer forms a non-flowing gel in which the other components are embedded, and b) upon gelation of the polymer composit at body temperature the amount of released components is sufficient for the therapeutic aims of the method of the present invention.

## VIII. PHARMACEUTICAL FORMULATIONS

In one embodiment, the therapeutic compositions of the present invention are incorporated into pharmaceutical compositions suitable for administration. Such compositions typically comprise the therapeutic compositions of the present invention and a pharmaceutically acceptable carrier. As used herein, a 'pharmaceutically acceptable carrier' is intended to include any and all solvents, dispersion media, coatings, antibacterial and antifungal agents, isotonic systems, and the like, compatible with the components of the therapeutic compositions of the present invention and pharmaceutical administration. The use of such media and agents for pharmaceutically active substances is well known in the art. Supplementary active compounds can also be incorporated into the composition.

Solutions or suspensions used for parenteral, intradermal, or subcutaneous application can include the following components: a sterile diluent such as water for injection, saline solution, fixed oils, polyethylene glycols, glycerine, propylene glycol or other synthetic solvents, antioxidants such as ascorbic acid or sodium bisulfite, chelating agents such as ethylenediaminetetraacetic acid, buffers such as acetates, citrates or phosphates and agents for the adjustment of toxicity such as sodium chloride or dextrose. The pH can be adjusted with acids or bases, such as hydrochloric acid or sodium hydroxide. In many cases, it will be preferable to include isotonic agents, for example, sugars, polyalcohols such as mannitol, sorbitol, sodium chloride in the composition. The composition should be fluid to the extent that easy syringability exists. The proper fluidity can be maintained, for example, by the use of a coating such as lecithin, by the maintenance of the required particle size in the case dispersion and by use of surfactants. The composition must be stable under the conditions of manufacture and storage and must be preserved against the contaminating action of microorganisms such as bacteria and fungi. Prevention of the action of microorganisms can be achieved by various antibacterial and antifungal agents such as parabens, chlorobutanol, phenol, ascorbic acid, thimoseral, and the like. In all cases, the composition must be sterile. Sterile injectable solutions can be prepared by filtered sterilization. The preparation can be enclosed in ampoules, disposable syringes or multiple dose vials made of glass or plastic. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof. For practical purposes it should be kept in mind that aluminum-adsorbed vaccines are frost sensitive and therefore not lyophilizable.

## IX. THERAPEUTIC METHODS

The present invention discloses therapeutic methods including modes of administration for the induction of allergen or autoantigen tolerance using the compositions of the present invention.

### IX.1. Therapeutic applications of suitable compositions

In one embodiment of the present invention, methods for local inhibition of TNFR1 or TNFR1-mediated functions at the site of allergen or autoantigen presentation as adjuvant therapy prior and during specific immunotherapy in patients with allergy, allergic asthma or autoimmune diseases are disclosed using suitable compositions of the present invention.

In a specific embodiment of the present invention, a suitable inhibitor of TNFR1 or TNFR1-mediated functions is administered subcutaneously or intradermally in a depot-mediating matrix for sustained delivery of the inhibitor. After a period of time, sufficient for local inhibition of TNFR1 or TNFR1-mediated functions, one or more allergens or autoantigens partially or completely adsorbed onto alum or onto another depot-mediating material are injected at the same site. Dependent on the properties of the inhibitor embedded in the depot-mediating matrix, additional injection(s) of embedded inhibitor at the site of allergen presentation may be performed to support local inhibition of TNFR1 or TNFR1-mediated functions within the period of allergen or autoantigen presentation to T cells.

In another specific embodiment of the present invention, a suitable inhibitor of TNFR1 or TNFR1-mediated functions is administered subcutaneously or intradermally in a depot-mediating matrix for sustained delivery of the inhibitor. After a period of time, sufficient for local inhibition of TNFR1 or TNFR1-mediated functions, the inhibitor of TNFR1 or TNFR1-mediated functions and one or more allergens or autoantigens both embedded in a depot-mediating matrix for sustained delivery, are administered subcutaneously or intradermally. Dependent on the properties of the inhibitor embedded in the depot-mediating matrix, additional injection(s) of embedded inhibitor at the site of allergen presentation may be performed to support local inhibition of TNFR1 or TNFR1-mediated functions within the period of allergen or autoantigen presentation to T cells.

In another embodiment of the present invention, methods for local inhibition of TNFR1 or TNFR1-mediated functions in combination with local C3-depletion and/or local inhibition of IL-4/IL-13-mediated effects at the site of allergen or autoantigen presentation as adjuvant therapy prior and during specific immunotherapy in patients with allergy, allergic asthma or autoimmune diseases are disclosed using suitable compositions of the present invention.

In a specific embodiment of the present invention, a depot-mediating matrix for sustained delivery of a suitable inhibitor of TNFR1 or TNFR1-mediated functions, a suitable recombinant human C3-derivative (rhC3-derivative) and/or a suitable inhibitor of IL-4/IL-13-mediated effects (all embedded in a depot-mediating matrix), are administered subcutaneously or intradermally. After a period of time, sufficient for local inhibition of TNFR1 or TNFR1-mediated functions, local depletion of C3 and/or local inhibition of IL-4/IL-13-mediated effects, one or more allergens or autoantigens partially or completely adsorbed onto alum or onto another depot-mediating material are injected at the same site. Dependent on the properties of the components embedded in the depot-mediating matrix, additional injection(s) of embedded TNFR1- and IL-4/IL-13-specific inhibitors and rhC3-derivative at the site of allergen presentation may be performed to support local inhibition of TNFR1 or TNFR1-mediated functions, local C3-depletion and/or local inhibition of IL-4/IL-13-mediated effects within the period of allergen or autoantigen presentation to T cells.

In another specific embodiment of the present invention, a depot-mediating matrix for sustained delivery of a suitable inhibitor of TNFR1 or TNFR1-mediated functions, a suitable recombinant human C3-derivative (rhC3-derivative) and/or a suitable inhibitor of IL-4/IL-13-mediated effects (all embedded in a depot-mediating matrix), are administered subcutaneously or intradermally. After a period of time, sufficient for local inhibition of TNFR1 or TNFR1-mediated functions, local depletion of C3 and local inhibition of IL-4/IL-13-mediated effects, another depot-mediating matrix for sustained delivery of the TNFR1- and IL-4/IL-13-specific inhibitors, the rhC3-derivative and one or more allergens or autoantigens (all embedded in a depot-mediating matrix), are administered subcutaneously or intradermally. Dependent on the properties of the supporting components embedded in the depot-mediating matrix, additional injection(s) of embedded inhibitors and rhC3-derivative at the site of allergen presentation may be performed to support local inhibition of TNFR1 or TNFR1-mediated functions, local C3-depletion and/or local inhibition of IL-4/IL-13-mediated effects within the period of allergen or autoantigen presentation to T cells.

### IX.2. Therapeutically effective doses of TNFR1 inhibitors

Determination of a therapeutically effective dose is well within the capability of those skilled in the art. The therapeutically effective dose can be estimated initially in animal models, usually mice, rats, rabbits, dogs, pigs, or non-human primates. The animal model also can be used to determine the appropriate concentration range and route of administration. Such information can then be used to determine useful doses and routes for administration in humans.

IX.2.1. *Effective doses of N-acetyl-L-cysteine (NAC) and other glutathione prodrugs.*

S-Methylglutathione (GSM; MW 321.35) is a glutathione donor and NAC (MW 163.20) a glutathione precursor. NAC is readily deacetylated in the body to form cysteine which efficiently supports glutathione (GSH) synthesis.

In cultures of peripheral blood T cells, 10 mM NAC proved to be an effective enhancer of T cell function and enhancer of growth (Eylar et al., 1993). Cytotoxicity studies with resting T cells from seven donors over a 3 day period in the absence and presence of NAC ranging from 1 mM (163.2 $\mu$g/ml) to 20 mM (3.26 mg/ml) revealed that NAC is nontoxic even at 20 mM (Eylar et al., 1993).

In isolated rat cardiomyocytes, exogenously added S-methylglutathione (GSM) has been demonstrated to reproduce the effects of in vivo NAC treatment (Cailleret et al., 2004). After preincubation for 2-3 hours in a medium containing 50 $\mu$M GSM, cardiomyocytes isolated from control rats were protected against the deleterious effects of TNF-alpha on contraction to the same extent as cardiomyocytes isolated from rats treated by two i.p. injections of 100 mg NAC, 48 and 24 hours before anesthesia. Cardiomyocytes isolated from control rats were not protected by exogenously added NAC under these experimental conditions, pointing to the necessity of a long-term preincubation (at least 2-3 days) in case of NAC administration and a short-term preincubation (at least 2-3 hours) in case of GSM administration.

Mice sensitized to ovalbumin have been treated for 8 days with drinking water containing 0.5 g/liter up to 2 g/liter NAC adjusted to pH 7.5 and changed every 2 days (Jeannine et al., 1995). Using 1.0 g/liter NAC, both ovalbumin-specific IgE and IgG1 (the murine isotype equivalent to human IgG4) responses were decreased by more than 70%. Even at 0.5 g/liter NAC the decrease of ovalbumin-specific IgE and IgG1 was still in the range of 30%. On the basis of the volume of water drunk in one day, the quantity of NAC swallowed by a mouse could be estimated at 50 mg/kg/day, comparable to 9 mg/kg per day given in humans as a mucolytic or 300 mg/kg per day as an antidote against acetaminophen-induced hepatotoxicity (Jeannine et al., 1995). This suggests that NAC acts in the range of doses that are already used in human therapeutics.

In patients, NAC has been administered orally at high doses in clinical studies for the treatment of idiopathic pulmonary fibrosis (Demedts et al., 2005) and cystic fibrosis (Tirouvanziam et al., 2006). Evaluation of NAC for the treatment of idiopathic pulmonary fibrosis was performed over one year in a double-blind, randomized, placebo-controlled multicenter study with a total of 182 patients. In this study, NAC was administered at a total oral dose of 1800 mg/day in addition to the standard therapy with prednisone and azathioprine. Although this NAC dose is three to nine times the usual approved dose of NAC when it is administered as an antioxidant and mucolytic agent in chronic obstructive pulmonary disease, the NAC and placebo groups had similar overall rates of side effects. The primary end points including vital capacity proved to be better in the group receiving NAC in addition to the standard therapy (Demedts et al., 2005). For the treatment of cystic fibrosis, high NAC doses in excess of 1800 mg/day (600 mg to 1000 mg NAC three times daily) were administered orally for 4 weeks in a phase 1 study with 18 cystic fibrosis patients (Tirouvanziam et al., 2006). The high doses proved to be safe and effective in augmenting the GSH level in blood neutrophils and decreasing the airway neutrophil count and elastase activity. The data of both studies confirm the proven safety record of long-term use of high doses of NAC (Kelly, 1998).

IX.2.2. *Effective doses of salicylates.*

The various inhibitory effects of acetylsalicylic acid (ASA; MW 180.2) and related salicylates (e.g., sodium salicylate; SA; MW 160.11) are concentration dependent. A 50% inhibition by ASA requires a concentration of approx. 2 x $10^{-6}$ M for COX-1, appprox. 3 x $10^{-4}$ M for COX-2, approx. 1 x $10^{-3}$ M for IL-4 gene transcription, and approx. 3 x $10^{-3}$ M for NF-kappa B translocation (Cianferoni et al., 2001). Selective inhibition of TNFR1-mediated activation of NF-kappa B requires even higher doses of salicylates in the range of 1-2 x $10^{-2}$ M (Thommesen and Laegreid, 2005).

The therapeutic range for ASA and SA has been restricted to 0.8-1.7 x $10^{-3}$ M, corresponding to a serum salicylate concentration of 150-300 mg/liter. Restriction of the therapeutic range is necessary to avoid salicylate-related toxicity such as tinnitus. It should be mentioned, however, that tinnitus may occur also occasionally at levels as low as 95 mg/liter (Furst et al., 1987).

Salicylate kinetics are complex, involving 2 capacity limited and 3 first order processes (Furst et al., 1987). Capacity limited processes mean that the enzyme(s) acting to metabolize a drug become saturated and are working at their maximum capacity, even at relatively low drug doses. Therefore, small increases in dose can result in a disproportionate increase in serum levels. First order processes are those which metabolize increasing amounts of drug as more drug is presented to them. Therefore, an increase in dose results in a directly proportional increase in serum concentration. Further complications of salicylate kinetics include the facts that protein binding is not linear, that urinary excretion of un-metabolized salicylate is urine pH dependent, and that there is inherent genetic variability.

Using choline magnesium trisalicylate (CMS) for the treatment of patients with rheumatoid arthritis (RA), a strategy for reaching therapeutic salicylate levels has been developed (Furst et al., 1987). Despite the complex salicylate kinetics, the authors used a simplified weight adjusted dose of 45 mg CMS/kg/day as an initial dose. After one to two weeks of 45 mg CMS/kg/day in two divided doses, 51 of 71 patients with RA had steady state serum salicylate levels between 150 and 300 mg/liter (mean value: 213 $\pm$ 10 mg/L). Seventeen patients required dose adjustment using the formula: dosing rate = total clearance x concentration. Since all salicylate preparations are metabolized similarly once they are adsorbed and broken down to the parent salicylate molecule, the results of this study apply to all salicylates.

IX.2.3. *Effective doses of TNFR1-specific antisense oligonucleotides.*

In mice, TNR1-specific antisense oligonucleotides have been administered as 2'-O-(2-methoxy)ethyl-modified antisense oligonucleotides (ASO). After i.p. injection of 25 mg/kg ASO every other day for a total of four times, mice were protected against subsequent radiation-induced liver damage (Huang et al., 2006).

In a preferred embodiment of the present invention, non-modified TNR1-specific antisense oligonucleotides are complexed with cationic liposomes to enhance cellular uptake and protect the oligonucleotides against enzymatic degradation in serum.

In one specific embodiment of the present invention, ultradeformable cationic liposomes (UCL) containing sodium cholate in addition to DOTAP (Kim et al., 2004) are used for complexing non-modified TNR1-specific antisense oligonucleotides. Sodium cholate is an edge activator and responsible for rendering flexibility in liposomes. The addition of sodium cholate provides an additional advantage in that instead of toxic organic solvents such as chloroform phosphate-buffered saline (PBS) can be used for the preparation of UCLs. PBS is not capable of dissolving DOTAP, but as soon as sodium cholate is added at a ratio of 1:6 (w/w) to the mixture of DOTAP and PBS (10 mg DOTAB/ml PBS), DOTAB is dissolved. Furthermore, sodium cholate inhibits the tendency of DOTAP for aggregation. The average particle size of this formulation is approximately 80 nm. Assessment of the in vitro transfection efficiency of plasmid DNA revealed an optimal ratio of 1:14 DNA/UCL (Kim et al., 2004). Recently, such liposomes have been used for transdermal delivery of non-modified IL-13-specific antisense oligonucleotides (IL-13 ASO) for the treatment of mice with atopic dermatitis (Kim et al., 2009). At a ratio of 6:1 (IL-13 ASO/UCL), maximum inhibition of IL-13 secretion was observed (Kim et al., 2004).

In a preferred specific embodiment of the present invention, multilammelar vesicles (MLVs) containing the cationic lipid 1,2-dioleoyl-3-trimethylammonium-propane (DOTAP) are used for complexing non-modified TNR1-specific antisense oligonucleotides. Such MLV lipoplexes have been shown to mediate efficient cellular association and uptake (Ross et al., 1998). The cellular uptake is believed to occur mainly through endocytosis with escape of the DNA into the cytoplasm of the cell by fusion of the liposomes with, or rupture of the endosomal membrane. In the presence of serum, multilammelar vesicles (>300 nm) are more efficient vectors for DNA transfer than small unilammelar vesicles (SUVs; <300 nm), which interact to higher extent with serum proteins (Ross et al., 1998). Serum proteins are thought to destabilize the bilayer membranes of liposomes, which may alter the lipoplex and prevent its ability to associate with cells, thus decreasing the uptake of DNA. A number of variables have been identified to influence the effectiveness of lipoplexes as vector including the charge ratio of cationic lipid to DNA. Using DOTAP-containing MLVs, optimal charge ratios of cationic lipid to DNA are below and up to about 1:1 (Ross et al., 1998). For example, for the preparation of lipoplexes with a ratio of 1:2 (cationic lipid to DNA) according to the method of Ross et al. (1998), 12 $\mu$g of DNA is added to 300 $\mu$l of a DOTAP-containing MLV suspension with a concentration of 0.133 $\mu$mole/ml.

In another preferred embodiment of the present invention, non-modified TNR1-specific antisense oligonucleotides complexed with cationic liposomes are embedded into hydrogels. Preferred hydrogels include those listed in section II.

IX.2.4. *Effective doses of dominant-negative sTNF mutants.* Several sTNF-alpha variants have been designed by exchange of one or two amino acid residues, which are capable of sequestering native TNF-alpha homotrimers from TNF receptors by formation of inactive native:variant heterotrimers (Steed et al., 2003). At concentrations as low as two-fold that of native TNF-alpha, variants A145R/Y87H and A145R/I97T attenuated TNF-induced caspase activity by 50% and at 20-fold excess, activity was reduced to baseline. At 10-fold excess over native TNF-alpha, variant A145R/Y87H blocked TNF-induced translocation of the NF-kappa B p-65-RelA subunit in HeLa cells. Several dominant-negative sTNF-alpha variants neutralize TNF-induced caspase and NF-kappa B-mediated transcriptional activity over a wide range of native TNF-alpha concentrations, including the clinically relevant range of 100 to 200 pg/ml found in the synovial fluid of rheumatoid arthritis patients (Steed et al., 2003).

Furthermore, variant A145R/Y87H proved to be non-toxic. Dosed as high as 30 mg/kg of mouse body weight (along with 30 $\mu$g/kg native TNF-alpha), A145R/Y87H did not induce mortality or hepatotoxicity. In a murine model of collagen-induced arthritis, variant A145R/Y87H reduced joint swelling when dosed once daily at 2 mg/kg subcutaneously with an intravenous loading dose of 2 mg/kg on the first treatment day (Steed et al., 2003).

IX.2.5. *Effective doses of TNFR1-specific TNF mutants.*

Useful TNFR1-specific sTNF-alpha mutants include but are not limited to R1antTNF containing mutations A84S, V85T, S86T, Y87H, Q88N, and T89Q (Shibata et al., 2008). R1antTNF neutralizes TNFR1-mediated responses without affecting TNFR2-mediated bioactivity. Like wild-type TNF-alpha, R1antTNF has a very short half-life of approximately 10 min in plasma of mice. By site-specific PEGylation of R1antTNF at its N-terminus, the plasma half-life of R1antTNF could be increased significantly (Shibata et al., 2009).

Administration of R1antTNF or PEG-R1antTNF at 1 μg or 3 μg/mouse twice a day for 3 weeks in mice with collagen-induced arthritis (the treatment was started 2 days after the second immunization) significantly decreased the severity of arthritis (Shibata et al., 2009). Only R1antTNF at 1 μg/mouse had no therapeutic effect, most likely due to the short plasma half-life of R1antTNF, since 1 μg/mouse of PEG-R1antTNF was effective.

IX.2.6. *General considerations.*

Dosage regimens may be adjusted to provide the optimum therapeutic response. The quantity of the polymer-embedded components depends on the release kinetics of the biodegradable thermogelling polymer and is adjusted to a level that guarantees the continuous release of therapeutically effective doses over a period of 3 to 5 days. The quantity of embedded components will vary according to factors such as the weight and the age of the individual, and the ability of the composition to induce an effective immune response in the individual.

In summary, these data provide useful guidelines for the determination of a therapeutically effective dose for those skilled in the art.

IX.3. Therapeutically effective doses of rhC3-derivatives and inhibitors of IL-4/IL-13-mediated effects

Therapeutically effective doses of rhC3-derivatives and inhibitors of IL-4/IL-13-mediated effects are described in patent application EP 12075059.1 and in patent application EP 11075261.5, respectively.

IX.4. Modes of administration of therapeutic compositions

Routes of administration of the compositions of the present invention by injection or by implantation include but are not limited to subcutaneous, intradermal, intramuscular, nasal, transbucal, transmucosal, sublingual, rectal, vaginal, intraocular, or topical administration. Preferred examples of routes of administration of the compositions of the present invention include but are not limited to intradermal, subcutaneous, and intramuscular administration.

For mucosal immunization, the physicochemical characteristics of the administered components and the delivery vehicle have to be adjusted to stimulate their uptake through the various mucosal routes. For example, alum salts are ineffective when administered by the oral or nasal route. In contrast, cationic chitosan derivatives are of special interest in nasal delivery because of their excellent biocompatibility and mucoadhesive nature (Hagenaars et al., 2010). For example, a thermal-sensitive hydrogel which was formulated as intranasal vaccine with N[(2-hydroxy-3-trimethylammonium)propyl] chitosan chloride (HTCC) and α,β-glycerophosphate, was shown to significantly prolong the antigen residence time in the nasal cavity and to enhance the transepithelial transport via the paracellular routes (Wu et al., 2012).

## LITERATURE

Abbott et al., BMC Immunol. 12: 72; 2011.
Abdulamir et al., J. Investig. Allergol. Clin. Immunol. 19: 218-224; 2009.
Abe et al., J. Immunol. 167: 4651-4660; 2001.
Abramson et al.,. Cochrane Database Syst. Rev. CD001186; 2003.
Adamy et al., J. Mol. Cell. Cardiology 43: 344-353; 2007. Akbar et al., Immunology 109: 319-325; 2003.
Akdis and Akdis, J. Allergy Clin. Immunol. 127: 18-27; 2011.
Alexopoulou et al., Eur. J. Immunol. 36: 2768-2780; 2006.
Alhalaweh et al., Eur. J. Pharm. Sci. 38: 206-214; 2009.
Amadou et al., Am. J. Physiol. 282: C1339-C1347; 2002.
Andersen, Nat. med. 10(Suppl.): S18-S25; 2004.

Arntz et al., Arthritis Res. Therapy 12: R61; 2010.
Barnes, Trends Pharmacol. Sci. 31: 335-343; 2010.
Bautsch et al., J. Immunol. 165: 5401-5405; 2000.
Biton et al., Joint Bone Spine 79: 119-123; 2012.
Bluestone et al., Nature 464: 1293,2010.
Bourraindeloup et al., Circulation 110: 2003-2009; 2004.
Cailleret et al., Circulation 109: 406-411; 2004.
Chang et al., Vaccine 19: 2884-2889; 2001.
Chen and Oppenheim. In: TNF pathophysiology. Molecular and cellular mechanisms. Curr. Dir. Autoimmun., pp. 119-134, Karger, Basel; 2010.
Chen et al., J. Immunl. 179: 154-161; 2007.
Chen et al., J. Immunol. 185: 174-182; 2010.
Choi et al., Pharmaceut. Res. 20: 2008-2010; 2003.
Cianferoni et al., Blood 97: 1742-1749; 2001.
Clapp et al., J. Pharm. Sci. 100: 388-401; 2011.
Coppieters et al., J. Exp. Med. 209: 51-60; 2012.
Defer et al., J. Biol. Chem. 282: 35564-35573; 2007.
Demedts et al., N. Engl. J. Med. 353: 2229-2242; 2005.
Diamyd (2011)

a) Diamyd US phase III trial: http://clinicaltrials.gov/ct2/show/NCT00751842
b) Diamyd European phase III trial: http://clinicaltrials.gov/ct2/show/NCT00723411
c) Diabetes prevention - immune tolerance (DIAPREV-IT)
http://clinicaltrials.gov/ct2/show/NCT01122446 ?term=diamyd&rank=6

Dong et al., J. Biol. Chem. 272: 9962-9970; 1997.
Drouin et al., J. Immunol. 167: 4141-4145; 2001.
Drouin et al., Am. J. Respir. Crit. Care Med. 173: 852-857; 2006.
Ehrenstein et al., J. Exp. Med. 200: 277-285; 2004.
Eylar et al., Int. Immunol. 1: 97-101; 1993.
Famulok et al., Curr. Top. Microbiol. Immunol. 243: 123-136; 1999.
Faria and Weiner, Immunol. Rev. 206: 232-259; 2005.
Feldmann et al., Nat. Med. 9: 1245-1250; 2003.
Furst et al., J. Rheumatol. 14: 342-347; 1987.
Garren et al., Ann. Neurol. 63 : 611-620 ; 2008.
Gilbert et al., J. Control. Release 5: 113-118; 1987.
Godebu et al., J. Immunol. 181: 1798-1805; 2008.
Gohil et al., Global J. Pharmacol. 4: 19-30; 2010.
Gong et al., Int. J. Pharm. 365: 89-99; 2009 a.
Gong et al., BMC Biotechnol. 9: 8; 2009 b.
Goverman, Nature Rev. immunol. 9: 393-407; 2009.
Grinberg-Bleyer et al., J. Clin. Invest. 120: 4558-4568; 2010.
Hagenaars et al., J. Control. Release 144: 17-24; 2010.
Hamano et al., Eur. J. Immunol. 41: 2010-2020; 2011.
Hartl et al., J. Allergy Clin. Immunol. 119: 1258-1266; 2007.
Hayakawa et al., EMBO J. 22: 3356-3366; 2003.
Heeger and Kemper, Immunobiology 217: 216-224,2012.
Helbling-Leclerc et al., Biochim. Biophys. Acta 1418: 165-175; 1999.
Hering et al., Am. J. Transplant. 4: 390-401; 2004.
Herold et al., N. Engl. J. Med. 346: 1692-1698; 2002.
Higuchi et al., J. Rheumatol. 27: 1038-1044 ; 2000.
Holgate and Polosa, Nature Rev. Immunol 8: 218-230; 2008.
Homann and von Herrath, Clin. Immunol. 112: 202-209; 2004.
Housley et al., J. Immunol. 186: 6779-6787; 2011.
Huang et al., Clin. Cancer Res. 12: 2849-2855; 2006.
Humbles et al., Nature 406: 998-1001; 2000.
Hyun et al., Biomacromolecules 8: 1093-1100; 2007.
Ichim et al., Expert Opin. Biol. Ther. 8: 191-199; 2008.

Jacobsen et al., Allergy 62: 943-948; 2007.
Jaeckel et al., Horm. Metab. Res. 40: 126-136; 2008.
Jeannin et al., J. Exp. Med. 182: 1785-1792; 1995.
Jeong et al., Nature 388: 860-862, 1997.
Juedes and von Herrath, Res. Rev. 20 : 446-451 ; 2004.
Jurynczyk et al., Ann. Neurol. 68 : 593-601 ; 2010.
Kamphuis et al., Lancet 366 : 50-56 ; 2005.
Kang et al., Biomaterials 31: 2453-2460; 2010.
Karagiannidis et al., J. Allergy Clin. Immunol. 114: 1425-1433, 2004.
Kassiotis and Kollias, J. Exp. Med. 1993: 427-434; 2001.
Kawamoto et al., J. Clin. Invest. 114: 399-407; 2004.
Kearley et al., J. Exp. Med. 202: 1539-1547; 2005.
Kearley et al., J. Allergy Clin. Immunol. 122: 617-624; 2008. Kelly, Altern. Med. Rev. 3: 114-127; 1998.
Keymeulen, N. Engl. J. Med. 352: 2598-2608; 2005.
Kim et al., Biomaterials 25: 305-313; 2004.
Kim et al., J. Gene Med. 11: 26-37; 2009.
Kleijwegt et al., J. Immunol. 185: 1412-1418; 2010.
Knip et al., Diabetes 54: S125-S136; 2005.
Köhl et al., J. Clin. Invest. 116: 783-796; 2006.
Koop and Ghosh, Science 265: 956-959; 1994.
Korn et al., Nature Med. 13: 423-431; 2007.
Kukreja et al., J. Clin. Invest. 109: 131-140; 2002.
Kwan et al., Immunol. Res. DOI 10.1007/s12026-012-8327-1; 2012.
Landewe et al., Ann. Rheum. Dis. 69: 1655-1659; 2010.
Langier et al., Israel Med. Assoc. J. 14: 180-183; 2012.
Larche et al., Nat. Rev. Immunol. 6: 761-771; 2006.
Lee et al., Clin. Exp. Immunol. 148: 53-63; 2007.
Leroux-Roels, Vaccine 285: C25-C36; 2010.
Li et al., Blood 112: 5084-5094, 2008.
Li et al., J. Mol. Cell Biol. 4: 38-47; 2012.
Liang et al., J. Biol. Chem. 264: 13519-13523; 1989.
Liang et al., J. Immunol. 146: 1909-1913; 1991.
Lin et al., Diabetes 59: 2247-2252; 2010.
Liu and Hannun, Biol. Chem. 272: 16281-16287; 1997.
Liu et al., J. Immunol. 180: 5882-5889; 2008.
Ludvigsson et al., N. Engl. J. Med. 359: 676-781; 2008.
MacroGenics press release (October 20, 2011), http://www.macrogenics.com/press_releases-284.html Mantel, et al., PLoS biology 5, e329: 2847-2861; 2007. Mazzeo et al., Eur. J. Immunol. 28: 3205-3213; 1998. McGeachy et al., J. Immunol. 175: 3025-3032; 2005. McTiernan et al., Gene Ther. 14 (23) :1613-22: 2007.
Moller et al., J. Allergy Clin. Immunol. 109: 251-256; 2002.
Munegowda et al., J. Clin. Immunol. 31: 811-826; 2011.
Nadkarni et al., J. Exp. Med. 204: 33-39: 2007.
Nicholls et al., Ann. N.Y. Acad. Sci. 1213: 46-61; 2010.
Nie et al., Internatl. J. Nanomed. 6: 151-166; 2011.
Nomura et al., J. Control. Release 149: 8-14; 2011.
Notley et al., J. Exp. Med. 205: 2491-2497; 2008.
O'Connor et al., J. Immunol. 179: 958-966; 2007.
Oral et al., J. Biol. Chem. 272: 4836-4842; 1997.
Pai et al., Am. Assoc. Pharmac. Sci. J. 11: 88-98; 2009.
Pendyala and Creaven, Cancer Epidemiol. Biomarkers Prevention 4: 245-251; 1995.
Peng et al., J. Immunol. 176: 3330-3341; 2006.
Peng et al., Blood 111: 2452-2461, 2008.
Peng et al., Biomaterials 31: 5227-5236; 2010.
Qiao et al., Int. J. Pharm. 294: 103-112; 2005.
Raedler et al., Am. J. Transplant. 9: 1784-1795; 2009.
Rolland et al., Pharmacol. Ther. 121: 273-284; 2009.
Ross et al., J. Liposome Res. 8: 499-520; 1998.
Ruel-Gariepy and Leroux, Eur. J. Pharmaceutics Biopharmaceutics 58: 409-426; 2004.

Sandstrom et al., J. Leukocyte Biol. 55: 221-226; 1994.
Schmidt-Weber and Blaser. Curr. Opin. Allergy Clin. Immunol. 5: 525-530; 2005.
Schmidt-Weber and Blaser. Inflamm. Allergy Drug Targets 5: 15-21; 2006.
Sen et al., Free Radical Biol. Med. 22: 1241-1257; 1997.
Sen, Nutritional Biochem. 8: 660-672, 1997.
Shibata et al., J. Biol. Chem. 283: 998-1007; 2008.
Shibata et al., Biomaterials 30: 6638-6647; 2009.
Sinha et al., Int. J. Pharm. 278: 1-23; 2004.
Skyler et al., Diabetes Care 28: 1068-1076; 2005.
Smyth et al., Chest 138: 905-912, 2010.
Staal et al., Proc. Natl. Acad. Sci. USA 87: 9943-9947; 1990. Stäger et al., Nat. Med. 9: 1287-1292; 2003.
Steed et al., Science 301: 1895-1898; 2003.
Steinman and Zamvil, Ann. Neurol. 68: 567-569; 2010.
Strainic et al., Immunity 28: 425-435; 2008.
Strickland et al., J. Exp. Med. 203: 2649; 2006.
Suresh et al., J. Immunol. 170: 788-794; 2003.
Suvannavejh et al., Cell. Immunol. 205: 24-33; 2000.
The Lenercept Multiple Sclerosis Study Group and The University of British Columbia MS/MRI Analysis Group, Neurology 53: 457-465; 1999.
Thommesen and Laegreid, J. Biochem. Mol. Biol. 38: 281-289; 2005.
Thorburn and Hansbro, Am. J. Respir. Cell Mol. Biol. 43: 511-519; 2010.
Tirouvanziam et al., Proc. Natl. Acad. Sci. USA 103: 4628-4633; 2006.
Tolerx, Inc. And GlaxoSmithKline (Novewmber 29, 2011), http://www.biospace.com/news_story.aspx?StoryID=21361&full=1
Tsuboi et al., Cytokine 7: 372-379; 1995.
Valencia et al., Blood 108:253-261; 2006.
Van Hauwermeiren et al., Cytokine Growth Factors Rev. 22: 311-319; 2011.
Van Mierlo et al., J. Immunol. 180: 2747-2751; 2008.
Vieyra et al., Am. J. Pathol. 179: 766-774; 2011.
Viglietta et al., J. Exp. Med. 199: 971-979; 2004.
Von Haehling et al., Basic Res. Cardiol. 99: 18-28; 2004. Walker et al. J. Clin. Invest. 112: 1437-1443; 2003.
Warren et al., Eur. J. Neurol. 13: 887-895; 2006.
Wenzel et al., Lancet 370: 1422-1431; 2007.
Wenzel et al., Am. J. Respir. Crit. Care Med. 179: 549-558; 2009.
Wu et al., Biomaterials 33: 2351-3260; 2012.
Yalcindag et al., J. Allergy Clin. Immunol. 117: 1455-1461; 2006.
Yan et al., Frontiers Biosci. 10: 1802-1827; 2005.
Yu and Malek, J. Immunol. 177 : 5115-5121; 2006.
Zaharoff et al., Vaccine 25: 2085-2094; 2007.
Zalevsky et al., J. Immunol. 179 : 1872-1883; 2007
Zettlitz et al., MAbs 2: 639-647; 2010.
Zhang et al., Biomacromolecules 7: 2492-2500; 2006.
Zhou, Immunobiology 217: 225-234; 2012.
Zozulya and Wiendl, Hum. Immunol. 69: 797-804; 2008.

## EXAMPLES

The following examples are intended to illustrate but not limit the present invention.

### EXAMPLE 1. TREATMENT OF CULTURED T CELLS WITH INHIBITORS OF TNFR1-MEDIATED FUNCTIONS

#### EXAMPLE 1.1. Preparation of human lymphocytes

This example describes procedures for the preparation and culture of human lymphocytes and lymphocyte subpopulations from peripheral blood (Biddison, 1998).

*1.1.1. Preparation of lymphocytes by Ficoll-Hypaque gradient centrifugation*

Density gradient centrifugation has proven to be an easy and rapid method for the separation of lymphocytes from other peripheral blood cell populations. Due to their lower densities, lymphocytes and platelets will float on top of a density gradient of Ficoll-Hapaque, whereas granulocytes and erythrocytes collect at the bottom of the centrifugation tube.

Fifteen ml of whole blood in a 50 ml conical centrifuge tube are mixed with 35 ml room temperature phosphate-buffered saline (PBS) and underplayed with 10 ml room temperature Ficoll-Hypaque solution. After centrifugation at 800 x g for 20 min (20°C, with the brake off), most of the plasma- and platelet-containing supernatant above the interface band (granulocytes and erythrocytes are in the red pellet) is removed by aspiration. Thereafter, the lymphocyte-containing interface band is aspirated (along with no more than 5 ml of fluid above the pellet) and transferred into a 50 ml conical centrifuge tube. After combining the bands from 2-3 Ficoll-Hypaque gradients into one 50 ml tube, PBS is added to the 50 ml mark, followed by centrifugation at 600 x g for 10 min (20°C, with the brake on). The supernatant is discarded and the pellet resuspended with 10 ml room temperature PBS. Resuspended pellets are combined in one 50 ml tube and PBS is added to the 50 ml mark. After centrifugation at 300 x g for 15 min (20°C, with the brake on), the platelet-containing supernatant is discarded and the lymphocyte pellet resuspended in lymphocyte culture medium. The low-speed centrifugation permits as many platelets as possible to remain above the pellet of lymphocytes.

Viable cells are determined by trypan blue exclusion and purity of the lymphocyte preparation is analyzed by flow cytometry using an anti-CD45 (anti-leukocyte common antigen) antibody (Robinson et al., 1998).

*1.1.2. Positive selection of T cells by monoclonal antibody-coated magnetic beads*

In this procedure, T cells are positively selected based on their cell-surface expression of CD3. Aliquots of the lymphocyte population purified as described in Example 1.1.1. (approx. 1 x $10^8$ cells) are resuspended in 5 ml PBS containing 0.5% (w/v) human serum albumin (PBS/HSA), kept on ice for 15 min, and then mixed with 2 ml ice-cold PBS/HSA containing an appropriate amount (according to the instructions of the manufacturer) of anti-CD3-coated magnetic beads (Dynabeads M-450; Dynal). The mixture is incubated at 4°C for 45 min with gentle tilting and rotation on a platform rocker. Thereafter, the tube is placed on a vertical magnet for 2 min and nonadherent cells are removed carefully by aspiration. In subsequent steps, beads are resuspended in 5 ml Iscoves's modified Dulbecco's medium (Life Technologies) containing 0.5% (w/v) human serum albumin (IMDM/HSA), the tube is placed on a vertical magnet for 2 min, nonadherent cells are removed by aspiration, then beads are resuspended again in 3 ml (IMDM/HSA), this time the tube is placed with its conical bottom on a horizontal magnet for 2 min at room temperature, and 2.5 ml of the supernatant is removed.

Specifically bound T lymphocytes are released by the addition of 0.2 ml Detachabead solution (Dynal), an antiserum specific for mouse Fab fragments which competes with the bead-coupled monoclonal antibody that is bound to the surface of the cells and thus causes the cells to come off. After incubation for 30 min at room temperature (with gentle resuspension every 5 min), 3 ml PBS/HSA are added, the tube is placed on a vertical magnet for 2 min and the supernatant containing detached T cells is saved. The detachment procedure is four times repeated and the combined supernatants are centrifuged at 600 x g for 10 min at room temperature. The supernatant is removed by careful aspiration and the pellet resuspended in IMDM/HAS.

Viable cells are determined by trypan blue exclusion and purity of the lymphocyte preparation is analyzed by flow cytometry using an anti-CD3 antibody (Robinson et al., 1998).

*1.1.3. Positive selection of T cell subpopulations by monoclonal antibody-coated magnetic beads*

The procedure of Example 1.1.2. is applied to purify CD4+ or CD8+ T cells, using either anti-CD4-coated magnetic beads or anti-CD8-coated magnetic beads. Isolation of CD4+CD25+ regulatory T cells is performed as described in the isolation kit #130-091-301 from Miltenyi Biotec (Germany). Briefly, the isolation is performed in two steps. First, non-CD4+ T cells are indirectly magnetically labeled with a cocktail of biotin-conjugated antibodies against CD8, CD14, CD16, CD19, CD36, CD56, CD123, TCRγ/δ, and DC235a (glycophorin A) and Anti-Biotin-Microbeads, followed by depletion of the labeled cells over a MACS column. In the second step, the flow-through fraction of pre-enriched CD4+ T cells is labeled with CD25 Microbeads for subsequent positive selection of CD4+CD25+ regulatory T cells.

*1.1.4. Positive selection of B cells by monoclonal antibody-coated magnetic beads*

The procedure of Example 1.1.2. is applied using anti-CD19-coated magnetic beads.

EXAMPLE 1.2. Culture of human T and B cells

*1.2.1. Culture of T cells.* T cells purified from human peripheral blood according to Example 1.1., are cultured in complete medium consisting of RPMI 160 (Gibco BRL) supplemented with 10% heat-inactivated human AB+ serum (CTS Annemasse, France), 2 mM glutamine, 20 mM sodium pyruvate (Sigma), 50 μg/ml streptomycin, and 50 U/ml penicillin (Gibco BRL) as described by Jeannin et al. (1995).

*1.2.2. Culture of B cells.* Human B cells isolated from tonsillar mononuclear cells according to Example 1.1., are cultured in complete medium (CM) consisting of Iscoves's modified Dulbecco's medium (Gibco BRL) supplemented with 10% heat-inactivated human AB+ serum (CTS Annemasse, France), 2 mM glutamine (Flow), 1% sodium pyruvate (Sigma), 1% nonessential amino acids (Sigma), 1 mM HEepes (Sigma), 100 μg/ml streptomycin, and 100 U/ml penicillin (Gibco BRL) as described by Jeannin et al. (1995).

**EXAMPLE 1.3. In vitro effects of NAC on T and B cells**

The experiments of EXAMPLE 1.3. demonstrate the various effects of N-acetyl-L-cysteine (NAC) on cultured T and B cells.

*1.3.1. NAC-mediated enhancement of T cell proliferation*

Using a protocol of Eylar et al. (1993), T cells purified from human peripheral blood according to Example 1.1., are incubated in flat bottomed microtiter plates (0.5 x $10^5$ cells in 50 μl per well) for 88 hours at 37°C in 6% $CO_2$ in RPMI medium (Gibco) with 10% fetal calf serum (FCS) in the presence of Con A (2.5 μg/ml) and 5 to 20 mM NAC. Proliferation is evaluated by incorporation of [3H]thymidine (Amersham International, UK) and cell number count.

NAC enhanced proliferation of T cells under these conditions by a factor of 2 to 2.5 at a concentration of 5-10 mM (Eylar et al., 1993). In cultures of peripheral blood T cells, 10 mM NAC stimulated growth by at least 4-6-fold after two passages.

Using a protocol of Jeannin et al. (1995), purified peripheral blood T cells (2 x $10^4$ cells/200 μl per well) are cultured in 96-well culture plates (Nunc, Denmark) according to Example 1.2. and stimulated with Con A (10 μg/ml) in the absence or presence of different concentrations (0.5-20 mM) of NAC. After 48 hours, cells are pulsed with [3H] thymidine (Amersham International, UK) for 6 hours before determination of incorporated radioactivity.

Using this protocol, NAC increased the proliferation of purified T in a dose-dependent manner (Jeannin et al., 1995). At 20 mM NAC, the increase in proliferation was approximately 4-fold.

*1.3.2. NAC-mediated enhancement of lymphokine production by T cells.*

Using a protocol of Eylar et al. (1993), T cells purified from human peripheral blood according to Example 1.1., are incubated in 24-well microtiter plates (1 x $10^6$ cells in 1 ml per well) for 40 hours at 37°C in 6% $CO_2$ in RPMI 1640 medium (Gibco BRL, Switzerland) with 10% fetal calf serum (FCS) in the presence of Con A (2.5 μg/ml) and 20 mM NAC. After removal of cells by centrifugation, aliquots of the supernatant are used for ELISA-analysis of lymphokines as described by Eylar et al. (1993).

Using this protocol, NAC enhanced IL-2 production by T cells approximately 1.5-fold (Eylar et al., 1993).

Using a protocol of Jeannin et al. (1995), purified peripheral blood T cells (2 x $10^5$ cells/200 μl per well) are cultured in 96-well culture plates (Nunc, Denmark) according to Example 1.2. and stimulated with Con A (10 μg/ml) in the absence or presence of different concentrations (0.5-20 mM) of NAC. After 16 and 72 hours of culture, cell-free supernatants are collected and assayed for IL-2, IL-4, IL-5 and IFNγ as described by Jeannin et al. (1995).

Using this protocol, NAC enhanced IL-2 production by T cells (up to 5 mM NAC), decreased in a dose-dependent manner IL-4 production (maximal at 20 mM NAC), whereas IL-5 and IFNγ production was not affected by NAC (Jeannin et al., 1995).

*1.3.3. NAC-mediated enhancement of* B *cell proliferation*

Using a protocol of Jeannin et al. (1995), purified tonsillar B cells (2 x $10^5$ cells/200 μl per well) are cultured in 96-well culture plates (Nunc, Denmark) according to Example 1.2. and stimulated with 3 ng/ml IL-4 (Prospec, USA) plus 1 μg/ml anti-CD40 monoclonal antibody (Serotec, UK) in the absence or presence of different concentrations (0.5-20 mM) of NAC. After 3 days cells are pulsed with [3H]thymidine (Amersham International, UK) for 6 hours before determination of incorporated radioactivity and/or counting of cell numbers.

Using this protocol, NAC increased the proliferation of purified B cells in a dose-dependent manner (Jeannin et al., 1995). At 20 mM NAC, the increase in proliferation was at least 5-fold.

*1.3.4. NAC-mediated decrease of IgE and IgG4 production by* B *cells.*

Using a protocol of Jeannin et al. (1995), purified tonsillar B cells (2 x $10^5$ cells/200 μl per well) are cultured in 96-well culture plates (Nunc, Denmark) according to Example 1.2. and stimulated with 3 ng/ml IL-4 (Prospec, USA) plus 1 μg/ml anti-CD40 monoclonal antibody (Serotec, UK) in the absence or presence of different concentrations (0.5-20 mM) of NAC. After 14 days, cell-free supernatants are collected and assayed for IgE or IgG4 as described (Jeannin et al., 1995).

Using this protocol, NAC decreased both IgE and IgG4 production in a dose-dependent manner. A significant decrease of IgE and IgG4 was observed with 0.5 mM NAC and was maximal with 20 mM NAC (Jeannin et al., 1995).

**EXAMPLE 1.4. In vitro effects of GSM on T and B cells**

The experiments of EXAMPLE 1.4. demonstrate the various effects of S-methylglutathione (GSM) on cultured T and B cells. GSM is a glutathione donor and has been shown to reproduce under in vitro conditions the in vivo effect of NAC on rat cardiomyocytes (Cailleret et al., 2004). The solubility of GSM in water is 2.61 g/liter (8.1 mM).

*1.4.1. GSM-mediated effects on T cell proliferation.*

The experiments are performed as described in Example 1.3.1. with the exception that NAC is replaced by GSM (0.1 mM-3 mM).

*1.4.2. GSM-mediated effects on lymphokine production by T cells.*

The experiments are performed as described in Example 1.3.2. with the exception that NAC is replaced by GSM (0.1 mM-3 mM).

*1.4.3. GSM-mediated effect on IgE and IgG4 production by B cells.*

The experiments are performed as described in Example 1.3.4. with the exception that NAC is replaced by GSM (0.1 mM-3 mM).

EXAMPLE 1.5. In vitro effects of ASA and SA on T cells

The experiments of EXAMPLE 1.5. demonstrate the various effects of acetylsalicylic acid (ASA, aspirin) and sodium salicylate (SA) on cultured T cells.

*1.5.1. ASA-mediated inhibition of IL-4 secretion by CD4+ T cells*

Using a protocol of Cianferoni et al. (2001), CD4+ T cells purified from human peripheral blood according to Example 1.1., are cultured in complete medium consisting of 90% RPMI 1640 (Biofluids, USA), 10% fetal calf serum (Summit, USA), 2 mM GluaMax-I (Life Technologies, USA), and 40 μg/ml gentamicin.

For the evaluation of ASA-mediated effects on lymphokine production including IL-2, IL-4, IL-13, and IFNγ, CD4+ T cells (2 x $10^6$/0.5 ml) are stimulated for 20 hours with the $Ca^{2+}$ ionophore A23167 (0.5 μg/ml) and the protein kinase agonist PMA (phorbol myristate acetate) (25 ng/ml) as described by Cianferoni et al. (2001). Fifteen minutes before stimulation, increasing concentrations of ASA (0.1 mM-3 mM) in 0.1% DMSO or the corresponding amount of DMSO carrier (0.1%) are added to the incubation mixture. Cytokine titers are measured in cell-free supernatants collected 18-20 hours after stimulation by using from Biosource (USA) Cytoscreen human IL-4 (detection limit: 7.8 pg/ml), Cytoscreen human IFNγ (detection limit: 15.6 pg/ml), an enzyme-amplified sensitivity immunoassay for human IL-2 (detection limit: 1.1 U/ml), and from Immunotech (France) an enzyme-linked immunosorbent assay for IL-13 (detection limit: 7.8 pg/ml).

Using this protocol, ASA did not affect the production of IL-2, IL-13 and IFNγ, but it inhibited IL-4 secretion by 47% at 1 mM ASA (Cianferoni et al., 2001).

*1.5.2. SA-mediated inhibition of IL-4 secretion by CD4+ T cells*

Using a protocol of Cianferoni et al. (2001), CD4+ T cells purified from human peripheral blood according to Example 1.1., are cultured in complete medium consisting of 90% RPMI 1640 (Biofluids, USA), 10% fetal calf serum (Summit, USA), 2 mM GluaMax-I (Life Technologies, USA), and 40 μg/ml gentamicin.

For the evaluation of SA-mediated effects on lymphokine production including IL-2, IL-4, IL-13, and IFNγ, CD4+ T cells (2 x $10^6$/0.5 ml) are stimulated for 20 hours with the $Ca^{2+}$ ionophore A23167 (0.5 μg/ml) and the protein kinase agonist PMA (phorbol myristate acetate) (25 ng/ml) as described by Cianferoni et al. (2001). Fifteen minutes before stimulation, increasing concentrations of SA (1 mM- 5 mM) in PBS are added to the incubation mixture. Cytokine titers are measured in cell-free supernatants collected 18-20 hours after stimulation by using from Biosource (USA) Cytoscreen human IL-4 (detection limit: 7.8 pg/ml), Cytoscreen human IFNγ (detection limit: 15.6 pg/ml), an enzyme-amplified sensitivity immunoassay for human IL-2 (detection limit: 1.1 U/ml), and from Immunotech (France) an enzyme-linked immunosorbent assay for IL-13 (detection limit: 7.8 pg/ml).

**EXAMPLE 1.6. In vitro inhibition of TNFR1 expression in MRC-5 cells by specific antisense oligonucleotides**

In this example, antisense oligodeoxynucleotides (ODNs) are used which target the 3'-polyadenylation [poly(A)] signal loci on the TNFR1 mRNA as described by Ojwang et al. (1997a) and Ojwang et al. (1997b). To enhance cellular uptake of the antisense ODNs and their subsequent release from endosomal compartments, the TNFR1-specific antisense ODNs are formulated with a cationic liposomal preparation (Cellfectin II; Life Technologies, USA) containing the cationic lipid tetramethyltetrapalmitylspermine and the phospholipid dioleoylphosphatidylethanolamine

Inhibition of TNFR1 expression by these antisense ODNs is analyzed in human lung embryo fibroblasts (MRC-5 cells) as described by Ojwang and Rando (1999). Analysis of TNFR2 expression in these cells is used as internal control. The effect of antisense ODNs to TNFR1 can be monitored by TNFR1 RNA synthesis, TNFR1 protein synthesis and TNF-alpha-dependent induction of IL6 and/or IL-8 (for details, see Ojwang and Rando, 1999).

*1.6.1. Synthesis of* a *pPT TNFR1-specific antisense ODN.*

The TNFR1-specific antisense ODN is synthesized using partial phosphorothioate linkages (pPT) to confer nuclease resistance and to reduce nonspecific side effects associated with ODNs having a sulfur moiety at every internucleoside linkage. The Beaucage reagent (Iyer et al., 1990) is used for the synthesis of the TNFR1-specific antisense pPT ODN.

*Sequence of the 22mer pPT TNFR1-specific antisense ODN* a* g* a* a* t t t t t a* g* t* g* t* a t g t a* c* a* a (asterics indicate phosphorothioate linkages)

Purification by anion exchange chromatography, desalting and analysis for purity is performed as described (Ojwang and Rando, 1999). Using the purified 22mer pPT antisense ODN, melting temperature evaluations revealed a Tm of 36.6°C for a DNA:DNA duplex and a Tm of 31.1°C for a DNA:RNA duplex (Ojwang and Rando, 1999).

*1.6.2. Culture of human lung embryo fibroblasts.*

Human lung embryo fibroblasts (MRC-5 cells) are grown in Opti-MEM consisting of minimal essential medium (MEM) supplemented with Earl's salts, L-glutamine (Gibco BRL, Life Technologies, USA), 10% heat inactivated fetal bovine serum (Gibco BRL), penicillin (100 U/ml), and streptomycin (100 μg/ml) as described (Ojwang and Rando, 1999).

*1.6.3. Inhibition TNFR1 expression by the 22mer pPT antisense ODN.*

*Formulation of ODN with Cellfectin.* Varying concentrations of the pPT antisense ODN (ranging from 0.1-1.0 μM) in 25 μl Opti-MEM (see 1.6.2.) are mixed with 2.5 μl of Cellfectin II in Opti-MEM (1 mg/ml). After 15 min incubation at room temperature, 225 μl Opti-MEM is added to each sample.

Treatment *of cells with formulated ODN.* MRC-5 cells are seeded at a cell density of 1 x $10^4$ cells/well in 96-well plates overnight at 37°C, then rinsed twice with 100 μl of Opi-MEM, followed by the addition of 100 μl of ODN/Cellfectin mixture in Opti-MEM. The final concentration of Cellfectinper well is μg/ml. After 4 hours at 37°C, the medium is removed and the wells are rinsed twice with 0.25% MEM and then 80 μl of fresh 0.25% MEM is added to each well. After another 18 hours at 37°C, samples are analyzed for TNFR1 and TNFR2 expression or stored at -80°C until used.

*Expression analysis of TNFR1 and TNFR2.* Cells are rinsed once with PBS and then treated with a buffer containing 40 mM Tris-HCl, pH 7.4, 1 mM EDTA, pH 8.0, and 150 mM NaCl for 10 min at room temperature. The rounded cells are then scraped, transferred into microfuge tubes and centrifuged at 3500 rpm (approx. 1000 x g) for 6 min at room temperature. The supernatants are removed and stored at -80°C until used. The cell pellets are resuspended in 200 μl of 0.25 mM Tris-HCl, pH 7.4. The cell-associated proteins are prepared by gentle freeze-thawing. The cells are placed on ethanol in dry ice for 5 min and then transferred into a water-bath incubator at 37°C for another 5 min. The cycle is repeated 5 times. The cell lysates are stored at -80°C until used.

Both supernatants and cell lysates are assayed for TNFR1 and TNFR2 (internal control) levels using ELISA kits (R&D Systems, USA) according to the manufacturer's instructions.

Using this protocol, the TNFR1-specific pPT antisense ODN effected at a concentration of 0.5 μM a 35% inhibition of TNFR1 expression in MRC-5 cells (Ojwang and Rando, 1999).

Literature for Example 1

Biddison, Curr. Protcols Cell Biol.; 1998.
Cailleret et al., Circulation 109: 406-411; 2004.
Cianferoni et al., Blood 97: 1742-1749; 2001.
Eylar et al., Intern. Immunol. 5: 97-101; 1993.
Iyer et al., J. Org. Chem. 55: 4699-4704 ; 1990.
Jeannin et al., J. Exp. Med. 182: 1785-1792; 1995.
Ojwang et al., Biochemistry 36 : 6033-6045 ; 1997a.
Ojwang et al., Antisense Nucleic Acid Drug Dev. 7 : 447-459 ; 1997b.
Ojwang and Rando, Methods : a companion to Meth Enzymol. 18: 244-251; 1999.
Robinson et al., Curr. Protcols Cytometry; 1998.


**EXAMPLE 2. SYNTHESIS AND CHARACTERIZATION OF THERMOGELLING PLGA-PEG-PLGA HYDROGELS**

**EXAMPLE 2.1. Synthesis and characterization of thermogelling PLGA-PEG-PLGA hydrogels**

The biodegradable triblock polymer described in this example has a PLG/PEG weight ratio of 2.3 (70/30), and a lactide/glycolide molar ratio of approx. 15/1. Synthesis of the triblock copolymer is performed according to published protocols (Qiao et al., 2005; patent application WO 02/102309).

*2.1.1. Copolymer synthesis*

Polyethylene glycol (PEG 1000) is purchased from Fluka, poly(DL-lactide) from Sigma, glycolide (1,4-Dioxane-2,5-dione) from Sigma, and stannous 2-ethylhexanoate from Aldrich.

A total of 25 g of DL-lactide, glycolide and PEG are used for polymerization (16.6 g DL-lactide, 0.9 g glycolide, 7.5 g PEG 1000). Under nitrogen atmosphere, PEG 1000 is dried under vacuum and stirring at 120°C for 2 h in a vigorously dried Erlenmeyer reaction flask. Then the reaction flask is filled with dry argon. DL-lactide and gycolide monomers are added under stirring followed by the addition of Stannous 2-ethylhexanoate (0.2% w/w). Then the tube is sealed under argon. The sealed flask was immersed and kept in an oil bath thermostated at 150°C. After 8 h the flask was cooled to room temperature, and the product was dissolved in cold water. After completely dissolved, the copolymer solution is heated to 80°C to precipitate the copolymer and to remove the water-soluble low molecular weight copolymers and unreacted monomers. The supernatant is decanted, the precipitated copolymer is again dissolved in cold water followed by heating to induce precipitation. This process of dissolution followed by precipitation is repeated three times. Finally, the copolymer is dried under vacuum at room temperature until constant weight.

*2.1.2. Molecular weight determination*

The molecular weight of the copolymer is determined by gel permeation chromatography using polystyrene standards as described by Qiao et al. (2005).

*2.1.3. Measurement of gelation temperature*

The gelation temperature is determined as described by Qiao et al. (2005). A 2 ml transparent vial is filled with 200 μl water solution of the copolymer (20% w/w and 25% w/w), is placed in a water bath. The solution is heated in 1°C steps beginning at 26°C in a thermomixing device (Eppendorf). At each temperature step the gelation is checked by careful inversion of the tube. When the solution is not free-flowing, gelation of the solution occurred, the temperature read from the thermometer is determined as gelation temperature.

**EXAMPLE 2.2. In vitro degradation of thermogelling PLGA-PEG-PLGA hydrogels**

The in vitro degradation behavior of the copolymer of Example 2.1 is evaluated by the mass loss and the molecular weight reduction with time upon incubation in phosphate-buffered saline.

Samples (0.5 ml) are incubated in phosphate-buffered saline pH 7.4 at 37°C under mild agitation in a water bath. The solid residues are removed from the incubation medium at scheduled time intervals and lyophilized. The samples are weighted and the weight loss is calculated. Then the solid residues are solved in cold water and analyzed by gel permeation chromatography using polystyrene standards as described by Qiao et al. (2005).

**EXAMPLE 2.3. Biodegradation of thermogelling PLGA-PEG-PLGA hydrogels**

To test the *in vivo* gel formation behavior and the degradation characteristics of PLGA-PEG-PLGA hydrogels, the hydrogel is injected subcutaneously into mice that have been anesthetized with ethyl ether. The resulting gel implants are then allowed to develop in vivo over the experimental period. At each of the post-injection sampling points, the mice are sacrificed, the gel implants are removed from the subcutaneous injection site, and the removed gel implants are analyzed as described in Example 2.2.

Two groups of wild-type BALB/c mice (each group: 16 mice) are analyzed for the in vivo gel formation behavior and the degradation characteristics: group 1: 20% (w/w) polymer; group 2: 25% (w/w) polymer. The analysis is performed according to following schedule: implantation of 200 μl polymer on day 0, analysis of size in two mice on days 1,2,3,4,5,10,20 and 30.

**Literature for Example 2**

Qiao et al., Int. J. Pharm. 294: 103-112; 2005.

**EXAMPLE 3. RELEASE OF TNFR1 INHIBITORS FROM THERMOGELLING PLGA-PEG-PLGA HYDROGELS**

**EXAMPLE 3.1.In vitro release characteristics of PLGA-PEG-PLGA/protein-composits**

This example shows the in vitro release characteristics of PLGA-PEG-PLGA/protein composits using polyclonal anti-lysozyme antibodies as model protein.

The PLGA-PEG-PLGA triblock copolymer of Example 2.1 is dissolved at room temperature in water, containing 30% w/v polymer. 20 μl 10xPBS, 12 μl water and 1 μl polyclonal rabbit anti-lysozyme serum (80 mg/ml) are added to 167 μl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released antibodies is determined by ELISA with lysozyme coated microtiter plates (96 wells, Greiner) and anti-rabbit alkaline phosphate antibody conjugate (Sigma) using para-nitrophenyl phosphate (pNPP) as chromogenic enzyme substrate. The absorbtion at 405 nm correlates with the amount of the released protein.

**EXAMPLE 3.2. In vitro release characteristics of PLGA-PEG-PLGA/N-acetyl-L-cysteine composits**

The PLGA-PEG-PLGA triblock copolymer of Example 2.1 is dissolved at room temperature in water, containing 30% w/v polymer. 20 μl 10xPBS, 3 μl water, and 10 μl of NAC (130.5 mg/ml PBS (0.8 M) at pH 7.4,) are added to 167 μl gel solution (final conc. 40 mM). Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released NAC is determined by an assay for sulfhydryl groups using the reaction of 4,4'-dithiopyridine (4-PDS) with thiols, which gives the corresponding 4-thiopyridone (4-TP) (Grassetti and Murray, 1967). Using this assay, 15 nmol-0.5 μmol NAC is dissolved in 1.5 ml PBS pH 7.2 and mixed with 1.5 ml 2 mM 4-PDS in PBS pH 7.2. UV absorbance of 4-TP is determined at 324 nm (molar extinction coefficient 1.198 x $10^4$) immediately after mixing. The method permits determination of less then 0.5 μg SH (Grassetti and Murray, 1967).

For the determination of NAC concentrations below 15 nmol/1.5 ml (10 μM), a more sensitive spectrophotometric assay is used which is based on a coupled two-step redox and complexation procedure (Kukoc-Modun and Radic, 2011). In the first step, Fe(III) is reduced by the sulfhydryl group of NAC to Fe(II). Subsequently, Fe(II) is complexed with 2,4,6-tripyridyl-S-triazine (TPTZ). Acetate buffer (0.5 M), pH 3.6 (2.0 ml) is mixed with 125 μl 10 mM $FeCl_3$, 125 μl 10 mM TPTZ in 0.12 M HCl, and 100 μl NAC solution. After incubation for 60 min at room temperature, the absorbance of the produced Fe(II)-TPTZ complex, which remains constant for 24 hours, is measured at 593 nm. The detection limit of this method for NAC is 0.14 μM.

**EXAMPLE 3.3. In vitro release characteristics of PLGA-PEG-PLGA/glutathione (GSH) composits**

In this example, reduced non-methylated glutathione (GSH) is used to study the release characteristics of glutathione and derivatives thereof including S-methylglutathione (GSM) from PLGA-PEG-PLGA hydrogels.

The PLGA-PEG-PLGA triblock copolymer of Example 2.1 is dissolved at room temperature in water, containing 30% w/v polymer. 20 μl 10xPBS, 3 μl water, and 10μl of a suspension containing 800 mM GSH in water (257.2 mg/ml), are added to 167 μl gel solution. The GSH dissolves completely and the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released GSH is determined by an assay for sulfhydryl groups using the reaction of 4,4'-dithiopyridine (4-PDS) with thiols, which gives the corresponding 4-thiopyridone (4-TP). Using this assay, 15 nmol-0.5 μmol GSH is dissolved in 1.5 ml PBS pH 7.2 and mixed with 1.5 ml 2 mM 4-PDS in PBS pH 7.2. UV absorbance of 4-TP is determined at 324 nm (molar extinction coefficient 1.198 x $10^4$) immediately after mixing. The method permits determination of less then 0.5 μg SH (Grassetti and Murray, 1967).

For the determination of GSH concentrations below 15 nmol/1.5 ml (10 μM), a more sensitive spectrophotometric assay is used which is based on a coupled two-step redox and complexation procedure (Kukoc-Modun and Radic, 2011). In the first step, Fe(III) is reduced by the sulfhydryl group of GSH to Fe(II). Subsequently, Fe(II) is complexed with 2,4,6-tripyridyl-S-triazine (TPTZ). Acetate buffer (0.5 M), pH 3.6 (2.0 ml) is mixed with 125 μl 10 mM $FeCl_3$, 125 μl 10 mM TPTZ in 0.12 M HCl, and 100 μl GSH solution. After incubation for 60 min at room temperature, the absorbance of the produced Fe(II)-TPTZ complex, which remains constant for 24 hours, is measured at 593 nm.

**EXAMPLE 3.4. In vitro release characteristics of PLGA-PEG-PLGA/sodium salicylate (SA) composits**

The PLGA-PEG-PLGA triblock copolymer of Example 2.1 is dissolved at room temperature in water, containing 30% w/v polymer. 20 μl 10xPBS, 3 μl water, and 10 μl of 800 mM SA (128.2 mg/ml) in water are added to 167 μl gel solution. Then the dissolved formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released SA is determined by derivatization with Fe(III) and quantification of the violet coloured tetraaquosalicylatroiron (III) complex at 530 nm (www.jenway.com (Application note A09-009A)).

**EXAMPLE 3.5. In vitro release characteristics of composits comprising PLGA-PEG-PLGA and liposome-embedded TNFR1-specific antisense oligonucleotides**

In this experiment, non-modified TNFR1-specific antisense oligodeoxynucleotides (TNFR1-ODN), targeting the 3'-polyadenylation [poly(A)] signal loci on the TNFR1 mRNA (Ojwang et al., 1997a; Ojwang et al., 1997b), are complexed with a cationic liposomal preparation (Cellfectin II; Life Technologies, USA) containing the cationic lipid tetramethyltetra-palmitylspermine and the phospholipid dioleoylphosphatidyl-ethanolamine, and then embedded into a PLG-PEG-PLGA hydrogel.

*Sequence of the TNFR1-specific antisense ODN*

a g a a t t t t t a g t g t a t g t a c a a

*3.5.1. Complexation of TNFR1-ODN with Cellfectin.*

TNFR1-specific antisense ODN (1.0 μM) in 200 μl PBS, pH 7.4, are mixed with 20 μl of Cellfectin in PBS, pH 7.4 (1 mg/ml) and incubated for a 15 min incubation at room temperature.

*3.5.2. Preparation of hydrogel-ODN/Cellfectin composits.*

The PLGA-PEG-PLGA triblock copolymer of Example 2.1 is dissolved at room temperature in water, containing 30% w/v polymer. 20 μl 10xPBS and 13 μl of TNFR1-ODN/Cellfectin complexes are added to 167 μl gel solution.

*3.5.3. Release of TNFR1-ODN from PLGA-PEG-PLGA.*

The formulation is placed in a 2 ml vial, incubated at 37°C for 2 min until gelling, and 1.8 ml of PBS, pH 7.4, is added. The vial is incubated at 37°C. At specified sample collection times, the supernatant is withdrawn and replaced by an identical volume of PBS pH 7.4 to maintain release conditions.

*3.5.4. Determination of released TNFR1-ODN.*

First, TNFR1-ODNs are liberated from the liposomal complex by the addition of 200 μl 100 mM Tris-HCl, pH 8.0, 5 mM EDTA, 10% Triton X-100 to the supernatant (1.8 ml).

Thereafter, the amount of released TNFR1-ODN is determined in 100 μl aliquots of the supernatants by a one-step hybridization ELISA (Wei et al., 2006). Capture and detection of TNFR1-ODN is accomplished on a one-strand capture probe, which is complementary to the TNFR1-ODN without an overhang and contains biotin at the 3'-end and digoxigenin at the 5'-end. For TNFR1-ODN capture probe hybridization 200 nM capture probe in hybridization buffer solution (capture ODN concentration of 200 nM ensures highest hybridization with analytes) is first heated to 95°C for 5 min in a heating block to disrupt possible secondary structures. Then 100 μl of the capture probe solution is added to 100 μl of the TNFR1-ODN solution in a polystyrene 96-well plate. The mixture is incubated at 42°C for 2.5 hours. After TNFR1-ODN capture probe hybridization, 150 μl of the solution is transferred to 96-well Reacti-Bind NeutrAvidin-coated polystyrene plates (Pierce, USA) and incubated for 30 min at 37°C to allow attachment of the biotin-labeled duplex to the coated plates. The plates are washed 6 times with washing buffer, followed by the addition of 150 μl S1 nuclease (60 U in 100 mM NaCl), After incubation for 2 hours at 37°C, the plates are washed 6 times with washing buffer, followed by the addition of 150 μl anti-digoxigenin-alkaline phosphatase (AP) (Roche, USA) diluted 1:2500 with bovine serum albumin blocking buffer (Superblock buffer in Tris-buffered saline, TBS; Pierce, USA) and subsequent incubation for further 30 min at 37°C with gentle shaking. Thereafter, the plates are washed again 6 times with washing buffer, followed by the addition of 150 μl Attophos (Promega, USA) substrate solution (36 mg Attophos in 60 ml diethanolamine buffer). After 30 min at 37°C the generated fluorescence is measured at Ex430/Em 560 (filter 550 nm) using a fluorescence microtiter plate reader.

Buffers used for the determination of TNFR1-ODNs include the hybridization buffer (60 mM Na phosphate, pH 7.4, 1.0 M NaCl, 5 mM EDTA, and 0.2% Tween 20), the washing buffer (25 mM Tris-HCl, pH 7.2, 0.15 M NaCl, and 0.2% Tween 20), and the dilution buffer for ODN standards (10 mM Tris-HCl, pH 8.0, 1 mM EDTA).

According to Wei et al. (2006) the one-step hybridization ELISA is linear from 0.025 to 50 nM ODN including phosphorothioate oligonucleotides. Using 60 U S1 nuclease per well, a cutting efficiency of 99.3-99.7% is achieved.

Another procedure to quantify the amount of released oligonucleotide is to run samples on a 1.5% agarose gel in 0.1x TAE buffer with ethidium bromide. The gel is illuminated with UV light and the taken photo of the resulting shading is analyzed using densitometric software (ImageJ, NIH Bethesda).

EXAMPLE 3.6. In vitro release characteristics of PLGA-PEG-PLGA/mutant TNF-alpha composits

In this example, mutant TNF-alpha R1antTNF (Shibata et al., 2008) capable of blocking the binding of wild-type TNF-alpha to TNFR1 is embedded into a PLG-PEG-PLGA hydrogel.

*3.6.1. Expression and purification of* the mutant *TNF-alpha R1antTNF.*

After transfection of E. *coli* BL21(DE3) with a plasmid encoding the R1antTNF gene under control of the T7 promotor, R1antTNF is overexpressed, recovered from the inclusion bodies, washed with 2.5% Triton X-100 and 0.5 M NaCl in TES buffer, and the solubilized in 6 M guanidine-HCl, 0.1 M Tris-HCl, pH 8.0, and 3 mM EDTA (Shibata et al., 2008). The solubilized protein is reduced with 10 mg/ml dithioerythritol for 4 h at room temperature, and then refolded by 100-fold dilution in a refolding buffer (0.1 M Tris-HCl, pH 7.4, 2 mM EDTA, 0.5 M arginine, and 551 mg/liter oxidized glutathione). After dialyzing against against 20 mM Tris-HCl, pH 7.4, containing 100 mM urea, the active trimeric proteins are purified by Q-Sepharose chromatography (GE Healthcare Bioscience, USA) and additional gel filtration on Superose 12 (GE Healthcare Bioscience, USA).

*3.6.2. Release of R1antTNF from PLGA-PEG-PLGA.*

The PLGA-PEG-PLGA triblock copolymer of Example 2.1 is dissolved at room temperature in phosphate buffered saline (PBS) at pH 7.4, containing 25% or 20% w/v polymer. Varying volumes (10 μl, 20 μl, 40 μl, 80 μl) of 1 mg R1antTNF/ml PBS, pH 7.4, are added to 200 μl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

The amount of released R1antTNF is determined by bicinchoninc acid (BCA) assay using BCA™ Protein Assay Kit (Pierce, USA).

**EXAMPLE 3.7. Combined in vitro release of N-acetylcysteine (NAC)and liposome-embedded TNFR1-specific antisense ODNs from PLGA-PEG-PLGA composits**

The combination of NAC and TNFR1-specific antisensense oligodeoxynucleotides (TNFR1-ODNs) may be useful, since both therapeutics act on different targets. NAC acts on T cells by increasing interleukin-2 (IL-2) production, synthesis and turnover of IL-2 receptors. Up-regulation of IL-2 and its receptor is important for the induction of tolerance since in the presence of IL-2 TNF-alpha-mediated activation of Tregs via interaction with TNFR2 results in proliferation, up-regulation of FoxP3 expression and increase of their suppressive activity. Furthermore, the decreased production of human IL-4 in stimulated peripheral blood T cells and T helper cells after treatment with NAC is helpful since IL-4 plays a key role in the development of allergic inflammation. On the other hand, specific inhibition of TNFR1-mediated signaling by TNFR1-specific antisense ODNs is important to effectively inhibit inflammatory processes at the site of antigen or allergen presentation and, thereby, to favor TNFR2-mediated activation of Tregs.

After complexation of TNFR1-ODNs with Cellfectin II as described in Example 3.5., the PLGA-PEG-PLGA triblock copolymer of Example 2.1 is dissolved at room temperature in water, containing 30% w/v polymer. 20 μl 10xPBS and 13 μl of a 1:1 mixture of TNFR1-ODN/Cellfectin complexes and 0.8 M NAC in PBS, pH 7.4, are added to 167 μl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

Analysis of released NAC and TNFR1-ODNs is performed as described in Example 3.2. and in Example 3.5., respectively.

**EXAMPLE 3.8. Combined in vitro release of sodium salicylate (SA) and liposome-embedded TNFR1-specific antisense ODNs from PLGA-PEG-PLGA composits**

The combination of SA and TNFR1-specific antisensense oligodeoxynucleotides (TNFR1-ODNs) may be useful, since both therapeutics act on different targets. The inhibitory effect of salicylates is caused by activation of the p38 mitogen-activated kinase which leads to inhibition of TNF-alpha-induced I-kappa B-alpha phosphorylation and degradation. As a result of NF-kappa B inhibition, high doses of salicylates can interfere with Th1 cell differentiation and effector responses. As mentioned in Example 3.8., specific inhibition of TNFR1-mediated signaling by TNFR1-specific antisense ODNs is important to effectively inhibit inflammatory processes at the site of antigen or allergen presentation and, thereby, to favor TNFR2-mediated activation of Tregs.

After complexation of TNFR1-ODNs with Cellfectin II as described in Example 3.5., the PLGA-PEG-PLGA triblock copolymer of Example 2.1 is dissolved at room temperature in water, containing 30% w/v polymer. 20 μl 10xPBS and 13 μl of a 1:1 mixture of TNFR1-ODN/Cellfectin complexes and 800 mM SA in water, are added to 167 μl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

Analysis of released SA and TNFR1-ODNs is performed as described in Example 3.4. and in Example 3.5., respectively.

**EXAMPLE 3.9. Combined in vitro release of NAC, SA and liposome-embedded TNFR1-specific antisense ODNs from PLGA-PEG-PLGA composits**

As described in Examples 3.7. and 3.8., NAC,SA and TNFR1-ODNs act on different targets. Therefore, a combination of the three therapeutics may be useful.

After complexation of TNFR1-ODNs with Cellfectin as described in Example 3.5., the PLGA-PEG-PLGA triblock copolymer of Example 2.1 is dissolved at room temperature in water, containing 30% w/v polymer. 20 μl 10xPBS and 13 μl of a 1:1:1 mixture of TNFR1-ODN/Cellfectin complexes, 0.8 M NAC in water, and 800 mM SA in water, are added to 167 μl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

Analysis of released NAC, SA and TNFR1-ODNs is performed as described in Example 3.2., Example 3.4., and in Example 3.5., respectively.

Literature for Example 3

Grassetti and Murray, Arch. Biochem. Biophys. 119: 41-49; 1967.
Kukoc-Modun and Radic, Intern. J. Analyt. Chem. 2011: article ID 140756; 2011
Shibata et al., J. Biol. Chem. 283: 998-1007; 2008.
Wei et al., Pharmaceutical Res. 23 : 1251-1264 ; 2006. www.jenway.com (Application note A09-009A) The quantitative determination of the aspirin content of tablets using UV and visible wavelength spectroscopy.

**EXAMPLE 4. COMBINED RELEASE OF TNFR1 INHIBITORS AND OTHER TOLERANCE-INDUCING THERAPEUTICS FROM THERMOGELLING PLGA-PEG-PLGA HYDROGELS**

The combination of TNFR1-specific inhibitors and other tolerance-inducing therapeutics including IL-4/IL-13 antagonists and complement component C3-depleting agents (e.g., rhC3-derivative H5) may be useful, since these therapeutics act on different targets as described in the preferred embodiment of the present invention.

**EXAMPLE 4.1. Combined in vitro release of TNFR1-ODNs and the IL-4/IL-13 antagonist QY from PLGA-PEG-PLGA composits**

*4.1.1. Cloning, expression and purification of the murine IL-4 antagonist QY*

The murine IL-4 mutant QY (Q116D/Y119D) is analogous to the R121D/Y124D double mutant of human Il-4, and an excess of the murine QY mutant has been shown to completely inhibit responses toward wild-type murine IL-4 (see patent application EP 11075261.5).

*Cloning of the murine IL-4 antagonist QY (Q116D/Y119D) according to patent application* EP 11075261.5*. The sequence of* the native mature murine IL4 protein is modified to include a thrombin cleavable N-terminal 6xHis-tag and the QY (Q116D/Y119D) mutations (see SEQID 10). The protein sequence is translated into DNA using codon optimization for expression in prokaryontic E. *coli* cells. The resulting sequence (SEQID 9) is synthesized and amplified using PCR with Pfu polymerase according to the manufacture and annealing temperatures rising from initial 5 cycles at 50°C to 60°C and 30 cycles total. The resulting PCF fragment is gel-purified in 1% agarose (GeneJET Gel Extraction Kit, Fermentas) and transferred to a restriction enzyme double digest containing Nde I and *Xho* I (Fermentas). The restriction digest is again gel-purified and the fragment is ligated into *Nde* I and *Xho I* cut pET-22b (Novagen) expression vector.

*Expression according* to *patent application* EP 11075261.5. Recombinant His-tagged murine IL-4 antagonist QY (Q116D/Y119D) is expressed in E. *coli* (BL21(DE3)pLysS; Novagen) at 37°C. Cultures are grown in 1-L batches in minimal medium containing 100 μg/ml ampicillin and 34 μg/ml chloramphenicol. The minimal medium contains the following components: 0.1 M phosphate buffer, pH 7.0, 2 mM $MgSO_4$, 17 mM NaCl, 0.1 mM $CaCl_2$, 5 mg/L $FeSO_4$, 0.2 mg/L $(NH_4)MO_7O_{24}$, 1 mg/L $H_3BO_3$, 0.2 mg/L $CoCl_2$, 0.2 mg/L $CuSO_4$, 2 mg/L $MnSO_4$, and 2 mg/L $ZnSO_4$, supplemented with thymidine, biotin, folic acid, pantothenic acid, niacinamide, pyroxidine phosphate, and thiamine (1 mg/L each). The expression is induced by the addition of isopropyl-D-thiogalactopyranoside to a final concentration of 1 mM after the culture reaches an $OD_{600}$ of approximately 0.6. The cells are allowed to grow for 4h and then harvested by centrifugation at 5000 x g for 10 min at 4°C. The pellet is stored at -20°C.

*Refolding from inclusion bodies.* The insoluble His-tagged murine IL-4 antagonist QY (Q116D/Y119D) is refolded according to patent application EP 11075261.5. The E. *coli* pellet containing the recombinant IL-4 antagonist QY is resuspended in 15 ml of 50 mM Tris-HCl, pH 8.0, and 5 mM EDTA (lysis buffer) containing 2 mg lysozyme and incubated at room temperature for 20 min. The cell lysate is diluted with 60 ml lysis buffer and sonicated. It is then centrifuged at 11,000 rpm for 30 min at 4°C to collect the pellet containing inclusion bodies. The pellet is washed twice with 60 ml with 2 M urea in lysis buffer and collected by centrifugation as described above. Finally, the pellet is resuspended in 25 ml of 6 M GdnHCl, 50 mM potassium phosphate, pH 8.0, and 1 mM reduced glutathione (extraction buffer) and stirred at room temperature for 10 min. The solution is centrifuged to remove the insoluble portion. The guanidine-extracted IL-4 antagonist QY is loaded onto 15 ml of Ni-charged HisTrap™ FF columns (GE Healthcare) equilibrated with the extraction buffer. The beads are washed with 8 column volumes of 7 M urea and 50 mM potassium phosphate, pH 6.8, to remove the cellular proteins. The His-tagged IL-4 antagonist QY is refolded on the column by the gradual removal of urea through a linear gradient expanding from 100% unfolding buffer to 100% refolding buffer (380 ml at a flow rate of 1 ml/min) using a fast protein liquid chromatography ÄKTA™ system (Pharmacia). The unfolding buffer is composed of 7 M urea, 100 mM NaCl, 50 mM potassium phosphate, pH 6.8, 1 mM reduced glutathione, and 1 mM oxidized glutathione; the refolding buffer has the same composition but without urea. After washing the column with 40 ml refolding buffer, the refolded His-tagged IL-4 antagonist QY is eluted from the column with 100 mM EDTA and 10 mM Tris-HCl, pH 8.0. EDTA is used to chelate divalent cations that can potentially cause protein aggregation during dialysis. The His-tagged IL-4 antagonist QY is then dialyzed against 50 mM TrisHCl, pH 8.0, 5 mM $CaCl_2$, and 100 mM NaCl to remove EDTA. The protein is concentrated to approximately 2 mg/ml.

*Removal of the His-tag.* The His-tag is cleaved overnight by incubation of each mg fusion protein with 10 U thrombin (Sigma) in PBS buffer. The mix is incubated at room temperature overnight. The reaction can be stopped with 1 mM PMSF. Cleavage products are separated by chromatography. Residual thrombin can be removed with pAminonezamidine-Agarose (Sigma) either by batch or chromatographic methods. The cleavage specificity is confirmed by N-terminus sequencing of blotted IL-4 antagonist QY. The His-tag is removed by passing the cleaved protein through Ni-fractogel beads. The purified samples are dialyzed in a desired buffer and kept at 4°C for short term storage or stored at -70°C in the presence of 25% glycerol.

*Analysis of correct refolding.* The structural integrity of the murine IL-4 antagonist QY is analyzed in a cellular binding assay of murine IL-4 and the murine IL-4 antagonist QY to the murine IL-4 receptor. The functionality of murine IL-4 and the murine IL-4 antagonist QY is determined in a T cell proliferation assay (for details see Example 4.1.2.).

*4.1.2. In vitro release of the murine IL-4 antagonist QY from PLGA-PEG-PLGA composits*

This experiment is performed as described in patent application EP 11075261.5. The PLGA-PEG-PLGA triblock copolymer of Example 2.1. is dissolved at room temperature in PBS pH 7.4 containing different concentrations of the murine IL-4 antagonist QY (0.5 mg/ml up to 2.0 mg/ml) to make a 25% w/w solution. Then 200 μl of the formulation is placed in a 2 ml vial, incubated at 37°C for 2 min until gelling, and 1.8 ml of PBS pH 7.4 is added. The vial is incubated at 37°C. At specified sample collection times, a sample is withdrawn and replaced by an identical volume of PBS pH 7.4 to maintain release conditions.

The amount of released murine IL-4 antagonist QY is determined by bicinchoninc acid (BCA) assay using BCA™ Protein Assay Kit (Pierce, USA) and by an ELISA-type assay as described in Example 19. The structural integrity of released murine IL-4 antagonist QY is determined by a cellular binding assay. The functionality of released murine IL-4 antagonist QY is determined by inhibition of IL-4-induced T cell proliferation.

*In vitro assays for murine IL-4* and *the murine IL-4* antagonist *QY.* These assays include an ELISA-type assay and cellular assays. The ELISA-type assay provides information about the integrity of the murine Il-4 epitope recognized by the anti-murine IL-4 antibody. Information about the structural integrity of the murine IL-4 antagonist QY is obtained from a cellular binding assay analyzing binding of murine IL-4 and the murine IL-4 antagonist QY to the murine IL-4 receptor. The functionality of murine IL-4 and the murine IL-4 antagonist QY is determined in a T cell proliferation assay according to patent application EP 11075261.5.

*ELISA analysis.* The concentration of murine IL-4 or murine IL-4 mutants is determined by ELISA (Mouse IL-4 ELISA MAX™ Deluxe, BioLegend GmbH, Fell) according to the manufacturers instructions.

*Cellular binding* assay *for murine IL-4 and murine IL-4 antagonist QY.* Binding of murine Il-4 or the murine IL-4 antagonist QY to IL-4 receptor molecules on the surface of murine CTLL-2 cells is detected by FACScan analysis using a biotinylated anti-IL-4 antibody and fluorescent-labeled streptavidin. Murine CTLL-2 cells are IL-2-dependent and typically express 2000-5000 Il-4 receptors per cell.

Murine CTLL-2 cells, obtained from ATCC (ATCC TIB 214), are cultured in RPMI 1640 containing 8% fetal calf serum, 100 units/ml penicillin and 100 μg/ml streptomycin, supplemented with 100 ng/ml IL-2. For the cellular binding assay 5 x $10^6$ cells are incubated for 30 min at 37°C in 150 μl of PBS (pH 7.4) containing 1% (w/v) BSA, 1 x $10^{-9}$ M recombinant murine (rmu) IL-4 or the murine IL-4 antagonist QY. The mixture is chilled to 4°C, washed once in a large volume of PBS (pH 7.4) containing 1% (w/v) BSA, resuspended in 150 μl of PBS (pH 7.4) and subjected to FACScan analysis.

*T cell proliferation assay.* Il-4 induced proliferation of CTLL-2 cells is determined by incorporation of [$^3$H] thymidine. The inhibitory effect of the murine IL-4 antagonist QY is determined by adding increasing amounts of the antagonist to IL-4.

CTLL-2 cells are incubated in 0.2 ml aliquots at a density of 5 x $10^5$/ml with log 2 dilutions of murine Il-4 for 3 days, before the amount of [$^3$H] thymidine incorporated during the final 4 hr is determined. The concentration of IL-4 yielding half-maximal response is used for determining the inhibitory activity of the murine IL-4 antagonist QY. In these experiments, log 2 dilutions of the murine IL-4 antagonist QY are added to wild-type murine IL-4.

*4.1.3. Combined in vitro release of TNFR1-ODNs and the murine IL-4 antagonist QY from PLGA-PEG-PLGA composits*

After complexation of TNFR1-ODNs with Cellfectin as described in Example 3.5., the PLGA-PEG-PLGA triblock copolymer of Example 2.1 is dissolved at room temperature in water, containing 30% w/v polymer. 20 μl 10xPBS and 13 μl of a 1:1 mixture of TNFR1-ODN/Cellfectin complexes and the murine IL-4 antagonist QY (2.0 mg/ml) in PBS, pH 7.4, are added to 167 μl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

Analysis of released the murine IL-4 antagonist QY and TNFR1-ODNs is performed as described in Example 4.1.2. and in Example 3.5., respectively.

EXAMPLE 4.2. Combined in vitro release of **TNFR1-ODNs** and the rhC3-derivative H5 from **PLGA-PEG-PLGA** composits

*4.2.1. Cloning, expression and purification of the rhC3-derivative H5 according US patent 7,553,931.*

The rhC3-derivative H5 contains the human β-chin and additionally the humanized Factor B- and Factor H-binding sites as well as the cleavage sites for Protease Factor I. In addition to the α-chain, the γ-chain as well as the C3a and the C3d regions were humanized (see US patent 7,553,931).

*Cloning of rhC3-derivative H5.* A fragment consisting of pUC18 and the 3'terminus of CVF is obtained from pUC18CVF utilizing Ecl136I and BglII restriction. Subsequently, said fragment is ligated with a fragment obtained from pcDNA3hC3 by EcoRI restriction, followed by mung bean nuclease digestion and BglII restriction. The latter fragment has a size of 1870 bp contained the 5' terminus of C3 cDNA. The vector ligated in this way (referred to as H2Δ2307 bp) contains 1800 bp of the C3 5' terminus and 1000 bp of the CVF 3' terminus. In order to completely construct rhC3-derivative H5, vector H2Δ2307bp is digested with BglII. The middle region of the C3-cDNA is isolated from plasmid pUC18hC3 via its BglII restriction sites and inserted into the vector. The resulting rhC3-derivative H5 is then inserted into an analogously digested pcDNA3-vector via the EagI-restriction sites. Subsequently, a Strep-tag is inserted between the signal sequence and the N-terminus as described in US patent 7,553,931.

*Expression and purification of rhC3-deri vative H5.* Expression of rhC3-derivative H5 is performed in CHO cells and confirmed by ELISA via Strep-Tactin, immunoblot analysis, and densitometry. All methods provided yields of 1-2 mg/l (US patent 7,553,931). For purification, supernatant (500 ml) obtained from stably transfected cells is adjusted to pH 7.5, passed through a 0.45 μm cellulose acetate membrane and loaded onto a Poros HQ/M anion exchange column equilibrated with 50 mM Tris, pH 7.5 using ÄKTA purifier (Amersham Bioscience, Freiburg, Germany). The recombinant protein is eluted using a linear (0-500 mM) NaCl gradient. Fractions (2 ml) are analyzed using 7.5% SDS-PAGE and western blotting, pooled and dialyzed against phosphate buffered saline (PBS). The pooled sample is diluted (1:9) in 50 mM sodium phosphate, 0.55 M sodium sulfate buffer, pH 7.0, filtered (0.2 μm) and applied to a thiophilic resin (1.5 ml, BD Bioscience, Heidelberg, Germany) equilibrated with 50 mM sodium phosphate, 0.5 M sodium sulfate buffer, pH 7.0. After extensive washing of non-adsorbed proteins with the equilibration buffer (> 30 column volumes), elution is performed using 50 mM sodium phosphate buffer, pH 7.0. Fractions (1.5 ml) are analyzed by 7.5% SDS-PAGE and western blotting. Fractions containing H5 are pooled, dialyzed against 100 mM Tris, 150 mM NaCl, pH 8.0 (buffer W), loaded onto Strep-Tactin sepharose (2 ml, IBA, Göttingen, Germany) equilibrated with buffer W, washed with 10 ml buffer W, and eluted with buffer W containing 2.5 mM desthiobiotin. Protein concentration and purity of the fractions are analyzed by 7.5% SDS-PAGE. Pooled fractions are dialyzed against PBS and employed for further characterization.

*4.2.2. In vitro release of rhC3-derivative H5 from PLGA-PEG-PLGA composits*

This experiment is performed as described in patent application EP 12075059.1. The PLGA-PEG-PLGA triblock copolymer of Example 2.1 is dissolved at room temperature in PBS pH 7.4, containing different concentrations of rhC3-derivative H5 (0.2 mg/ml up to 2.0 mg/ml) to make a 20% w/w or 25% w/w solution. Then 200 μl of the formulation is placed in a 2 ml vial, incubated at 37°C for 2 min until gelling, and 1.8 ml of PBS pH 7.4 is added. The vial is incubated at 37°C. At specified sample collection times, a sample is withdrawn and replaced by an identical volume of PBS pH 7.4 to maintain release conditions.

The amount of released rhC3-derivative H5 is determined by bicinchoninc acid (BCA) assay using BCA™ Protein Assay Kit (Pierce, USA). The structural and functional integrity of released rhC3-derivative H5 is determined by a complement consumption assay.

*Complement consumption assay.* First, quantification of human serum is performed by serum titration to achieve hemolysis of sheep erythrocytes by 70-90%. For this purpose, different serum concentrations (serum value) in 2 ml reaction tubes are filled up to 40 μl with $GVBS^{++}$. Additionally, controls are prepared, which contain 40 μl $GVBS^{++}$ (buffer control) only, or 40 μl $H_2O$ (complete lysis) only. All reaction mixtures are incubated for 30 min at 37°C under agitation (Thermomixer 5437, Eppendorf, Hamburg, Germany). Then, 100 μl cold $GVBS^{++}$ or 100 μl $H_2O$ (complete lysis) and 30 μl sensitized sheep erythrocytes are added and the reaction mixtures are incubated for 30 min as described above. Subsequently, the samples are kept on ice and 850 μl cold $VBS^{++}$ or 850 μl $H_2O$ (complete lysis) are added. The supernatants are transferred into cuvettes, and optical density is measured at 412 nm. Hemolysis is calculated according to the following formula:

$$\%\ \text{hemolysis} = \frac{OD_{412}\ \text{serum value} - OD_{412}\ \text{buffer control}}{OD_{412}\ \text{complete lysis} - OD_{412}\ \text{buffer control}} \times 100\%$$

For the complement consumption assay, the quantity of serum determined in prior tests and the samples (max.

20 μl) are supplemented with GVBS$^{++}$ to give 40 μl. Additionally, reaction mixtures of the following controls are prepared: determined quantity of serum, supplemented with GVBS$^{++}$ to give 40 μl (serum control, 4 to 5 samples); 40 μl GVBS$^{++}$ (buffer control) and 40 μl $H_2O$ (complete lysis). All reaction mixtures are incubated for 3 hours at 37°C under agitation. After the addition of 100 μl cold GVBS$^{++}$ or 100 μl $H_2O$ (complete lysis) and 30 μl adjusted sensitized sheep erythrocytes, the reaction mixtures are incubated for 30-40 min as described above. After 15 min, the serum control as well as the complete lysis control are measured according to the following principle. The samples are kept on ice, 850 μl cold VBS$^{++}$ or 850 μl $H_2O$ for complete lysis are added and centrifuged (4°C, 2,000 x g, 2 min.). Supernatants are transferred into cuvettes and the optical density is measured at 412 nm. Lysis is calculated according to the following formla:

$$\%\ \text{hemolysis} = \frac{OD_{412}\ \text{serum control} - OD_{412}\ \text{buffer control}}{OD_{412}\ \text{complete lysis} - OD_{412}\ \text{buffer control}} \times 100\%$$

In case the value for the serum control is clearly below 80% of the complete lysis value, a second serum control is taken after 10 further minutes of incubation and measured as described above. Once a value of 70-80 % hemolysis is obtained, all reaction mixes are measured and evaluated according to the same principle. In order to facilitate comparison of different series of measurements, values are referred to the corresponding serum control.

*4.2.3. Combined in vitro release of TNFR1-ODNs and the rhC3-derivative H5 from PLGA-PEG-PLGA composits*

After complexation of TNFR1-ODNs with Cellfectin as described in Example 3.5., the PLGA-PEG-PLGA triblock copolymer of Example 2.1 is dissolved at room temperature in phosphate buffered saline (PBS) at pH 7.4, containing 25% or 20% w/v polymer. Varying volumes (10 μl, 20 μl, 40 μl, 80 μl) of a 1:1 mixture of TNFR1-ODN/Cellfectin complexes and rhC3-derivative H5 (2.0 mg/ml) in PBS, pH 7.4, are added to 200 μl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

Analysis of released rhC3-derivative H5 and TNFR1-ODNs is performed as described in Example 4.2.2. and in Example 3.5., respectively.

**EXAMPLE 4.3. In vitro release of liposome-embedded TNFR1-specific antisense ODNs, IL-4 antagonist QY, and rhC3-derivative H5 from PLGA-PEG-PLGA composits**

After complexation of TNFR1-ODNs with Cellfectin as described in Example 3.5., the PLGA-PEG-PLGA triblock copolymer of Example 2.1 is dissolved at room temperature in water, containing 30% w/v polymer. 20 μl 10xPBS and 13 ml of a 1:1:1 mixture of TNFR1-ODN/Cellfectin complexes, the murine IL-4 antagonist QY (2.0 mg/ml in PBS, pH 7.4), and the rhC3-derivative H5 (2.0 mg/ml in PBS, pH 7.4), are added to 200 μl gel solution. Then the formulation is placed in a 2 ml vial, incubated at 37°C for 2 min until gelling, and 1.8 ml of PBS pH 7.4 is added. The vial is incubated at 37°C. At specified sample collection times, the supernatant is withdrawn and replaced by an identical volume of PBS pH 7.4 to maintain release conditions.

Analysis of released murine IL-4 antagonist QY, rhC3-derivative H5, and TNFR1-ODNs is performed as described in Example 4.1.2., Example 4.2.2., and in Example 3.5., respectively.

**EXAMPLE 5. OVALBUMIN-INDUCED ALLERGY IN MICE: IMMUNE RESPONSE AFTER TREATMENT WITH TNFR1 INHIBITORS AND OTHER TOLERANCE-INDUCING AGENTS**

TNFR1-specific inhibitors applied in this example include TNFR1-specific antisense oligo-deoxynucleotides and inhibitors of TNFR1-mediated effects such as N-acetyl-L-cysteine, S-methylglutathione, and sodium salicylate. Other tolerance-inducing agents include IL-4/IL-13 antagonists (e.g., IL-4 mutants), and complement component C3-depleting agents (e.g., rhC3-derivative H5).

**EXAMPLE 5.1. Humoral immune response in OVA-sensitized mice after oral administration of N-acetyl-L-cysteine**

This example shows the effect of oral administration of N-acetyl-L-cysteine (NAC) upon sensitization with alum-adsorbed ovalbumin (OVA) on IgE and IgG1 antibody responses in mice.

*Animals and materials.* Female C57BL/6 mice between 6 and 8 weeks of age are purchased from Charles River (Sulzfeld, Germany). The animals are kept under specific pathogen-free conditions and maintained on OVA-free diets. Ovalbumin (OVA) Grade V and NAC is purchased from Sigma, Imject Alum from Pierce/KMF, rat monoclonal antibodies against murine IgE and IgGl, and goat antisera against murine IgG2a, IgG2b, IgG3 and IGM from BD Pharmigen.

*Preparation of alum-adsorbed ovalbumin (OVA).* OVA (100 μg) is solved in 0.3 ml of PBS, pH 7.4, and mixed with 0.70 ml Imject Alum.

*Treatment procedure.* Mice are injected i.p. 100 μl alum-adsorbed OVA (10 μg OVA in 100 μl of PBS/Imject Alum) and then treated with drinking water containing 1 g N-acetyl-L-cysteine/liter (6.1 mM) adjusted to pH 7.5 and changed every 2 days. On the basis of water consume per day, the quantity o NAC swallowed by a mouse per day is approximately 50 mg NAC/kg. Eight days after OVA-sensitization, serum levels of OVA-specific antibodies are determined by ELISA.

*Analysis of serum levels of OVA-specific antibodies.* Murine anti-OVA IgE, IgG subclasses (IgGl, IgG2a, IgG2b, IgG3) and IgM are determined by ELISA. Plates are coated with 10 μg OVA in 100 μl 0.1 M $NaHCO_3$ for 6 h at 37°C, followed by blocking with 200 μl 3% BSA in PBS, pH 7.4, for 2 h at 37°C. After washing, 100 μl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgG1, IgG2a, IgG2b, IgG3, IgM). Analysis is performed at 405 nm in a microplate autoreader.

Treating mice for 8 days with drinking water containing 1 g NAC/liter after i.p. immunization with alum-adsorbed OVA, both OVA-specific IgE and IgG1 (the murine isotype equivalent to human IgG4) responses were found to be reduced by 68% and 78%, respectively, compared with untreated mice. The synthesis of other Ig isotypes was not significantly affected (Jeannin et al., 1995).

**EXAMPLE 5.2. Humoral immune response in OVA-sensitized mice after injection of hydrogel-embedded TNFR1-specific antisense ODNs**

This example shows the effect of subcutaneously injected polymer-embedded TNFR1-specific antisense oligodeoxynucleotides (TNFR1-ODNs) followed by the injection of alum-adsorbed ovalbumin (OVA) at the site of the gelled polymer composit, on the humoral immune response and development of allergic symptoms in mice.

For this experiment, antisense oligodeoxynucleotides (ODNs) are used which target the 3'-polyadenylation [poly(A)] signal loci on the TNFR1 mRNA as described by Ojwang et al. (1997a) and Ojwang et al. (1997b). To enhance cellular uptake of the antisense ODNs and their subsequent release from endosomal compartments, the TNFR1-specific antisense ODNs are formulated with a cationic liposomal preparation (Cellfectin; Life Technologies, USA) containing the cationic lipid tetramethyltetrapalmitylspermine and the phospholipid dioleoylphosphatidylethanolamine

*Animals and materials.* Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgGl, and goat antisera against murine IgG2a, IgG2b, and IgG3 from BD Pharmigen.

*Preparation of alum-adsorbed ovalbumin (OVA).* OVA (100 μg) is solved in 0.3 ml of PBS, pH 7.4, and mixed with 0.70 ml Imject Alum.

*Synthesis of a pPT TNFR1-specific antisense ODN.* The TNFR1-specific antisense ODN is synthesized using partial phosphorothioate linkages (pPT) to confer nuclease resistance and to reduce nonspecific side effects associated with ODNs having a sulfur moiety at every internucleoside linkage. The Beaucage reagent (Iyer et al., 1990) is used for the synthesis of the TNFR1-specific antisense pPT ODN.

*Sequence of the 22mer pPT TNFR1-specific antisense ODN* a* g* a* a* t t t t t a* g* t* g* t* a t g t a* c* a* a (asterics indicate phosphorothioate linkages)

Purification by anion exchange chromatography, desalting and analysis for purity is performed as described (Ojwang and Rando, 1999). Using the purified 22mer pPT antisense ODN, melting temperature evaluations revealed a Tm of 36.6°C for a DNA:DNA duplex and a Tm of 31.1°C for a DNA:RNA duplex (Ojwang and Rando, 1999).

*Formulation of the 22mer pPT TNFR1-specific antisense ODN with Cellfectin.* TNFR1-specific pPT antisense ODN (1.0 μM) in 25 μl PBS, 7.4, are mixed with 2.5 μl of Cellfectin II in PBS, pH 7.4 (1 mg/ml) and incubated for 15 min incubation at room temperature.

*Treatment procedure.* Mice are injected subcutaneously 210 μl PBS, pH 7.4, containing 140 μl of the PLGA-PEG-PLGA triblock copolymer of Example 2.1. (30% w/v polymer) and 70 μl of TNFR1-ODN/Cellfectin complexes. A control group receives the copolymer without embedded TNFR1-ODN. Before immunization with OVA, analyses of OVA-specific antibodies, cytokine levels in serum and FOXP3 and GATA3 mRNA expression are performed. Six hours later, 100 μl alum-adsorbed OVA (10 μl OVA in 100 μl of PBS/Imject Alum) are injected subcutaneously at the site of the gelled polymer composit (50 μl at each side of the gelled polymer). At day 3 and day 6 after immunization with OVA, one group of mice (group 4) are injected again 210 μl of the PLGA-PEG-PLGA triblock copolymer containing the TNFR1-ODN/Cellfectin complexes. At day 9 after immunization with OVA, serum levels of OVA-specific antibodies are analyzed.

Furthermore, immediate hypersensitivity in skin responses upon challenge via intradermal injection of OVA (3 mice of each group) and the development of anaphylactic shock upon i.v. injection of OVA (3 mice of each group) is assessed. Inguinal lymphnodes are used for a detailed analysis of T cell phenotypes.

Four groups of wild-type BALB/c mice (each group: 6 mice) are analyzed: group 1 (control 1): non-loaded polymer, mock immunization; group 2 (control 2): non-loaded polymer, immunization with alum-OVA; group 3: one injection of loaded polymer, immunization with alum-OVA; group 4: three injections of loaded polymer, immunization with alum-OVA.

*Analysis of serum levels of OVA-specific antibodies.* Murine anti-OVA IgE, IgG subclasses (IgGl, IgG2a, IgG2b, IgG3) and IgM are determined by ELISA. Plates are coated with 10 μg OVA in 100 μl 0.1 M $NaHCO_3$ for 6 h at 37°C, followed by blocking with 200 μl 3% BSA in PBS, pH 7.4, for 2 h at 37°C. After washing, 100 μl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgGl, IgG2a, IgG2b, IgG3, IgM). Analysis is performed at 405 nm in a microplate autoreader.

*Analysis of serum levels of cytokines.*

The cytokine supernatant is profiled using a panel of 27 cytokines including the Th2 cytokines IL-4, IL-5 and IL-13. *Analysis of FOXP3 and GATA3 mRNA expression.* The read-out for T cell differentiation is FOXP3 and GATA3 mRNA expression, which are inversely regulated. In addition the release is followed in realtime using STAT6 responsive Luciferase systems reporter systems.

*Analysis of immediate cutaneous hypersensitivity.* Active cutaneous anaphylaxis is tested by skin test after i.v. injection of 200 μl of 0.5% Evans Blue dye in PBS, pH 7.4. Thereafter, the skin of the belly is shaved and four injection sites are marked with a felt tip pen on the skin. Two of the marked sites are injected intradermally with 50 μl PBS, pH 7.4, containing 50 μg OVA, and the other two sites with protein-free PBS, pH 7.4. After 15 min, the mice were killed by cervical dislocation and the skin is stripped off for inspection of the injection sites. The intensity of blue patch formation on the dorsal side of the skin, resulting from fluid extravasation into the injection site upon mast cell degranulation, is scored by two independent observers. Reactions are rated as positive when the diameter of the blue patch exceeds 5 mm, which is pre-marked on the mouse skin. The intensity of bluing is rated in the following manner: 0 = no blue patch formation; 1 = slight bluing; 2 = marked bluing; 3 = strong bluing.

*Analysis of anaphylactic shock symptoms.* Mice are injected i.v. 500 μl OVA in 200 μl 0.5% Evans Blue solution. After 15 min, symptoms of an anaphylactic shock are assessed including bluing (no = 0; slight = 1; strong = 2), pilo erection (no = 0; slight = 1; strong = 2), spontaneous activity (running around = 0; sitting passively = 1; lying = 2), and responsiveness to external stimuli (running away upon touching = 0; slight reaction = 1; no reaction = 2). The animals are considered to be in a state of shock if at least three of the four indicated symptoms are observed by two independent observers who are unaware of the sensitization status of each animal.

*Analysis of T cell phenotype in inguinal lymphnodes.* Right and left inguinal lymph nodes are removed and T cell phenotype is tested by *in vitro* re-stimulation with allergen (ELISPOT/Luminex).

**EXAMPLE 5.3. Humoral immune response in OVA-sensitized mice after injection of hydrogel-embedded S-methylglutathione (GSM)**

This example shows the effect of subcutaneously injected polymer-embedded S-methylglutathione (GSM) followed by the injection of alum-adsorbed ovalbumin (OVA) at the site of the gelled polymer composit, on the humoral immune response and development of allergic symptoms in mice.

*Animals and materials.* Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgGl, and goat antisera against murine IgG2a, IgG2b, and IgG3 from BD Pharmigen.

*Preparation of alum-adsorbed ovalbumin* (*OVA*). OVA (100 μg) is solved in 0.3 ml of PBS, pH 7.4, and mixed with 0.70 ml Imject Alum.

*Treatment procedure.* Mice are injected subcutaneously 210 μl PBS, pH 7.4, containing 140 μl of the PLGA-PEG-PLGA triblock copolymer of Example 2.1. (25% w/v polymer) and 70 μl of 5 mM S-methylglutathione (GSM). A control group receives the copolymer without embedded GSM. Before immunization with OVA, analyses of OVA-specific antibodies, cytokine levels in serum and FOXP3 and GATA3 mRNA expression are performed. Six hours later, 100 μl alum-adsorbed OVA (10 μl OVA in 100 μl of PBS/Imject Alum) are injected subcutaneously at the site of the gelled polymer composit (50 μl at each side of the gelled polymer). At day 3 and day 6 after immunization with OVA, one group of mice (group 4) are injected again 210 μl of the PLGA-PEG-PLGA triblock copolymer containing GSM. At day 9 after immunization with OVA, serum levels of OVA-specific antibodies are analyzed. Furthermore, immediate hypersensitivity in skin responses upon challenge via intradermal injection of OVA (3 mice of each group) and the development of anaphylactic shock upon i.v. injection of OVA (3 mice of each group) is assessed. Inguinal lymphnodes are used for a detailed analysis of T cell phenotypes.

Four groups of wild-type BALB/c mice (each group: 6 mice) are analyzed: group 1 (control 1): non-loaded polymer, mock immunization; group 2 (control 2): non-loaded polymer, immunization with alum-OVA; group 3: one injection of loaded polymer, immunization with alum-OVA; group 4: three injections of loaded polymer, immunization with alum-OVA.

Analysis of serum levels of OVA-specific antibodies, serum levels of cytokines, FOXP3 and GATA3 mRNA expression, immediate cutaneous hypersensitivity, anaphylactic shock symptoms, and T cell phenotypes in inguinal lymphnodes are performed as described in Example 5.2.

**EXAMPLE 5.4. Humoral immune response in OVA-sensitized mice after injection of hydrogel-embedded sodium salicylate (SA)**

This example shows the effect of subcutaneously injected polymer-embedded sodium salicylate (SA) followed by the injection of alum-adsorbed ovalbumin (OVA) at the site of the gelled polymer composit, on the humoral immune response and development of allergic symptoms in mice.

*Animals and materials.* Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgG1, and goat antisera against murine IgG2a, IgG2b, and IgG3 from BD Pharmigen.

*Preparation of alum-adsorbed ovalbumin* (*OVA*). OVA (100 μg) is solved in 0.3 ml of PBS, pH 7.4, and mixed with 0.70 ml Imject Alum.

*Treatment procedure.* Mice are injected subcutaneously 210 μl PBS, pH 7.4, containing 140 μl of the PLGA-PEG-PLGA triblock copolymer of Example 2.1. (30% w/v polymer) and 70 μl of 10 mM sodium salicylate (SA). A control group receives the copolymer without embedded GSM. Before immunization with OVA, analyses of OVA-specific antibodies, cytokine levels in serum and FOXP3 and GATA3 mRNA expression are performed. Six hours later, 100 μl alum-adsorbed OVA (10 μl OVA in 100 μl of PBS/Imject Alum) are injected subcutaneously at the site of the gelled polymer composit (50 μl at each side of the gelled polymer). At day 3 and day 6 after immunization with OVA, one group of mice (group 4) are injected again 210 μl of the PLGA-PEG-PLGA triblock copolymer containing SA. At day 9 after immunization with OVA, serum levels of OVA-specific antibodies are analyzed. Furthermore, immediate hypersensitivity in skin responses upon challenge via intradermal injection of OVA (3 mice of each group) and the development of anaphylactic shock upon i.v. injection of OVA (3 mice of each group) is assessed. Inguinal lymphnodes are used for a detailed analysis of T cell phenotypes.

Four groups of wild-type BALB/c mice (each group: 6 mice) are analyzed: group 1 (control 1): non-loaded polymer, mock immunization; group 2 (control 2): non-loaded polymer, immunization with alum-OVA; group 3: one injection of loaded polymer, immunization with alum-OVA; group 4: three injections of loaded polymer, immunization with alum-OVA.

Analysis of serum levels of OVA-specific antibodies, serum levels of cytokines, FOXP3 and GATA3 mRNA expression, immediate cutaneous hypersensitivity, anaphylactic shock symptoms, and T cell phenotypes in inguinal lymphnodes are performed as described in Example 5.2.

**EXAMPLE 5.5. Humoral immune response in OVA-sensitized mice after injection of hydrogel-embedded TNFR1-ODNs, GSM, and SA**

This example shows the effect of subcutaneously injected polymer-embedded TNFR1-specific antisense oligodeoxynucleotides (TNFR1-ODNs), S-methylglutathione (GSM) and sodium salicylate (SA) followed by the injection of alum-adsorbed ovalbumin (OVA) at the site of the gelled polymer composit, on the humoral immune response and development of allergic symptoms in mice.

*Animals and materials.* Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgGl, and goat antisera against murine IgG2a, IgG2b, and IgG3 from BD Pharmigen.

*Preparation of alum-adsorbed ovalbumin (OVA).* OVA (100 μg) is solved in 0.3 ml of PBS, pH 7.4, and mixed with 0.70 ml Imject Alum.

*Synthesis of a pPT TNFR1-specific antisense ODN.* The TNFR1-specific antisense ODN is synthesized using partial phosphorothioate linkages (pPT) to confer nuclease resistance and to reduce nonspecific side effects associated with ODNs having a sulfur moiety at every internucleoside linkage. The Beaucage reagent (Iyer et al., 1990) is used for the synthesis of the TNFR1-specific antisense pPT ODN.
*Sequence of the 22mer pPT TNFR1-specific antisense ODN* a* g* a* a* t t t t t a* g* t* g* t* a t g t a* c* a* a (asterics indicate phosphorothioate linkages)

Purification by anion exchange chromatography, desalting and analysis for purity is performed as described (Ojwang and Rando, 1999). Using the purified 22mer pPT antisense ODN, melting temperature evaluations revealed a Tm of 36.6°C for a DNA:DNA duplex and a Tm of 31.1°C for a DNA:RNA duplex (Ojwang and Rando, 1999).

*Formulation of the 22mer pPT TNFR1-specific antisense ODN with Cellfectin.* TNFR1-specific pPT antisense ODN (1.0 μM) in 25 μl PBS, 7.4, are mixed with 2.5 μl of Cellfectin in PBS, pH 7.4 (1 mg/ml) and incubated for 15 min incubation at room temperature.

*Treatment procedure.* Mice are injected subcutaneously 225 μl PBS, pH 7.4, containing 150 μl of the PLGA-PEG-PLGA triblock copolymer of Example 2.1. (30% w/v polymer), 25 μl of TNFR1-ODN/Cellfectin complexes, 25 μl of 5 mM S-methylglutathione (GSM), and 25 μl of 10 mM sodium salicylate (SA). A control group receives the copolymer without embedded TNFR1-ODN, GSM, and SA. Before immunization with OVA, analyses of OVA-specific antibodies, cytokine levels in serum and FOXP3 and GATA3 mRNA expression are performed. Six hours later, 100 μl alum-adsorbed OVA (10 μl OVA in 100 μl of PBS/Imject Alum) are injected subcutaneously at the site of the gelled polymer composit (50 μl at each side of the gelled polymer). At day 3 and day 6 after immunization with OVA, one group of mice (group 4) are injected again 225 μl of the PLGA-PEG-PLGA triblock copolymer containing TNFR1-ODN, GSM, and SA. At day 9 after immunization with OVA, serum levels of OVA-specific antibodies are analyzed. Furthermore, immediate hypersensitivity in skin responses upon challenge via intradermal injection of OVA (3 mice of each group) and the development of anaphylactic shock upon i.v. injection of OVA (3 mice of each group) is assessed. Inguinal lymphnodes are used for a detailed analysis of T cell phenotypes.

Four groups of wild-type BALB/c mice (each group: 6 mice) are analyzed: group 1 (control 1): non-loaded polymer, mock immunization; group 2 (control 2): non-loaded polymer, immunization with alum-OVA; group 3: one injection of loaded polymer, immunization with alum-OVA; group 4: three injections of loaded polymer, immunization with alum-OVA.

Analysis of serum levels of OVA-specific antibodies, serum levels of cytokines, FOXP3 and GATA3 mRNA expression, immediate cutaneous hypersensitivity, anaphylactic shock symptoms, and T cell phenotypes in inguinal lymphnodes are performed as described in Example 5.2.

**EXAMPLE 5.6. Humoral immune response in OVA-sensitized mice after injection of hydrogel-embedded TNFR1-ODNs, and IL-4 antagonist QY**

This example shows the effect of subcutaneously injected polymer-embedded TNFR1-specific antisense oligodeoxynucleotides (TNFR1-ODNs) and polymer-embedded murine IL-4 mutant QY followed by the injection of alum-adsorbed ovalbumin (OVA) at the site of the gelled polymer composit, on the humoral immune response and development of allergic symptoms in mice.

*Animals and materials.* Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgGl, and goat antisera against murine IgG2a, IgG2b, and IgG3 from BD Pharmigen.

*Preparation of alum-adsorbed ovalbumin (OVA).* OVA (100 μg) is solved in 0.3 ml of PBS, pH 7.4, and mixed with 0.70 ml Imject Alum.

*Synthesis of* a *pPT TNFR1-specific antisense ODN.* The TNFR1-specific antisense ODN is synthesized using partial phosphorothioate linkages (pPT) to confer nuclease resistance and to reduce nonspecific side effects associated with ODNs having a sulfur moiety at every internucleoside linkage. The Beaucage reagent (Iyer et al., 1990) is used for the synthesis of the TNFR1-specific antisense pPT ODN.
*Sequence of the 22mer pPT TNFR1-specific antisense ODN* a* g* a* a* t t t t t a* g* t* g* t* a t g t a* c* a* a (asterics indicate phosphorothioate linkages)

Purification by anion exchange chromatography, desalting and analysis for purity is performed as described (Ojwang and Rando, 1999). Using the purified 22mer pPT antisense ODN, melting temperature evaluations revealed a Tm of 36.6°C for a DNA:DNA duplex and a Tm of 31.1°C for a DNA:RNA duplex (Ojwang and Rando, 1999).

*Formulation of the 22mer pPT TNFR1-specific antisense ODN with Cellfectin.* TNFR1-specific pPT antisense ODN (1.0 μM) in 35 μl PBS, 7.4, are mixed with 3.5 μl of Cellfectin in PBS, pH 7.4 (1 mg/ml) and incubated for 15 min incubation at room temperature.

*Treatment procedure.* Mice are injected subcutaneously 210 μl PBS, pH 7.4, containing 140 μl of the PLGA-PEG-PLGA triblock copolymer of Example 2.1. (30% w/v polymer), 35 μl of TNFR1-ODN/Cellfectin complexes, and 35 μl of murine IL-4/IL-13 antagonist QY (2.0 mg/ml; see Example 4.1). A control group receives the copolymer without embedded TNFR1-ODN and murine IL-4/IL-13 antagonist QY. Before immunization with OVA, analyses of OVA-specific antibodies, cytokine levels in serum and FOXP3 and GATA3 mRNA expression are performed. Six hours later, 100 μl alum-adsorbed OVA (10 μg OVA in 100 μl of PBS/Imject Alum) are injected subcutaneously at the site of the gelled polymer composit (50 μl at each side of the gelled polymer). At day 3 and day 6 after immunization with OVA, one group of mice (group 4) are injected again 210 μl of the PLGA-PEG-PLGA triblock copolymer containing TNFR1-ODN/Cellfectin complexes and the murine IL-4/IL-13 antagonist QY. At day 9 after immunization with OVA, serum levels of OVA-specific antibodies are analyzed. Furthermore, immediate hypersensitivity in skin responses upon challenge via intradermal injection of OVA (3 mice of each group) and the development of anaphylactic shock upon i.v. injection of OVA (3 mice of each group) is assessed. Inguinal lymphnodes are used for a detailed analysis of T cell phenotypes.

Four groups of wild-type BALB/c mice (each group: 6 mice) are analyzed: group 1 (control 1): non-loaded polymer, mock immunization; group 2 (control 2): non-loaded polymer, immunization with alum-OVA; group 3: one injection of loaded polymer, immunization with alum-OVA; group 4: three injections of loaded polymer, immunization with alum-OVA.

Analysis of serum levels of OVA-specific antibodies, serum levels of cytokines, FOXP3 and GATA3 mRNA expression, immediate cutaneous hypersensitivity, anaphylactic shock symptoms, and T cell phenotypes in inguinal lymphnodes are performed as described in Example 5.2.

**EXAMPLE 5.7. Humoral immune response in OVA-sensitized mice after injection of hydrogel-embedded TNFR1-ODNs, and rhC3-derivative H5**

This example shows the effect of subcutaneously injected polymer-embedded TNFR1-specific antisense oligodeoxynucleotides (TNFR1-ODNs) and polymer-embedded rhC3-derivative H5 followed by the injection of alum-adsorbed ovalbumin (OVA) at the site of the gelled polymer composit, on the humoral immune response and development of allergic symptoms in mice.

*Animals and materials.* Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgGl, and goat antisera against murine IgG2a, IgG2b, and IgG3 from BD Pharmigen.

*Preparation of alum-adsorbed ovalbumin* (*OVA*). OVA (100 μg) is solved in 0.3 ml of PBS, pH 7.4, and mixed with 0.70 ml Imject Alum.

*Synthesis of* a *pPT TNFR1-specific antisense ODN.* The TNFR1-specific antisense ODN is synthesized using partial phosphorothioate linkages (pPT) to confer nuclease resistance and to reduce nonspecific side effects associated with ODNs having a sulfur moiety at every internucleoside linkage. The Beaucage reagent (Iyer et al., 1990) is used for the synthesis of the TNFR1-specific antisense pPT ODN.

*Sequence of the 22mer pPT TNFR1-specific antisense ODN* a* g* a* a* t t t t a* g* t* g* t* a t g t a* c* a* a (asterics indicate phosphorothioate linkages)

Purification by anion exchange chromatography, desalting and analysis for purity is performed as described (Ojwang and Rando, 1999). Using the purified 22mer pPT antisense ODN, melting temperature evaluations revealed a Tm of 36.6°C for a DNA:DNA duplex and a Tm of 31.1°C for a DNA:RNA duplex (Ojwang and Rando, 1999).

*Formulation of the 22mer pPT TNFR1-specific antisense ODN with* Cellfectin. TNFR1-specific pPT antisense ODN (1.0 μM) in 35 μl PBS, 7.4, are mixed with 3.5 μl of Cellfectin in PBS, pH 7.4 (1 mg/ml) and incubated for 15 min incubation at room temperature.

*Treatment procedure.* Mice are injected subcutaneously 210 μl PBS, pH 7.4, containing 140 μl of the PLGA-PEG-PLGA triblock copolymer of Example 2.1. (30% w/v polymer), 35 μl of TNFR1-ODN/Cellfectin complexes, and 35 μl of rhC3-derivative H5 (2.0 mg/ml; see Example 4.2.). A control group receives the copolymer without embedded TNFR1-ODN and murine IL-4/IL-13 antagonist QY. Before immunization with OVA, analyses of OVA-specific antibodies, cytokine levels in serum and FOXP3 and GATA3 mRNA expression are performed. Six hours later, 100 μl alum-adsorbed OVA (10 μg OVA in 100 μl of PBS/Imject Alum) are injected subcutaneously at the site of the gelled polymer composit (50 μl at each side of the gelled polymer). At day 3 and day 6 after immunization with OVA, one group of mice (group 4) are injected again 210 μl of the PLGA-PEG-PLGA triblock copolymer containing TNFR1-ODN/Cellfectin complexes and the rhC3-derivative H5. At day 9 after immunization with OVA, serum levels of OVA-specific antibodies are analyzed. Furthermore, immediate hypersensitivity in skin responses upon challenge via intradermal injection of OVA (3 mice of each group) and the development of anaphylactic shock upon i.v. injection of OVA (3 mice of each group) is assessed. Inguinal lymphnodes are used for a detailed analysis of T cell phenotypes.

Four groups of wild-type BALB/c mice (each group: 6 mice) are analyzed: group 1 (control 1): non-loaded polymer, mock immunization; group 2 (control 2): non-loaded polymer, immunization with alum-OVA; group 3: one injection of loaded polymer, immunization with alum-OVA; group 4: three injections of loaded polymer, immunization with alum-OVA.

Analysis of serum levels of OVA-specific antibodies, serum levels of cytokines, FOXP3 and GATA3 mRNA expression, immediate cutaneous hypersensitivity, anaphylactic shock symptoms, and T cell phenotypes in inguinal lymphnodes are performed as described in Example 5.2.

**EXAMPLE 5.8. Humoral immune response in OVA-sensitized mice after injection of hydrogel-embedded TNFR1-ODNs, IL-4 antagonist QY, and rhC3-derivative H5**

This example shows the effect of subcutaneously injected polymer-embedded TNFR1-specific antisense oligodeoxynucleotides (TNFR1-ODNs), polymer-embedded murine IL-4 mutant QY and polymer-embedded rhC3-derivative H5 followed by the injection of alum-adsorbed ovalbumin (OVA) at the site of the gelled polymer composit, on the humoral immune response and development of allergic symptoms in mice.

*Animals and materials.* Female wild-type BALB/c mice between 8 and 12 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgGl, and goat antisera against murine IgG2a, IgG2b, and IgG3 from BD Pharmigen.

*Preparation of alum-adsorbed ovalbumin (OVA).* OVA (100 μg) is solved in 0.3 ml of PBS, pH 7.4, and mixed with 0.70 ml Imject Alum.

*Synthesis of a pPT TNFR1-specific antisense ODN.* The TNFR1-specific antisense ODN is synthesized using partial phosphorothioate linkages (pPT) to confer nuclease resistance and to reduce nonspecific side effects associated with ODNs having a sulfur moiety at every internucleoside linkage. The Beaucage reagent (Iyer et al., 1990) is used for the synthesis of the TNFR1-specific antisense pPT ODN.

*Sequence of the 22mer pPT TNFR1-specific antisense ODN* a* g* a* a* t t t t t a* g* t* g* t* a t g t a* c* a* a (asterics indicate phosphorothioate linkages)

Purification by anion exchange chromatography, desalting and analysis for purity is performed as described (Ojwang and Rando, 1999). Using the purified 22mer pPT antisense ODN, melting temperature evaluations revealed a Tm of 36.6°C for a DNA:DNA duplex and a Tm of 31.1°C for a DNA:RNA duplex (Ojwang and Rando, 1999).

*Formulation of the 22mer pPT TNFR1-specific antisense ODN with Cellfectin.* TNFR1-specific pPT antisense ODN (1.0 μM) in 25 μl PBS, 7.4, are mixed with 2.5 μl of Cellfectin in PBS, pH 7.4 (1 mg/ml) and incubated for 15 min incubation at room temperature.

*Treatment procedure.* Mice are injected subcutaneously 225 μl PBS, pH 7.4, containing 150 μl of the PLGA-PEG-PLGA triblock copolymer of Example 2.1. (30% w/v polymer), 25 μl of TNFR1-ODN/Cellfectin complexes, 25 μl of murine IL-4/IL-13 antagonist QY (2.0 mg/ml; see Example 4.1), and 25 μl of rhC3-derivative H5 (2.0 mg/ml; see Example 4.2.). A control group receives the copolymer without embedded TNFR1-ODN, murine IL-4/IL-13 antagonist QY, andrhC3-derivative H5. Before immunization with OVA, analyses of OVA-specific antibodies, cytokine levels in serum and FOXP3 and GATA3 mRNA expression are performed. Six hours later, 100 μl alum-adsorbed OVA (10 μg OVA in 100 μl of PBS/Imject Alum) are injected subcutaneously at the site of the gelled polymer composit (50 μl at each side of the gelled polymer). At day 3 and day 6 after immunization with OVA, one group of mice (group 4) are injected again 225 μl of the PLGA-PEG-PLGA triblock copolymer containing TNFR1-ODN, murine IL-4/IL-13 antagonist QY, andrhC3-derivative H5. At day 9 after immunization with OVA, serum levels of OVA-specific antibodies are analyzed. Furthermore, immediate hypersensitivity in skin responses upon challenge via intradermal injection of OVA (3 mice of each group) and the development of anaphylactic shock upon i.v. injection of OVA (3 mice of each group) is assessed. Inguinal lymphnodes are used for a detailed analysis of T cell phenotypes.

Four groups of wild-type BALB/c mice (each group: 6 mice) are analyzed: group 1 (control 1): non-loaded polymer, mock immunization; group 2 (control 2): non-loaded polymer, immunization with alum-OVA; group 3: one injection of loaded polymer, immunization with alum-OVA; group 4: three injections of loaded polymer, immunization with alum-OVA.

Analysis of serum levels of OVA-specific antibodies, serum levels of cytokines, FOXP3 and GATA3 mRNA expression, immediate cutaneous hypersensitivity, anaphylactic shock symptoms, and T cell phenotypes in inguinal lymphnodes are performed as described in Example 5.2.

**Literature for example 5**

Iyer et al., J. Org. Chem. 55: 4699-4704; 1990.

Jeannin et al., J. Exp. Med. 182: 1785-1792; 1995.

Ojwang et al., Biochemistry 36: 6033-6045; 1997a.

Ojwang et al., Antisense Nucleic Acid Drug Dev. 7: 447-459; 1997b.

Ojwang and Rando, Methods: a companion to Meth Enzymol. 18: 244-251; 1999.

**EXAMPLE 6: IMMUNOTHERAPY OF OVA-ALLERGIC MICE: Immunotherapy with alum-adsorbed OVA and PLGA-PEG-PLGA composits containing TNFR1-ODN, murine IL-4 antagonist QY, and rhC3-derivative H5**

This example shows the efficacy of immunotherapy of OVA-allergic mice with increasing doses of subcutaneously injected alum-adsorbed OVA and co-injected polymer-embedded TNFR1-specific antisense oligonucleotides (TNFR1-ODNs), murine IL-4/IL-13 antagonist QY, and rhC3-derivative H5 at the site of OVA presentation.

*Animals and materials.* Female wild-type C57BL/6 mice 6 to 10 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgGl, and goat antisera against murine IgG2a, IgG2b, and IgG3 from BD Pharmigen. Murine IL-4/IL-13 antagonist QY is prepared as described in Example 4.1, and rhC3-derivative H5 as described in Example 4.2.

*Synthesis of a pPT TNFR1-specific antisense ODN.* The TNFR1-specific antisense ODN is synthesized using partial phosphorothioate linkages (pPT) to confer nuclease resistance and to reduce nonspecific side effects associated with ODNs having a sulfur moiety at every internucleoside linkage. The Beaucage reagent (Iyer et al., 1990) is used for the synthesis of the TNFR1-specific antisense pPT ODN.

*Sequence of the 22mer pPT TNFR1-specific antisense ODN* a* g* a* a* t t t t t a* g* t* g* t* a t g t a* c* a* a (asterics indicate phosphorothioate linkages)

Purification by anion exchange chromatography, desalting and analysis for purity is performed as described (Ojwang and Rando, 1999). Using the purified 22mer pPT antisense ODN, melting temperature evaluations revealed a Tm of 36.6°C for a DNA:DNA duplex and a Tm of 31.1°C for a DNA:RNA duplex (Ojwang and Rando, 1999).

*Formulation of the 22mer pPT TNFR1-specific antisense ODN with Cellfectin.* TNFR1-specific pPT antisense ODN (1.0 μM) in 500 μl PBS, 7.4, are mixed with 50 μl of Cellfectin in PBS, pH 7.4 (1 mg/ml) and incubated for 15 min incubation at room temperature.

*Preparation of alum-adsorbed ovalbumin* (*OVA*). For immunization, 100 μg of OVA are solved in 0.3 ml of PBS, pH 7.4, and mixed with 0.70 ml Imject Alum (Pierce/KMF). For immunotherapy, 10 mg of OVA are solved in 3.0 ml of PBS, pH 7.4, and mixed with 7.0 ml Imject Alum (Pierce/KMF). From this mixture containing 100 μg alum-adsorbed OVA in 0.1 ml, dilutions are prepared containing in 0.1 ml 90 μg, 60 μg, 30 μg, 15 μg, and 7.5 μg alum-adsorbed OVA.

*Allergic sensitization procedure.* Mice are immunized three times by i.p. injection of 100 μl alum-adsorbed OVA (10 μg OVA in 100 μl of PBS/Imject Alum). The second i.p. injection is performed 7 days after the first injection and the third injection 14 days after the first injection.

*Immunotherapy.* One week after the third immunization with OVA, mice are subjected to two different modalities of immunotherapy, one based on conventional treatment with increasing doses of alum-adsorbed OVA, the other based on treatment with increasing doses of alum-adsorbed OVA in the presence of polymer-embedded TNFR1-ODNs, murine IL-4/IL-13 antagonist QY, and rhC3-derivative. Subcutaneous administration of increasing doses of alum-adsorbed OVA is performed at 9-day intervals. Subcutaneous administration of polymer-embedded TNFR1-ODNs, murine IL-4/IL-13 antagonist QY, and rhC3-derivative is performed at day 0, day 3 and day 6 within the 9-day intervals.

For immunotherapy of mice with alum-adsorbed OVA only, 100 μl of PBS/Imject Alum containing increasing doses of OVA (7.5, 15, 30, 60, 90 μg) are injected subcutaneously at 9-day intervals.

For immunotherapy of mice with alum-adsorbed OVA in the presence of polymer-embedded TNFR1-ODNs, murine IL-4/IL-13 antagonist QY, and rhC3-derivative, 225 μl PBS, pH 7.4, containing 150 μl of the PLGA-PEG-PLGA triblock copolymer of Example 2.1. (25% w/v polymer), 25 μl of TNFR1-ODN/Cellfectin complexes, 25 μl of murine IL-4/IL-13 antagonist QY (2.0 mg/ml) and 25 μl of rhC3-derivative H5 (2.0 mg/ml) are injected subcutaneously first. After 60 min, 100 μl of PBS/Imject Alum containing increasing doses of OVA (7.5, 15, 30, 60, 90 μg) are injected subcutaneously at the site of the gelled polymer composit (50 μl at each side of the gelled polymer).

At day 3 and day 6 within each 9-day interval, additional 225 μl PBS, pH 7.4, containing 150 μl of the PLGA-PEG-PLGA triblock copolymer of Example 2.1. (25% w/v polymer), 25 μl of TNFR1-ODN/Cellfectin complexes, 25 μl of murine IL-4/IL-13 antagonist QY (2.0 mg/ml) and 25 μl of rhC3-derivative H5 (2.0 mg/ml) are injected subcutaneously at the site of the first injection. The injection procedure is repeated at a different subcutaneous site until the highest dose of alum-adsorbed OVA is injected.

Three groups of wild-type C57BL/6 mice (each group: 10 mice) are analyzed: group 1: immunization (3 x alum-OVA), therapy with non-loaded polymer and PBS; group 2: immunization (3 x alum-OVA), therapy with loaded polymer and increasing doses of alum-OVA; and group 3: immunization (3 x alum-OVA), therapy with increasing doses of alum-OVA only.

Mice are bled at day 0 before immunization with OVA, at day 7 after immunization with OVA (before starting immunotherapy), and at day 9 after the last immunotherapeutic treatment. Analyses include the determination of serum levels of OVA-specific antibodies, immediate hypersensitivity in skin responses upon challenge via intradermal injection of OVA and the development of anaphylactic shock upon i.v. injection of OVA.

*Analysis of serum levels of OVA-specific antibodies.*

Murine anti-OVA IgE and IgG subclasses are determined by ELISA. Plates are coated with 10 μl OVA in 100 μl 0.1 M $NaHCO_3$ for 6 h at 37°C, followed by blocking with 200 μl 3% BSA in PBS, pH 7.4, for 2 h at 37°C. After washing, 100 μl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgGl, IgG2a, IgG2b, IgG3). Analysis is performed at 405 nm in a microplate autoreader.

*Analysis of serum levels of cytokines.* The cytokine supernatant is profiled using a panel of 27 cytokines including the Th2 cytokines IL-4, IL-5 and IL-13.

*Analysis of FOXP3 and GATA3 mRNA expression.* The read-out for T cell differentiation is FOXP3 and GATA3 mRNA expression, which are inversely regulated. In addition the release is followed in realtime using STAT6 responsive Luciferase systems reporter systems.

*Analysis of immediate cutaneous hypersensitivity.* Active cutaneous anaphylaxis is tested by skin test after i.v. injection of 200 μl of 0.5% Evans Blue dye in PBS, pH 7.4. Thereafter, the skin of the belly is shaved and four injection sites are marked with a felt tip pen on the skin. Two of the marked sites are injected intradermally with 50 μl PBS, pH 7.4, containing 50 μg OVA, and the other two sites with protein-free PBS, pH 7.4. After 15 min, the mice were killed by cervical dislocation and the skin is stripped off for inspection of the injection sites. The intensity of blue patch formation on the dorsal side of the skin, resulting from fluid extravasation into the injection site upon mast cell degranulation, is scored by two independent observers. Reactions are rated as positive when the diameter of the blue patch exceeds 5 mm, which is pre-marked on the mouse skin. The intensity of bluing is rated in the following manner: 0 = no blue patch formation; 1 = slight bluing; 2 = marked bluing; 3 = strong bluing.

*Analysis of anaphylactic shock symptoms.* Mice are injected i.v. 500 μl OVA in 200 μl 0.5% Evans Blue solution. After 15 min, symptoms of an anaphylactic shock are assessed including bluing (no = 0; slight = 1; strong = 2), pilo erection (no = 0; slight = 1; strong = 2), spontaneous activity (running around = 0; sitting passively = 1; lying = 2), and responsiveness to external stimuli (running away upon touching = 0; slight reaction = 1; no reaction = 2). The animals are considered to be in a state of shock if at least three of the four indicated symptoms are observed by two independent observers who are unaware of the sensitization status of each animal.

*Analysis of T cell phenotype in inguinal lymphnodes.* Right and left inguinal lymph nodes are removed and T cell phenotype is tested by *in vitro* re-stimulation with allergen (ELISPOT/Luminex).

**Literature for example 6**

Iyer et al., J. Org. Chem. 55: 4699-4704; 1990.
Ojwang and Rando, Methods: a companion to Meth Enzymol. 18: 244-251; 1999.

**EXAMPLE 7: IMMUNOTHERAPY OF OVA-INDUCED ALLERGIC AIRWAY INFLAMMATION IN MICE:**

**Immunotherapy with alum-adsorbed OVA and PLGA-PEG-PLGA composits containing TNFR1-ODN, murine IL-4 antagonist QY, and rhC3-derivative H5**

This example shows the efficacy of immunotherapy of OVA-sensitized mice exhibiting allergic airway inflammation after inhaled OVA administration, using increasing doses of subcutaneously injected alum-adsorbed OVA and co-injected polymer-embedded TNFR1-specific antisense oligonucleotides (TNFR1-ODNs), murine IL-4/IL-13 antagonist QY, and rhC3-derivative H5 at the site of OVA presentation.

*Animals and materials.* Female wild-type C57BL/6 mice 6 to 10 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, rat monoclonal antibodies against murine IgE and IgGl, and goat antisera against murine IgG2a, IgG2b, and IgG3 from BD Pharmigen. Murine IL-4/IL-13 antagonist QY is prepared as described in Example 4.1, and rhC3-derivative H5 as described in Example 4.2.

*Synthesis of a pPT TNFR1-specific antisense ODN.* The TNFR1-specific antisense ODN is synthesized using partial phosphorothioate linkages (pPT) to confer nuclease resistance and to reduce nonspecific side effects associated with ODNs having a sulfur moiety at every internucleoside linkage. The Beaucage reagent (Iyer et al., 1990) is used for the synthesis of the TNFR1-specific antisense pPT ODN.

*Sequence of the 22mer pPT TNFR1-specific antisense ODN* a* g* a* a* t t t t t a* g* t* g* t* a t g t a* c* a* a (asterics indicate phosphorothioate linkages)

Purification by anion exchange chromatography, desalting and analysis for purity is performed as described (Ojwang and Rando, 1999). Using the purified 22mer pPT antisense ODN, melting temperature evaluations revealed a Tm of 36.6°C for a DNA:DNA duplex and a Tm of 31.1°C for a DNA:RNA duplex (Ojwang and Rando, 1999).

*Formulation of the 22mer pPT TNFR1-specific antisense ODN with Cellfectin.* TNFR1-specific pPT antisense ODN (1.0 μM) in 500 μl PBS, 7.4, are mixed with 50 μl of Cellfectin II in PBS, pH 7.4 (1 mg/ml) and incubated for 15 min incubation at room temperature.

*Preparation of alum-adsorbed ovalbumin (OVA).* For immunization, 100 μg of OVA are solved in 0.3 ml of PBS, pH 7.4, and mixed with 0.70 ml Imject Alum (Pierce/KMF). For immunotherapy, 10 mg of OVA are solved in 3.0 ml of PBS, pH 7.4, and mixed with 7.0 ml Imject Alum (Pierce/KMF). From this mixture containing 100 μg alum-adsorbed OVA in 0.1 ml, dilutions are prepared containing in 0.1 ml 90 μg, 60 μg, 30 μg, 15 μg, and 7.5 μg alum-adsorbed OVA.

*Allergic sensitization procedure.* Mice are immunized three times by i.p. injection of 100 μl alum-adsorbed OVA (10 μg OVA in 100 μl of PBS/Imject Alum). The second i.p. injection is performed 7 days after the first injection and the third injection 14 days after the first injection.

One week after the last injection the mice are exposed in a Plexiglas chamber (approx. 10 x 15 x 25 cm) to 1% (wt/vol) aerosolized OVA in 0.9% saline (using a nebulizer with an airflow rate of 10L/min), 30 min/day for 10 days.

*Immunotherapy.* One week after the final aerosol exposure, mice are subjected to two different modalities of immunotherapy, one based on conventional treatment with increasing doses of alum-adsorbed OVA, the other based on treatment with increasing doses of alum-adsorbed OVA in the presence of polymer-embedded TNFR1-ODNs, murine IL-4/IL-13 antagonist QY, and rhC3-derivative. Subcutaneous administration of increasing doses of alum-adsorbed OVA is performed at 9-day intervals. Subcutaneous administration of polymer-embedded GSM, rhC3-derivative H5 and murine IL-4/IL-13 antagonist QY is performed at day 0, day 3 and day 6 within each 9-day interval.

For immunotherapy of mice with alum-adsorbed OVA only, 100 μl of PBS/Imject Alum containing increasing doses of OVA (7.5, 15, 30, 60, 90 μg alum-adsorbed OVA) are injected subcutaneously at 9-day intervals.

For immunotherapy of mice with alum-adsorbed OVA in the presence of polymer-embedded TNFR1-ODNs, murine IL-4/IL-13 antagonist QY, and rhC3-derivative, 225 μl PBS, pH 7.4, containing 150 μl of the PLGA-PEG-PLGA triblock copolymer of Example 2.1. (30% w/v polymer), 25 μl of TNFR1-ODN/Cellfectin complexes, 25 μl of murine IL-4/IL-13 antagonist QY (2.0 mg/ml) and 25 μl of rhC3-derivative H5 (2.0 mg/ml) are injected subcutaneously first. After 60 min, 100 μl of PBS/Imject Alum containing increasing doses of OVA (7.5, 15, 30, 60, 90 μg) are injected subcutaneously at the site of the gelled polymer composit (50 μl at each side of the gelled polymer). At day 3 and day 6 within each 9-day interval, additional 225 μl PBS, pH 7.4, containing 150 μl of the PLGA-PEG-PLGA triblock copolymer of Example 2.1. (25% w/v polymer), 25 μl of TNFR1-ODN/Cellfectin complexes, 25 μl of murine IL-4/IL-13 antagonist QY (2.0 mg/ml) and 25 μl of rhC3-derivative H5 (2.0 mg/ml) are injected subcutaneously at the site of the first injection. The injection procedure is repeated at a different subcutaneous site until the highest dose of alum-adsorbed OVA is injected.

*Experimental animal groups.* Three groups of wild-type C57BL/6 mice (each group: 10 mice) are analyzed: group 1: immunization (3 x alum-OVA), therapy with non-loaded polymer and PBS; group 2: immunization (3 x alum-OVA), therapy with increasing doses of alum-OVA only; group 3: immunization (3 x alum-OVA), therapy with loaded polymer and increasing doses of alum-OVA.

*Challenge procedure.* One week after completed immune-therapeutic treatment, mice are challenged with 1% aerosolized OVA (as described above), 30 min/day for 3 consecutive days.

*Measurement of AHR: Lung resistance.* Measurement of airway hyperreactivity (AHR), defined as bronchoconstriction in response to methacholine, is performed in two animals of each group. Three days after the final aerosol exposure, the mice are sacrificed by exsanguination after i.p. injection of a ketamine/xylazine overdose, and analyses of bronchoalveolar lavage (BAL), lung tissue, and blood samples are performed.

Measurements of pulmonary resistance ($R_L$) are performed in anesthetized, mechanically ventilated mice (Yiamouyiannis et al., 1999). After anesthetizing the animals with pentobarbital (75 mg/kg i.p. injection), the abdominal inferior vena cava is cannulated, and a tracheostomy catheter is placed. The chest is opened by a small anterior incision, and the animal is placed in a whole-body plethysmograph. Mechanical ventilation is established with a small rodent respirator delivering a 10 ml/kg tidal volume at 140 breaths/minute, with a positive end-expiratory pressure (PEEP) of 3 cm $H_2O$. Values for $R_L$ are calculated by analysis of electrical signals proportional to lung volume, airflow, and transpulmonary pressure. Changes in lung volume are determined from the measured changes in plethysmographic pressure and are differentiated over time to obtain flow measurements. Transpulmonary pressure is obtained from the difference between measured pressures at the airway opening and within the plethysmograph. After the establishment of baseline lung function, the animal receives sequentially increasing intravenous doses of methacholine (Sigma; 3 to 3000 μg/ml in 1 ml/kg body weight increments). Maximal $R_L$ responses are determined from measurements averaged over 6-second intervals. Pulmonary function is allowed to return to baseline values before each subsequent dose.

*Bronchoalveolar lavage (BAL) analysis.* The lungs from five animals of each group are lavaged *in situ* with five 1 ml aliquots of sterile saline, with 3 to 4 ml BAL fluid recovered from each animal. The BAL is centrifuged and resulting cell pellets are suspended in 250 μl saline. BAL protein concentrations are measured in the supernatants by the bicinchoninc acid (BCA) assay using the BCA™ Protein Assay Kit (Pierce, USA) and bovine serum albumin as standard. Total leukocytes are counted in a hemocytometer using trypan blue dye exclusion as a measure of viability. Cytospin slides are made and stained with May-Grunwald/Giemsa to determine the BAL cell differential. The remaining cells are analyzed by fluorescence flow cytometry. For these analyses, BAL samples are washed in phosphate-buffered saline (PBS) containing 0.2% bovine serum albumin and 0.1% $NaN_3$. Aliquots containing $10^4$ to $10^5$ cells are incubated with 100 μl of appropriately diluted antibodies for 30 min at 4°C. After staining, the cells are washed twice with the above PBS solution, and relative fluorescence intensities are determined on a 4-decade log scale by flow cytometric analysis using a FACScan (Becton Dickinson). Fluorescent monoclonal antibodies used for the fluorescence flow cytometric analyses are directed against B cell and T cell antigens including CD3ε, TCRβ, TCRδ, CD4, CD8, and CD45.

*Analysis of serum levels of OVA-specific antibodies.* Mice are bled at day 0 before immunization with OVA, one week after immunization with OVA (before starting immunotherapy), and two weeks after the last immunotherapeutic treatment. Analyses include the determination of serum levels of OVA-specific antibodies and cytokines.

Murine anti-OVA IgE and IgG subclasses are determined by ELISA. Plates are coated with 10 μg OVA in 100 μl 0.1 M $NaHCO_3$ for 6 h at 37°C, followed by blocking with 200 μl 3% BSA in PBS, pH 7.4, for 2 h at 37°C. After washing, 100 μl of 1:40 serum dilutions with PBS, pH 7.4, containing 1% BSA are incubated overnight at 4°C. The amount of bound antibody is analyzed using horseradish peroxidise-conjugated antibodies with specificity for murine heavy chain classes (IgE, IgGl, IgG2a, IgG2b, IgG3). Analysis is performed at 405 nm in a microplate autoreader.

*Analysis of serum levels of cytokines.* The cytokine supernatant is profiled using a panel of 27 cytokines including the TH2 cytokines IL-4, IL-5 and IL-13.

*Tissue analysis.* For tissue immunofluorescence, unmanipulated lungs (not exposed to BAL or methacholine) are excised, cut into small pieces, and are rapidly frozen in optimal cutting temperature embedding media. The pieces are then cut into 5 μm frozen sections using a Hacker cryostat, mounted onto microscope slides, and stored at -20°C. For immunofluorescence staining, the slides are fixed in acetone (-20°C) for 5 minutes, dried, and blocked with 1% ChromPure IgG solution (Jackson ImmunoResearch) for 30 min at room temperature. After two washes with PBS containing 1% $NaN_3$, specific fluorescent monoclonal antibodies are added to the tissue and incubated for 60 min in a humidity chamber. Slides are then washed twice with PBS containing 1% $NaN_3$, and then analyzed by fluorescence microscopy.

For staining with hematoxylin and eosin (H&E), unmanipulated lungs (not exposed to BAL or methacholine) are excised, fixed with 10% buffered formalin, and stained with H&E according to standard protocols.

**Literature for example 7**

Yiamouyiannis et al., Am. J. Pathol. 154 : 1911-1921,1999.

**EXAMPLE 8. INHIBITORY EFFECT OF ANTI-TNF THERAPY ON ALLERGEN-SPECIFIC IMMUNOTHERAPY IN A PATIENT WITH INSECT VENOM ALLERGY**

This example demonstrates that total TNF blockade by treatment with etanercept (a fusion protein composed of the extracellular part of two TNFR2 molecules coupled to the Fc region of an IgGl molecule) and the mouse-human chimeric anti-TNF antibody infliximab, both of which inhibit interactions of sTNF with TNFR1 and of mTNF with both TNFR1 and TNFR2, interferes with the development of allergen-specific sIgG4 during specific immunotherapy, a humoral indicator for SIT success.

*Case report from the Allergy Center of the Technical University of Munich (TUM), Germany.* A female patient (born in 1946, 57 years old at the first visit in July 2003) with systemic vespid venom allergy was under anti-TNF therapy with the soluble TNF receptor etanercept (Enbrel®) for ankolysing spondylitis since July 2002 (see Figure 9). In 07/2003, the patient experienced a mild to moderate anaphylactic reaction (grade 1-2 according to Ring and Messmer) with generalized urticaria, subjective breathing problems and dizziness after a wasp sting.

**Table 1: Intradermal skin test results of the patient before SIT in 2003**

| Venom Conc. (μg(/ml) | HBV-Wheal Size[1] | HBV-Erythema Size[1] | W-Wheal Size[1] | W-Erythema Size[1] |
|---|---|---|---|---|
| 0.0001 | 0 | 0 | 0 | 0 |
| 0.001 | 0 | 0 | 10 | 10 |
| 0.01 | 0 | 0 | 12 | 18 |
| 0.1 | 0 | 5 | 18 | 25 |

[1]diameter (mm); W, vespula vulgaris; HBV, honeybee venom
Materials were obtained from Allergy Therapeutic Bencard

Due to the high re-exposure risk and because of the exceedingly high levels of specific IgE antibodies against vespid venom allergens in the patient's serum (see tables 1 and 2), allergen-specific immunotherapy (SIT) was initiated in this patient in 12/2003.

**Table 2: IgE laboratory results of the patient before SIT in 2003**

| | VV-sIgE (i3) | HBV-sIgE (i1) | CCD Marker (w203) | Total IgE (TIE) |
|---|---|---|---|---|
| sIgE (kU/L) | >100 | <0.1 | <0.1 | -- |
| tIgE (IU/mL) | -- | -- | -- | 237 |
| W, vespula vulgaris; HBV, honeybee venom; CCD, carbohydrate cross-reactive determinants<br>Materials were obtained from Thermo Fisher Scientific Phadia and/or Siemens Diagnostics | | | | |

Shortly after initiation of SIT, the consulting rheumatologist of the patient changed the anti-TNF treatment regimen to infliximab infusions (Remicade®) in 6-week intervals as the patient had suffered from increasing iritis symptoms under etanercept which were stopped by changing to infliximab. Infliximab was continued ever since in the patient without major side effects.

SIT at a maintenance dosage of 100 μg was continued until August 2005, when the patient underwent a sting challenge test with a living Vespula vulgaris. During the period from 2003 to 2005, only mild and local, but no systemic side effects were noted in the patient. In general, SIT was tolerated very well by the patient.

During the sting challenge, the patient started to develop the same type of general allergic reaction as originally in 2003, which was treated and stopped by systemic application of antihistamine and steroid.

Since SIT under 100 μg was not successful in this patient, the maintenance dosage was increased to 200 μg in December 2005 according to the guidelines. This SIT dosage was continued until 2009. Further control visits took place in the years 2006 through 2009. SIT was stopped without performing a second sting challenge as the patient refused to it.

Laboratory analysis by Western blot and by ELISA of the patient's sIgE and sIgG4 antibodies against whole insect venoms and against recombinant insect venom allergens (Ves v 1, Ves v 2, Ves v 3, Ves v 5) over the time period from July 2003 to July 2007 showed that the patient had very high sIgE levels to Ves v 5 and low levels to Ves v 1. No sIgE was detectable against Ves v 2 and Ves v 3.

The sIgE levels gradually decreased over time, but were still readily detectable even in the period between 2007 and 2009 (compare Tables 2 and 3).

**Table 3: IgE laboratory results of the patient during SIT with 200 μg in 2007**

| | VV-sIgE (i3) | HBV-sIgE (i1) | CCD Marker (w203) | Total IgE (TIE) |
|---|---|---|---|---|
| sIgE (kU/L) | 14.4 | <0.1 | n.d. | -- |
| tIgE (IU/mL) | -- | -- | -- | 110 |
| VV, vespula vulgaris; HBV, honeybee venom; CCD, carbohydrate cross-reactive determinants<br>Materials were obtained from Thermo Fisher Scientific Phadia and/or Siemens Diagnostics | | | | |

This was also reflected by the Western blot results, which also showed a slight decrease of the intensity of the sIgE bands (not shown).

Western blots also demonstrated very low intensity bands for sIgG4 against Ves v5 which was commercially available in 2003, but showed no increase in intensity any time during SIT from 2003 to 2009. Similar results were also obtained in the ELISA analysis, where sIgG4 levels were detectable already in 2003 before SIT mainly against Ves v 1 and Ves v 5, which decreased slightly over time during SIT.

*Discussion.* The lack of any allergen-specific IgG4 response is highly unusual, since out of >200 patients under venom immunotherapy at the Allergy Center of TUM, who had been analyzed for sIgE and sIgG4 levels, this patient was the first and only one who failed to develop any detectable increase in the allergen-specific IgG4 response, a humoral indicator for SIT success. Even amongst the group of SIT therapy failures/non-responders, we routinely observed an increase of sIgG4 levels to at least one allergen. Thus, this patient was the first and only one ever without any increase in sIgG4 to any of the vespid venom allergens. Apparently, total TNF blockade via combined inhibition of interactions of sTNF with TNFR1 and mTNF with both TNFR1 and TNFR2 by treatment with etanercept (Enbrel®) or infliximab (Remicade®) interferes with the development of allergen-specific sIgG4 during specific immunotherapy. To our knowledge this was the only patient worldwide that received immunotherapy under anti-TNF inhibition treatment. Since there are several lines of evidence that TNFR2-mediated signaling is essential for the induction of tolerance, inhibition of TNFR2 appears to be responsible for the inhibitory effect of anti-TNF-alpha therapy on the efficacy of specific immunotherapy.

**EXAMPLE 9. INHIBITORY EFFECT OF ANTI-TNF TREATMENT ON OVA-SPECIFIC IMMUNOTHERAPY IN MICE**

Confirmation of the inhibitory effect of anti-TNF treatment on the efficacy of specific immunotherapy is obtained from experiments with OVA-sensitized mice.

*Animals and materials.* Female wild-type C57BL/6 mice 6 to 10 weeks of age are purchased from Charles River (Sulzfeld, Germany). The animals are kept under specific pathogen-free conditions and maintained on OVA-free diets. Ovalbumin (OVA) Grade V is purchased from Sigma, Imject Alum from Pierce/KMF, hamster anti-TNF-alpha monoclonal IgG antibody TN3-19.12 from Natutec-GmbH (eBioscience), rat monoclonal antibodies against murine IgE and IgG1, and goat antisera against murine IgG2a, IgG2b, and IgG3 from BD Pharmigen.

*Preparation of alum-adsorbed ovalbumin* (*OVA*). OVA (100 μg) is solved in 0.3 ml of PBS, pH 7.4, and mixed with 0.70 ml Imject Alum.

*Treatment procedure.* Mice are injected intraperitoneally 100 μl alum-adsorbed OVA (10 μg OVA in 100 μl of PBS/Imject Alum) on Days 1, 7, 14 and 28. On Days 42, 44 and 46 mice are injected subcutaneously 1 mg OVA in 200 μl of PBS for hyposensitization. One group of animals receives 100 μg hamster anti-TNF-alpha moAb TN3-19.12 on Days 42 and 53. On Days 53, 56, 59 and 60 mice are challenged by inhalative exposure to OVA aerosol (6% in PBS) for 1 hour using a Pari-Boy nebulizer (Pari, Starnberg, Germany). On Days 0, 14, 28, 42, 53 and 60 serum levels of OVA-specific antibodies are determined and bronchoalveolar lavage (BAL) analyses are performed (see Fig. 9 for an overview of the treatment schedule).

Five groups of wild-type C57BL/6 mice (each group: 10 mice) are analyzed:

group 1: sensitization with OVA, hyposensitization, inhalative OVA-challenge
group 2: sensitization with OVA, no hyposensitization (placebo), inhalative OVA-challenge
group 3: sensitization with OVA, anti-TNF-alpha treatment, hyposensitization, inhalative OVA-challenge
group 4: sensitization with OVA, anti-TNF-alpha treatment, no hyposensitization (placebo), inhalative OVA-challenge
group 5: only placebo treatment

*Bronchoalveolar lavage (BAL) analysis.* The lungs are lavaged in situ and analysed as described (Aguilar-Pimentel et al., 2010). Fig. 10 demonstrates that with anti-TNF-alpha treatment, the beneficial effect of SIT is significantly reduced as evident from a higher degree of airway eosinophilia.

*Analysis of serum levels of OVA-specific antibodies.* Mice are bled at day 0 before immunization with OVA, and on Days 14, 28, 42, 53 and 60. Fig. 11 demonstrates that the SIT-induced allergen-specific IgG1 response, the humoral hallmark of successful SIT in the mouse, comparable to human IgG4, is dramatically reduced in the anti-TNF-alpha-treated group.

*Discussion.* In this murine model, many of the features seen in the anti-TNF-alpha-treated patient (see Example 8) are also observed. Most strikingly is the dramatically reduced allergen-specific IgG1 response, but other parameters such as airway eosinophilia demonstrate also the inhibitory effect of anti-TNF-alpha treatment on the efficacy of specific immunotherapy.

**Literature for example 9**

Aguilar-Pimentel et al., Am. J. Respir. Crit. Care Med. 181: 7-16; 2010

**Claims**

1. Pharmaceutical composition for modulation of T cell and B cell responses made of one or more preparations and comprising a therapeutically effective dose of at least one inhibitor of tumor necrosis factor receptor 1 (TNFR1)-mediated functions and at least one antigen or allergen.

2. Composition of claim 1, comprising in addition a therapeutically effective dose of at least one inhibitor of IL-4/IL-13-mediated effects, and/or a therapeutically effective dose of at least one recombinant human C3-derivative.

3. Composition according to claim 1 or 2, wherein at least one inhibitor of TNFR1-mediated functions and at least one antigen or allergen and, optionally, at least one inhibitor of IL-4/IL-13-mediated effects, and/or at least one recombinant human C3-derivative are coated or adsorbed on or embedded in a matrix, wherein the matrix is selected as to enable sustained release of one or more antigens or allergens and one or more inhibitors of TNFR1-mediated functions, and, optionally of one or more inhibitors of IL-4/IL-13-mediated effects, and/or of one or more recombinant human C3-derivatives.

4. Composition according to claim 1 or 2, wherein at least one inhibitor of TNFR1-mediated functions and, optionally, at least one inhibitor of IL-4/IL-13-mediated effects, and/or at least one recombinant human C3-derivative are coated or adsorbed on or embedded in a first matrix, wherein the first matrix is selected as to enable sustained release of one or more inhibitors of TNFR1-mediated functions, and, optionally of one or more inhibitors of IL-4/IL-13-mediated effects, and/or of one or more recombinant human C3-derivatives, and wherein at least one antigen or allergen is coated or adsorbed on or embedded in a second matrix, different from the first matrix, wherein the second matrix is an adjuvant providing a depot effect for antigen or allergen presentation, wherein the first and the second matrices are provided in separate preparations.

5. Composition according to any of the claims 1 to 4, wherein the inhibitor of TNFR1-mediated functions is selected from one of three groups of inhibitors, wherein the inhibitors of the first group include TNFR1-specific antisense oligonucleotides, antagonistic TNF mutants with specificity for TNFR1, anti-TNFR1 aptamers, anti-TNFR1 antibodies and fragments thereof, and allow specific inhibition of TNFR1 while leaving TNFR2 signaling intact, wherein inhibitors of the second group include glutathione precursors such as N-acetyl-L-cysteine, glutathione and derivatives thereof such as S-methylglutathione, and salicylates such as acetylsalicylic acid and sodium salicylate, and allow selective inhibition of TNFR1-mediated functions, and wherein inhibitors of the third group include dominant-negative sTNF mutants and allow specific inhibition of sTNF while leaving the mTNF-TNFR1 and mTNF-TNFR2 interactions intact.

6. Composition according to any of the claims 1 to 5, wherein the inhibitors of TNFR1-mediated functions are small inhibitor molecules which can readily access sites such as the immunological synapse formed between the antigen presenting cells and the T cells, and wherein the inhibitors of TNFR1-mediated functions optionally exhibit a short plasma half-life to minimize potential systemic side effects upon diffusion away from the delivery site.

7. Composition according to any of the claims 2 to 6, wherein the inhibitor of IL-4/IL-13-mediated effects is a human IL-4 mutant, preferably with one to three mutations, most preferably in at least one of the positions R121, Y124, and S125.

8. Composition according to any of the claims 2 to 7, wherein the recombinant human C3-derivative is selected from a group of C3 convertases capable of forming a physicochemically stable convertase with an extended half-life of its decay-dissociation, providing resistance to the action of Factors H and I, and exerting little or negligible C5-cleaving activity, and wherein the recombinant human C3-derivative is selected from a group of C3 convertases providing a sequence identity with human C3 of at least 70%.

9. Composition according to any of the claims 3 to 8, wherein the matrix of claim 3 and the first matrix of claim 4 is a biodegradable or biostable polymer, preferably biodegradable, more preferably thermogelling, even more preferably reverse thermogelling, in particular selected from the group consisting of polyethylene, polypropylene, polyethylene oxide (PEO), polypropylene oxide (PPO), polyurethane, polyurea, polyamides, polycarbonates, polyaldehydes, polyorthoesters, polyiminocarbonates, poly caprolactone (PCL), poly-D,L-lactic acid (PDLLA), poly-L-lactic acid (PLLA), lactides of said lactic acids, polyphosphazenes, polyglycolic acids, albumin, monomethoxypoly(ethylene glycol) (MPEG), trimethylated chitosan derivatives, or copolymers or mixtures of any of the above including poly(lactic-co-glycolic acid) (PLGA), copolymers of L-lactide and D,L-lactide, polyester copolymers, diblock copolymers consisting of MPEG and PCL, MPEG and PCL-ran-PLLA, MPEG and PLGA, PEO and PLLA, trimethylated chitosan and $\alpha$, $\beta$-glycerophosphate, triblock copolymers consisting of PEO and PLLA, PLGA-PEG-PLGA, PEG-PLGA-PEG, PEG-PCL-PEG, and PEO-PPO-PEO (Poloxamers), wherein the polymer is preferably reverse thermogelling and wherein the gelling temperature is between 20°C and 40°C, preferably between 25°C and 35°C, and/or wherein the 90% degradation of the polymer weight in body environment and/or 90% release of one or more antigens or allergen and/or of one or more inhibitors of TNFR1-mediated functions and/or of one or more inhibitors of IL-4/IL-13-mediated effects and/or of one or more recombinant human C3-derivatives from the polymer is completed within 1 to 10 days, preferably within 1 to 3 days.

10. Composition according to any of the claims 4 to 9, wherein the second matrix is selected from the group consisting of aluminium salts, oil in water or water in oil emulsions such as Montanide, Adjuvant 65 and Lipovant, liposomes, polymeric microsphere adjuvants, and virosomes, preferably selected from aluminium phosphate or aluminium hydroxide gels.

**11.** Composition according to one of the claims 1 to 10, wherein all components are mixed as a single preparation, or wherein the first matrix and at least one inhibitor of TNFR1-mediated functions and, optionally, at least one inhibitor of IL-4/IL-13-mediated effects and/or at least one rhC3-derivative are mixed as a first preparation and the antigen or allergen and the second matrix are mixed as a second preparation, and/or wherein the composition is galenically prepared for administration by injection or by implantation, subcutaneously, intradermally, intramuscularly, nasally, transbucally, transmucosally, sublingually, rectally, vaginally, intraocularly, intra tumor, or topically.

**12.** Use of a composition according to one of the claims 1 to 11 for local selective inhibition of TNFR1-mediated functions and, optionally, local IL-4/IL-13 inhibition and/or local C3 depletion in an organism, preferably a human, in need thereof, in particular for the treatment of T cell-mediated diseases, preferably selected from the group consisting of allergy, allergic asthma, acute respiratory stress (ARDS), type 1 diabetes, systemic lupus erythematodes, glomerulonephritis, rheumatoid arthritis, multiple sclerosis, dermatomyositis, nephritis, Parkinson's disease, Alzheimer's disease, pemphigoid, sepsis, myocardial ischemia, acute myocardial infarction (AMI), reperfusion, hemodialysis, paroxysmal nocturnal hemoglobinuria (PNH), age-related macula degeneration (AMD), cardiopulmonary bypass (CPB), angioplasty, hyperacute rejection, transplant rejection, stroke, burns, spinal cord injury, and traumatic brain injury (TBI), wherein preferred fields of application include allergy, allergic asthma, type 1 diabetes, rheumatoid arthritis, and multiple sclerosis, and wherein the pharmaceutical composition is administered in a therapeutically effective dose.

**13.** Method for manufacturing a pharmaceutical composition according to one of the claims 1 to 11, wherein the components are mixed with each other in a therapeutically effective quantity, wherein optionally galenic compounds are additionally admixed to one or all of the preparations.

FIG. 1

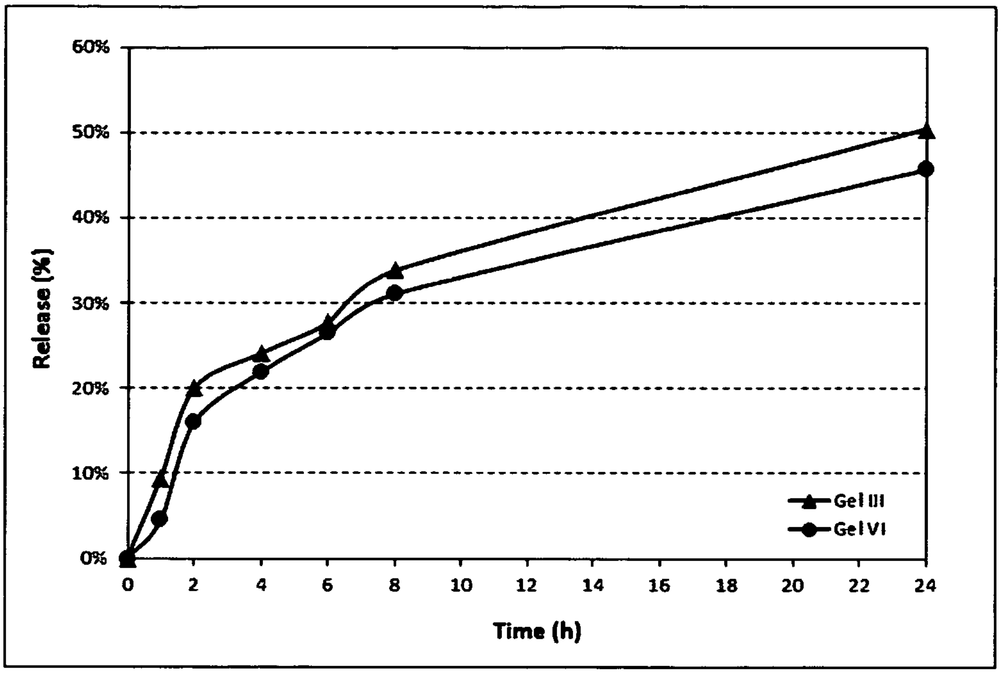
60%
50%
40%
30%
20%
10%
0%
Release (%)
0
2
4
6
8
10
12
14
16
18
20
22
24
Time (h)
Gel III
Gel VI

FIG. 2

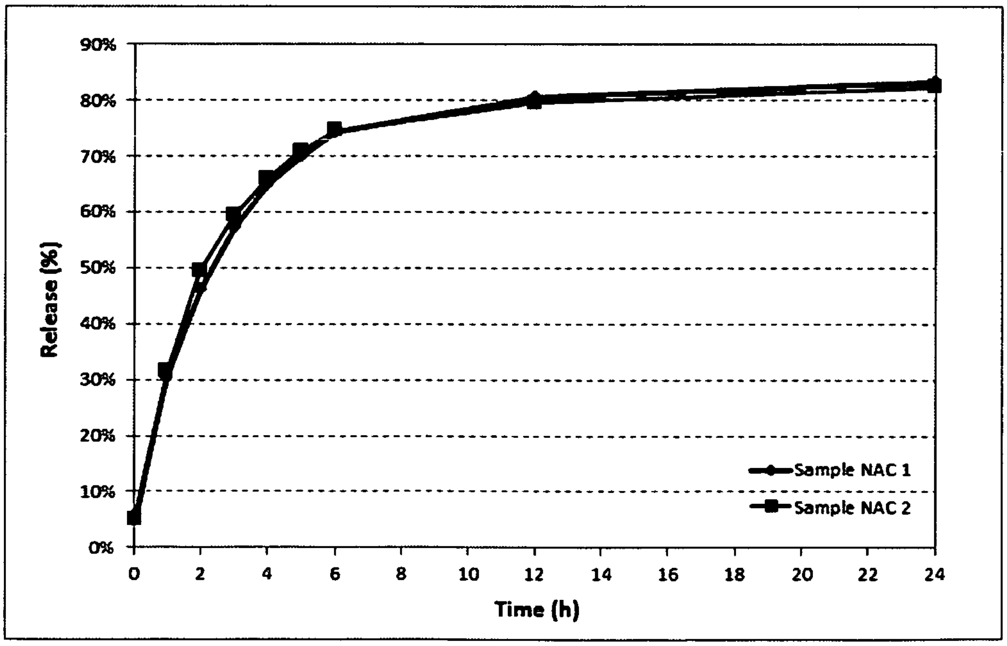
90%
80%
70%
60%
50%
40%
30%
20%
10%
0%
Release (%)
0
2
4
6
8
10
12
14
16
18
20
22
24
Time (h)
Sample NAC 1
Sample NAC 2

FIG. 3

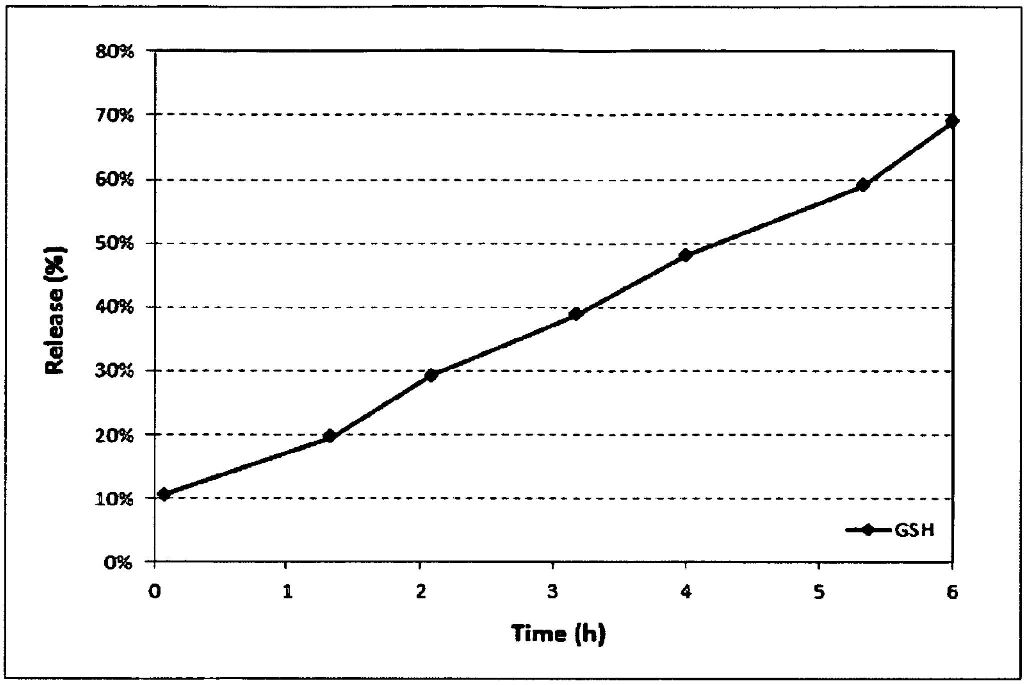
80%
70%
60%
50%
40%
30%
20%
10%
0%
Release (%)
0
1
2
3
4
5
6
Time (h)
GSH

FIG. 4

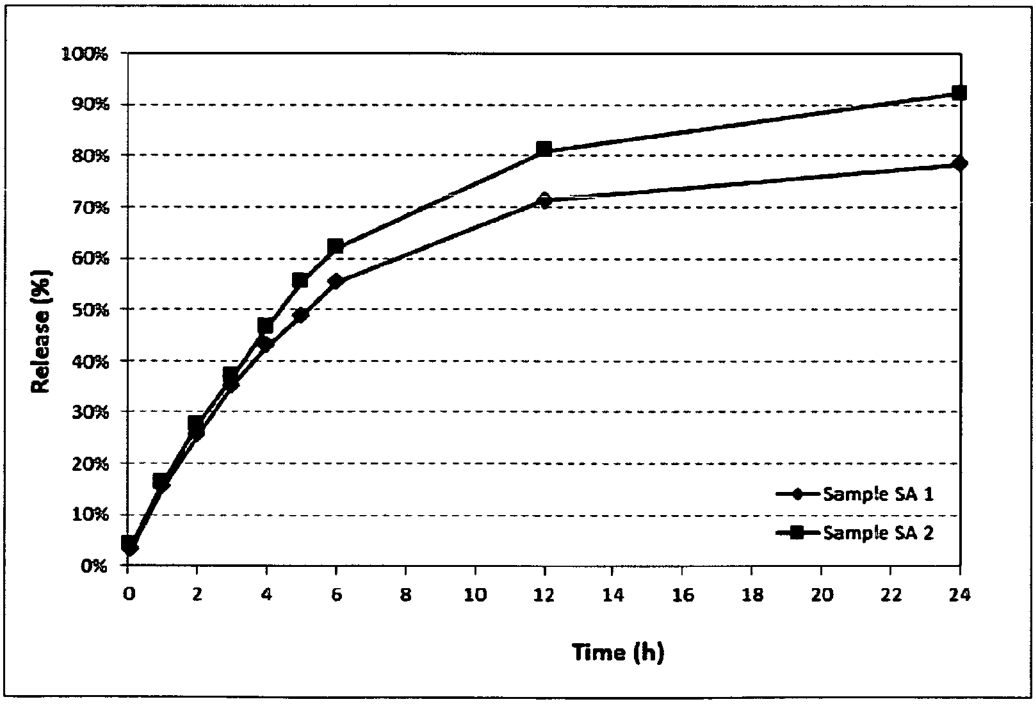
100%
90%
80%
70%
60%
50%
40%
30%
20%
10%
0%
Release (%)
0
2
4
6
8
10
12
14
16
18
20
22
24
Time (h)
Sample SA 1
Sample SA 2

FIG. 5

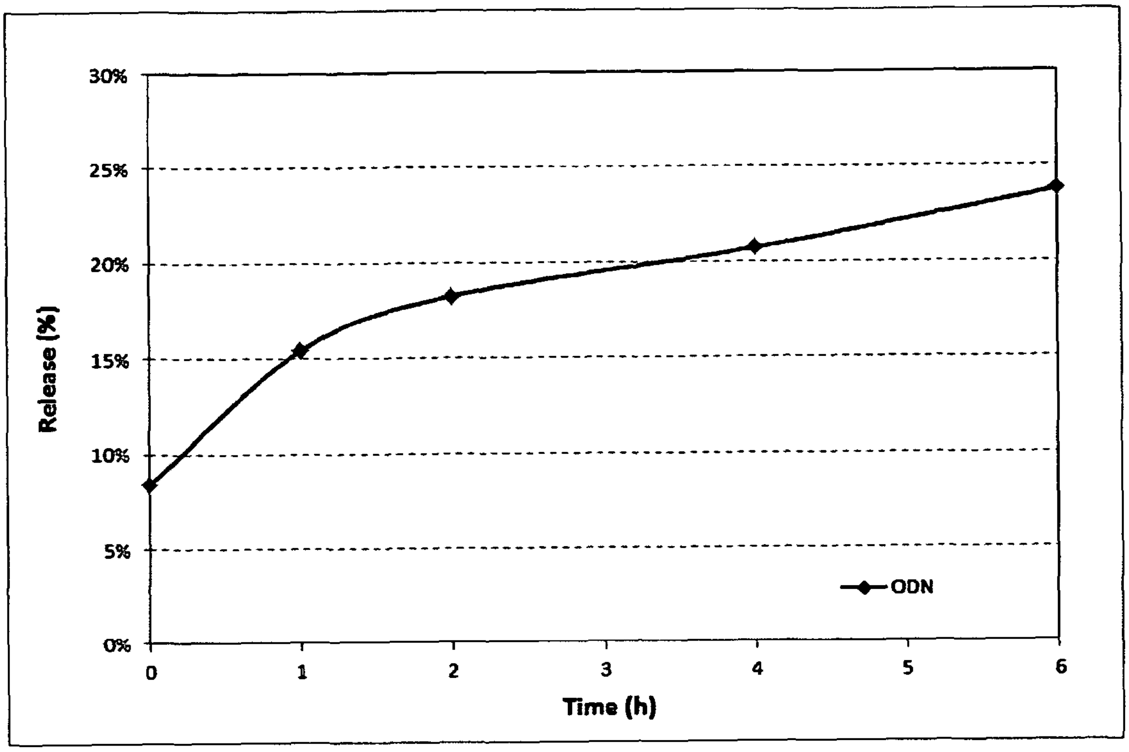
30%
25%
20%
15%
10%
5%
0%
Release (%)
0
1
2
3
4
5
6
Time (h)
ODN

FIG. 6

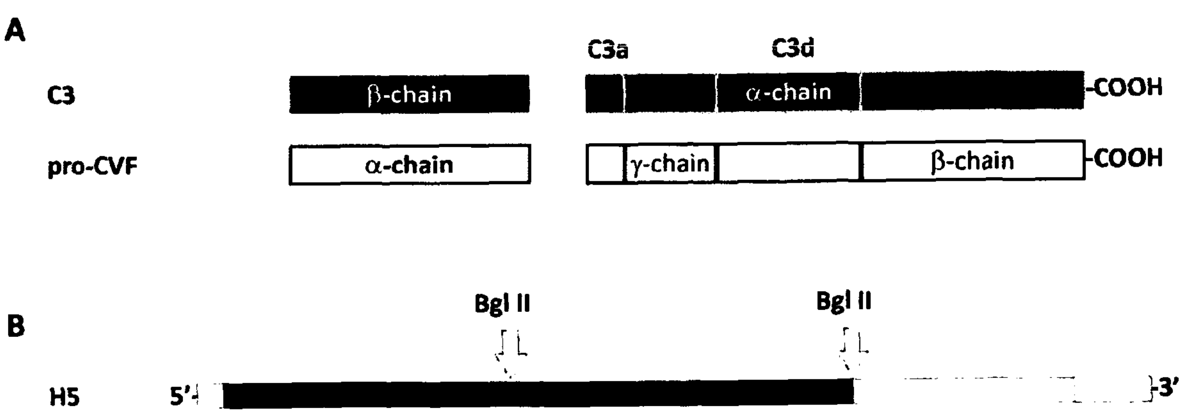
A
C3a
C3d
C3
β-chain
α-chain
-COOH
pro-CVF
α-chain
γ-chain
β-chain
-COOH
B
Bgl II
Bgl II
H5
5'
3'

FIG. 7

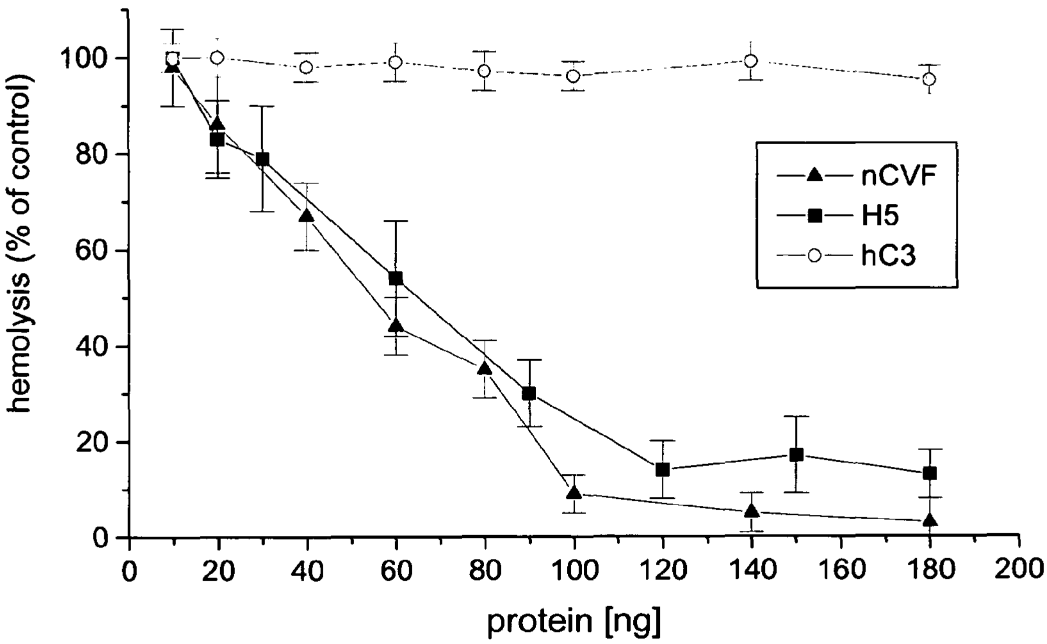
hemolysis (% of control)
100
80
60
40
20
0
0 20 40 60 80 100 120 140 160 180 200
protein [ng]
nCVF
H5
hC3

FIG. 8

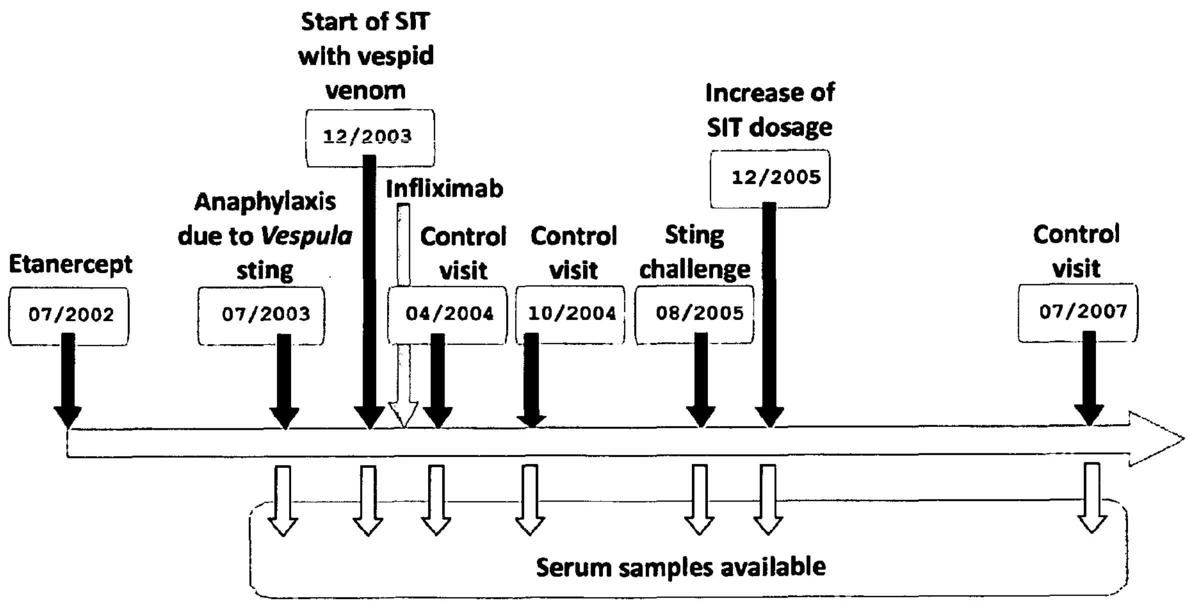
Start of SIT with vespid venom
12/2003
Increase of SIT dosage
12/2005
Infliximab
Anaphylaxis due to *Vespula* sting
07/2003
Etanercept
07/2002
Control visit
04/2004
Control visit
10/2004
Sting challenge
08/2005
Control visit
07/2007
Serum samples available

FIG. 9

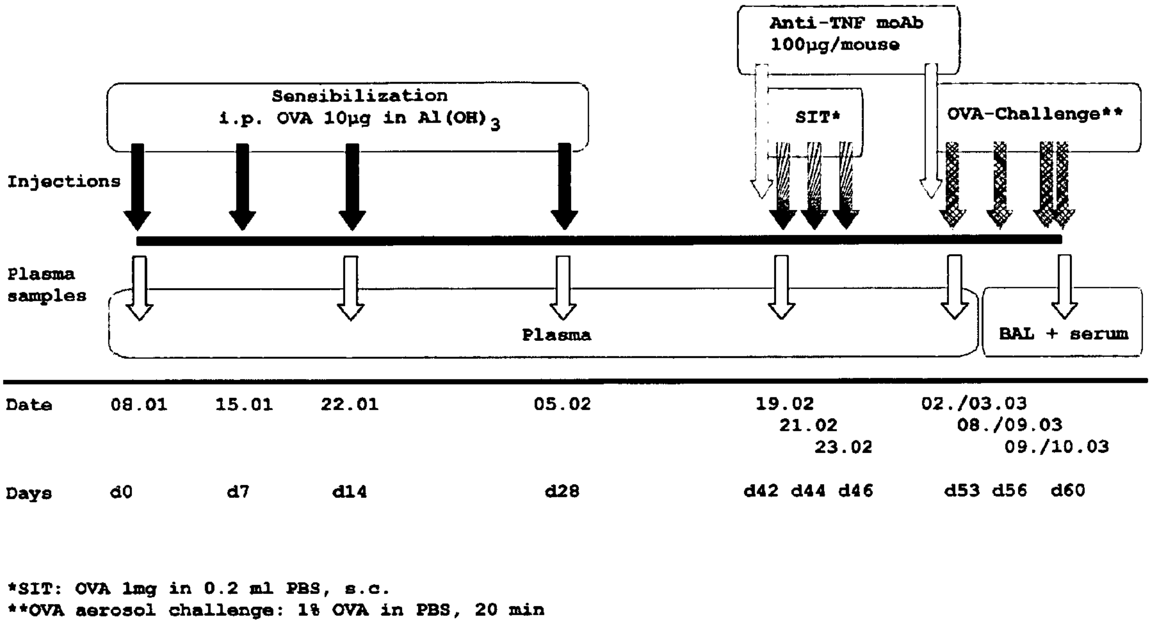
Anti-TNF moAb
100µg/mouse
Sensibilization
i.p. OVA 10µg in Al(OH)3
SIT*
OVA-Challenge**
Injections
Plasma
samples
Plasma
BAL + serum
Date
08.01
15.01
22.01
05.02
19.02
21.02
23.02
02./03.03
08./09.03
09./10.03
Days
d0
d7
d14
d28
d42 d44 d46
d53 d56 d60
*SIT: OVA 1mg in 0.2 ml PBS, s.c.
**OVA aerosol challenge: 1% OVA in PBS, 20 min

FIG. 10

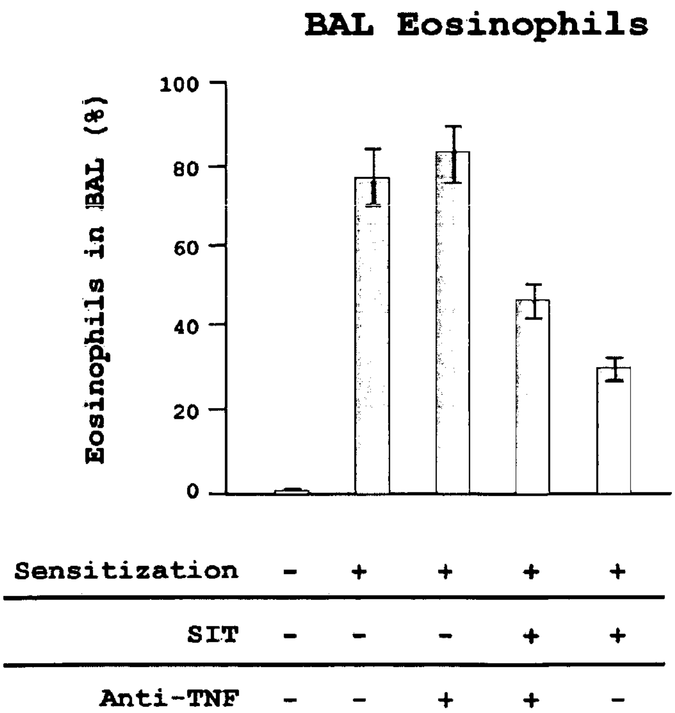
BAL Eosinophils
Eosinophils in BAL (%)
100
80
60
40
20
0
Sensitization - + + + +
SIT - - - + +
Anti-TNF - - + + -

FIG. 11

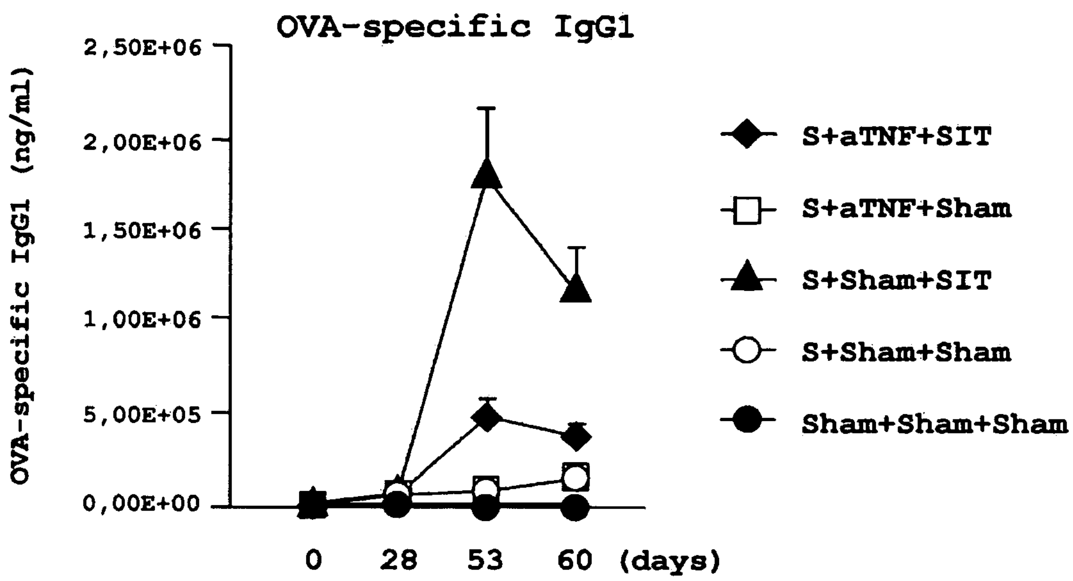
OVA-specific IgG1
OVA-specific IgG1 (ng/ml)
2,50E+06
2,00E+06
1,50E+06
1,00E+06
5,00E+05
0,00E+00
0
28
53
60
(days)
S+aTNF+SIT
S+aTNF+Sham
S+Sham+SIT
S+Sham+Sham
Sham+Sham+Sham

## FIG. 12

1 ctcctccagc tcttcctgtc ccgctgttgc aacactgcct cactcttccc ctcccacctt
61 ctctcccctc ctctctgctt taattttctc agaattctct ggactgaggc tccagttctg
121 gcctttgggg ttcaagatca ctgggaccag gccgtgatct ctatgcccga gtctcaaccc
181 tcaactgtca ccccaaggca cttgggacgt cctggacaga ccgagtcccg ggaagcccca
241 gcactgccgc tgccacactg ccctgagccc aaatggggga gtgagaggcc atagctgtct
301 ggcatgggcc tctccaccgt gcctgacctg ctgctgccac tggtgctcct ggagctgttg
361 gtgggaatat acccctcagg ggttattgga ctggtccctc acctagggga cagggagaag
421 agagatagtg tgtgtcccca aggaaaatat atccaccctc aaaataattc gatttgctgt
481 accaagtgcc acaaaggaac ctacttgtac aatgactgtc caggcccggg gcaggatacg
541 gactgcaggg agtgtgagag cggctccttc accgcttcag aaaaccacct cagacactgc
601 ctcagctgct ccaaatgccg aaaggaaatg ggtcaggtgg agatctcttc ttgcacagtg
661 gaccgggaca ccgtgtgtgg ctgcaggaag aaccagtacc ggcattattg gagtgaaaac
721 cttttccagt gcttcaattg cagcctctgc ctcaatggga ccgtgcacct ctcctgccag
781 gagaaacaga acaccgtgtg cacctgccat gcaggtttct ttctaagaga aaacgagtgt
841 gtctcctgta gtaactgtaa gaaaagcctg gagtgcacga agttgtgcct accccagatt
901 gagaatgtta agggcactga ggactcaggc accacagtgc tgttgcccct ggtcattttc
961 tttggtcttt gccttttatc cctcctcttc attggtttaa tgtatcgcta ccaacggtgg
1021 aagtccaagc tctactccat tgtttgtggg aaatcgacac ctgaaaaaga gggggagctt
1081 gaaggaacta ctactaagcc cctggcccca aacccaagct tcagtcccac tccaggcttc
1141 acccccaccc tgggcttcag tcccgtgccc agttccacct tcacctccag ctccacctat
1201 acccccggtg actgtcccaa ctttgcggct ccccgcagag aggtggcacc accctatcag
1261 ggggctgacc ccatccttgc gacagccctc gcctccgacc ccatccccaa ccccccttcag
1321 aagtgggagg acagcgccca caagccacag agcctagaca ctgatgaccc cgcgacgctg
1381 tacgccgtgg tggagaacgt gcccccgttg cgctggaagg aattcgtgcg gcgcctaggg
1441 ctgagcgacc acgagatcga tcggctggag ctgcagaacg ggcgctgcct gcgcgaggcg
1501 caatacagca tgctggcgac ctggaggcgg cgcacgccgc ggcgcgaggc cacgctggag
1561 ctgctgggac gcgtgctccg cgacatggac ctgctgggct gcctggagga catcgaggag
1621 gcgctttgcg gccccgccgc cctcccgccc gcgcccagtc ttctcagatg aggctgcgcc
1681 cctgcgggca gctctaagga ccgtcctgcg agatcgcctt ccaaccccac tttttctgg
1741 aaaggagggg tcctgcaggg gcaagcagga gctagcagcc gcctacttgg tgctaacccc
1801 tcgatgtaca tagcttttct cagctgcctg cgcgccgccg acagtcagcg ctgtgcgcgc
1861 ggagagaggt gcgccgtggg ctcaagagcc tgagtgggtg gtttgcgagg atgagggacg
1921 ctatgcctca tgcccgtttt gggtgtcctc accagcaagg ctgctcgggg gcccctggtt
1981 cgtccctgag ccttttcac agtgcataag cagtttttt tgtttttgtt ttgttttgtt
2041 ttgtttttaa atcaatcatg ttacactaat agaaacttgg cactcctgtg ccctctgcct
2101 ggacaagcac atagcaagct gaactgtcct aaggcagggg cgagcacgga acaatggggc
2161 cttcagctgg agctgtggac ttttgtacat acactaaaat tctgaagtta aagctctgct
2221 cttggaaaaa aaaaaaaaaa aaaaaaaaaa aaaaaaaa

## FIG. 13

1 VRSSSRTPSD *K*PVAHVVANP QAEGQLQW**L**N **RR**ANALLANG VELRDNQLVV PSEGLYLIYS

61 QVLF*K*GQGCP STHVLLTHTI SRI**AVSYQT***K* VNLLSAI*K*SP CQRETPEGAE A*K*PWYEPIYL

121 GGVFQLE*K*GD RLSAEINRPD YLDF**AES**GQV YFGIIAL

## FIG. 14

**A) Human IL4, native form, DNA sequence**

```
  1 CACAAGTGCG ATATCACCTT ACAGGAGATC ATCAAAACTT TGAACAGCCT CACAGAGCAG
 61 AAGACTCTGT GCACCGAGTT GACCGTAACA GACATCTTTG CTGCCTCCAA GAACACAACT
121 GAGAAGGAAA CCTTCTGCAG GGCTGCGACT GTGCTCCGGC AGTTCTACAG CCACCATGAG
181 AAGGACACTC GCTGCCTGGG TGCGACTGCA CAGCAGTTCC ACAGGCACAA GCAGCTGATC
241 CGATTCCTGA AACGGCTCGA CAGGAACCTC TGGGGCCTGG CGGGCTTGAA TTCCTGTCCT
301 GTGAAGGAAG CCAACCAGAG TACGTTGGAA AACTTCTTGG AAAGGCTAAA GACGATCATG
361 AGAGAGAAAT ATTCAAAGTG TTCGAGCTGA
```

**Human IL4, native form, translated protein sequence**

```
  1 HKCDITLQEI IKTLNSLTEQ KTLCTELTVT DIFAASKNTT EKETFCRAAT VLRQFYSHHE
 61 KDTRCLGATA QQFHRHKQLI RFLKRLDRNL WGLAGLNSCP VKEANQSTLE NFLERLKTIM
121 REKYSKCSS*
```

**B) Human IL-4, RY mutant construct, DNA sequence**

```
  1 ATGGGTAGCA GCCATCATCA TCATCATCAC TCCAGCGGTC TGGTTCCTCG TGGTAGTCAT
 61 ATGCACAAAT GTGACATCAC CCTGCAGGAG ATTATCAAAA CACTGAATAG CCTGACGGAG
121 CAGAAAACAC TGTGTACCGA ACTGACGGTT ACCGACATCT TTGCCGCCTC CAAAAACACC
181 ACCGAGAAAG AAACCTTTTG TCGTGCTGCC ACTGTACTGC GTCAATTCTA TAGCCACCAT
241 GAAAAAGACA CCCGTTGCCT GGGAGCGACT GCCCAACAAT TCCATCGTCA TAAACAGCTG
301 ATCCGCTTCC TGAAACGTCT GGATCGTAAT CTGTGGGGAC TGGCAGGTCT GAATTCATGT
361 CCGGTGAAAG AGGCAAATCA GTCAACCCTG GAGAACTTTC TGGAACGCCT GAAAACCATT
421 ATGGATGAAA AAGACTCCAA ATGCTCGAGC TGA
```

**Human IL-4, RY mutant construct, translated protein sequence**

```
  1 MGSSHHHHHH SSGLVPRGSH MHKCDITLQE IIKTLNSLTE QKTLCTELTV TDIFAASKNT
 61 TEKETFCRAA TVLRQFYSHH EKDTRCLGAT AQQFHRHKQL IRFLKRLDRN LWGLAGLNSC
121 PVKEANQSTL ENFLERLKTI MDEKDSKCSS *
```

## FIG. 15

**A) Murine IL-4, native form, DNA sequence**

1 CATATCCACG GATGCGACAA AAATCACTTG AGAGAGATCA TCGGCATTTT GAACGAGGTC
61 ACAGGAGAAG GGACGCCATG CACGGAGATG GATGTGCCAA ACGTCCTCAC AGCAACGAAG
121 AACACCACAG AGAGTGAGCT CGTCTGTAGG GCTTCCAAGG TGCTTCGCAT ATTTTATTTA
181 AAACATGGGA AAACTCCATG CTTGAAGAAG AACTCTAGTG TTCTCATGGA GCTGCAGAGA
241 CTCTTTCGGG CTTTTCGATG CCTGGATTCA TCGATAAGCT GCACCATGAA TGAGTCCAAG
301 TCCACATCAC TGAAAGACTT CCTGGAAAGC CTAAAGAGCA TCATGCAAAT GGATTACTCG
361 **TAG**

**Murine IL-4, native form, translated protein sequence**

1 HIHGCDKNHL REIIGILNEV TGEGTPCTEM DVPNVLTATK NTTESELVCR ASKVLRIFYL
61 KHGKTPCLKK NSSVLMELQR LFRAFRCLDS SISCTMNESK STSLKDFLES LKSIMQMDYS
121 *

**B) Murine IL4, QY mutant construct, DNA sequence**

1 ATGGGTAGCA GCCATCATCA TCATCATCAC TCCAGCGGTC TGGTTCCTCG TGGTAGTCAT
61 ATGCACATTC ACGGGTGTGA CAAAAATCAT CTGCGCGAGA TTATCGGTAT TCTGAACGAA
121 GTGACCGGAG AAGGCACTCC TTGTACGGAA ATGGATGTCC CGAACGTCCT GACAGCGACG
181 AAAAACACAA CGGAATCGGA ACTGGTTTGC CGTGCCAGCA AAGTCCTGCG CATCTTCTAT
241 CTGAAACATG GTAAAACGCC GTGTCTGAAA AAAAACAGCA GCGTTCTGAT GGAACTGCAA
301 CGCCTGTTTC GTGCTTTCCG CTGCCTGGAT AGCAGTATCA GCTGTACGAT GAACGAGTCC
361 AAATCAACCT CCCTGAAAGA CTTCCTGGAA TCACTGAAAT CGATCATGGA TATGGATGAC
421 AGCTGA**TAA**

**Murine IL4, QY mutant construct, translated protein sequence**

1 MGSSHHHHHH SSGLVPRGSH MHIHGCDKNH LREIIGILNE VTGEGTPCTE MDVPNVLTAT
61 KNTTESELVC RASKVLRIFY LKHGKTPCLK KNSSVLMELQ RLFRAFRCLD SSISCTMNES
121 KSTSLKDFLE SLKSIMDMDD S*

## FIG. 16

**Human complement component 3 construct H5 (rhC3-H5)**

```
   1 SPMYSIITPN ILRLESEETM VLEAHDAQGD VPVTVTVHDF PGKKLVLSSE KTVLTPATNH
  61 MGNVTFTIPA NREFKSEKGR NKFVTVQATF GTQVVEKVVL VSLQSGYLFI QTDKTIYTPG
 121 STVLYRIFTV NHKLLPVGRT VMVNIENPEG IPVKQDSLSS QNQLGVLPLS WDIPELVNMG
 181 QWKIRAYYEN SPQQVFSTEF EVKEYVLPSF EVIVEPTEKF YYIYNEKGLE VTITARFLYG
 241 KKVEGTAFVI FGIQDGEQRI SLPESLKRIP IEDGSGEVVL SRKVLLDGVQ NLRAEDLVGK
 301 SLYVSATVIL HSGSDMVQAE RSGIPIVTSP YQIHFTKTPK YFKPGMPFDL MVFVTNPDGS
 361 PAYRVPVAVQ GEDTVQSLTQ GDGVAKLSIN THPSQKPLSI TVRTKKQELS EAEQATRTMQ
 421 ALPYSTVGNS NNYLHLSVLR TELRPGETLN VNFLLRMDRA HEAKIRYYTY LIMNKGRLLK
 481 AGRQVREPGQ DLVVLPLSIT TDFIPSFRLV AYYTLIGASG QREVVADSVW VDVKDSCVGS
 541 LVVKSGQSED RQPVPGQQMT LKIEGDHGAR VVLVAVDKGV FVLNKKNKLT QSKIWDVVEK
 601 ADIGCTPGSG KDYAGVFSDA GLTFTSSSGQ QTAQRAELQC PQPAASVQLT EKRMDKVGKY
 661 PKELRKCCED GMRENPMRFS CQRRTRFISL GEACKKVFLD CCNYITELRR QHARASHLGL
 721 ARSNLDEDII AEENIVSRSE FPESWLWNVE DLKEPPKNGI STKLMNIFLK DSITTWEILA
 781 VSMSDKKGIC VADPFEVTVM QDFFIDLRLP YSVVRNEQVE IRAVLYNYRQ NQELKVRVEL
 841 LHNPAFCSLA TTKRRHQQTV TIPPKSSLSV PYVIVPLKTG LQEVEVKAAV YHHFISDGVR
 901 KSLKVVPEGI RMNKTVAVRT LDPERLGREG VQKEDIPPAD LSDQVPDTES ETRILLQGTP
 961 VAQMTEDAVD AERLKHLIVT PSGCGEQNMI GMTPTVIAVH YLDETEQWEK FGLEKRQGAL
1021 ELIKKGYTQQ LAFRQPSSAF AAFVKRAPST WLTAYVVKVF SLAVNLIAID SQVLCGAVKW
1081 LILEKQKPDG VFQEDAPVIH QEMIGGLRNN NEKDMALTAF VLISLQEAKD ICEEQVNSLP
1141 GSITKAGDFL EANYMNLQRS YTVAIAGYAL AQMGRLKGPL LNKFLTTAKD KNRWEDPGKQ
1201 LYNVEATSYA LLALLQLKDF DFVPPVVRWL NEQRYYGGGY GSTQATFMVF QALAQYQKDA
1261 PDHQELNLDV SLQLPSRSSK ITHRIHWESA SLLRSEETKE NEGFTVTAEG KGQGTLSVVT
1321 MYHAKAKDQL TCNKFDLKVT IKPAPETEKR PQDAKNTMIL EICTRYLGEV DSTMTIIDIS
1381 MLTGFLPDAE DLTRLSKGVD RYISRYEVDN NMAQKVAVII YLNKVSHSED ECLHFKILKH
1441 FEVGFIQPGS VKVYSYYNLD EKCTKFYHPD KGTGLLNKIC IGNVCRCAGE TCSSLNHQER
1501 IDVPLQIEKA CETNVDYVYK TKLLRIEEQD GNDIYVMDVL EVIKQGTDEN PRAKTHQYIS
1561 QRKCQEALNL KVNDDYLIWG SRSDLLPTKD KISYIITKNT WIERWPHEDE CQEEEFQKLC
1621 DDFAQFSYTL TEFGCPT*
```

Europäisches Patentamt
European Patent Office
Office européen des brevets

# EUROPEAN SEARCH REPORT

Application Number
EP 12 07 5141

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TOMOKO SHINDO ET AL: "Effective induction of oral anaphylaxis to ovalbumin in mice sensitized by feeding of the antigen with aid of oil emulsion and salicylate", THE JOURNAL OF TOXICOLOGICAL SCIENCES, vol. 37, no. 2, 1 April 2012 (2012-04-01), pages 307-315, XP055060967, ISSN: 0388-1350, DOI: 10.2131/jts.37.307 * table 1 * ----- | 1-13 | INV. C12N15/113 A61K31/7125 A61K39/35 A61K9/00 A61P37/00 |
| A | P. JOHANSEN ET AL: "Toll-like receptor ligands as adjuvants in allergen-specific immunotherapy", CLINICAL & EXPERIMENTAL ALLERGY, vol. 35, no. 12, 2 December 2005 (2005-12-02), pages 1591-1598, XP055060999, ISSN: 0954-7894, DOI: 10.1111/j.1365-2222.2005.02384.x * figures 2,6; table 1 * ----- | 1-13 | |
| | | | TECHNICAL FIELDS SEARCHED (IPC) |
| A | JULIA M MARTÍNEZ GÓMEZ ET AL: "A Protective Allergy Vaccine Based on CpG and Protamine-Containing PLGA Microparticles", PHARMACEUTICAL RESEARCH, KLUWER ACADEMIC PUBLISHERS-PLENUM PUBLISHERS, NL, vol. 24, no. 10, 31 May 2007 (2007-05-31), pages 1927-1935, XP019532578, ISSN: 1573-904X, DOI: 10.1007/S11095-007-9318-0 * figures 5,6; table I * ----- | 1-13 | C12N A61K A61P |

-/--

~~The present search report has been drawn up for all claims~~

2

| Place of search | Date of completion of the search | Examiner |
|---|---|---|
| The Hague | 6 May 2013 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 07 5141

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | YOUNGMI KIM ET AL: "DNA methyl transferase I acts as a negative regulator of allergic skin inflammation", MOLECULAR IMMUNOLOGY, vol. 53, no. 1-2, 10 July 2012 (2012-07-10), pages 1-14, XP055060924, ISSN: 0161-5890, DOI: 10.1016/j.molimm.2012.06.010 * figure 9 * ----- | 1-13 | |
| X | LUTFI R ET AL: "Dendritic cell-derived tumor necrosis factor [alpha] modifies airway epithelial cell responses", JOURNAL OF INNATE IMMUNITY, S. KARGER AG, CH, vol. 4, no. 5-6, 17 April 2012 (2012-04-17), pages 542-552, XP008161686, ISSN: 1662-811X [retrieved on 2012-04-17] * figure 8 * ----- | 1-13 | TECHNICAL FIELDS SEARCHED (IPC) |
| A | GRANADO M ET AL: "Anti-tumor necrosis factor agent PEG-sTNFRI improves the growth hormone/insulin-like growth factor-I system in adjuvant-induced arthritic rats", EUROPEAN JOURNAL OF PHARMACOLOGY, ELSEVIER SCIENCE, NL, vol. 536, no. 1-2, 2 March 2006 (2006-03-02), pages 204-210, XP028029569, ISSN: 0014-2999, DOI: 10.1016/J.EJPHAR.2006.02.035 [retrieved on 2006-04-24] * the whole document * ----- | 1-13 | |
| | -/-- | | |

~~The present search report has been drawn up for all claims~~

2

| Place of search | Date of completion of the search | Examiner |
|---|---|---|
| The Hague | 6 May 2013 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 07 5141

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JULIANA DE SOUZA REBOUÇAS ET AL: "Nanoparticulate Adjuvants and Delivery Systems for Allergen Immunotherapy", JOURNAL OF BIOMEDICINE AND BIOTECHNOLOGY, vol. 156, no. 8, 26 February 2012 (2012-02-26), pages 2809-13, XP055061036, ISSN: 1110-7243, DOI: 10.1073/pnas.0509541102 * the whole document * ----- | 1-13 | |
| A | WO 02/48168 A1 (ISIS PHARMACEUTICALS INC [US]; BAKER BRENDA F [US]; COWSERT LEX M [US]) 20 June 2002 (2002-06-20) * claims 1,12,17; examples 17,18,21,22 * ----- | 1-13 | |
| A | WO 2007/137129 A2 (ALCON MFG LTD [US]; YANNI JOHN M [US]; CHATTERTON JON E [US]; SENCHYNA) 29 November 2007 (2007-11-29) * claims 12,19,21; example 1 * ----- | 1-13 | TECHNICAL FIELDS SEARCHED (IPC) |
| A | MASCARELL LAURENT ET AL: "Induction of allergen-specific tolerance via mucosal routes", CURRENT TOPICS IN MICROBIOLOGY AND IMMUNOLOGY; VACCINES AGAINST ALLERGIES, SPRINGER, BERLIN, DE, vol. 352/820, 12 May 2011 (2011-05-12), pages 85-105, XP008161703, ISSN: 0070-217X, DOI: 10.1007/82_2011_132 * page 96 - page 97 * ----- -/-- | 1-13 | |

~~The present search report has been drawn up for all claims~~

2

| Place of search | Date of completion of the search | Examiner |
|---|---|---|
| The Hague | 6 May 2013 | Bucka, Alexander |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

Europäisches Patentamt
European Patent Office
Office européen des brevets

# EUROPEAN SEARCH REPORT

Application Number
EP 12 07 5141

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | ANIL MAHAPATRO ET AL: "Biodegradable nanoparticles are excellent vehicle for site directed in-vivo delivery of drugs and vaccines", JOURNAL OF NANOBIOTECHNOLOGY, vol. 9, no. 1, 28 November 2011 (2011-11-28), page 55, XP055045680, ISSN: 1477-3155, DOI: 10.1186/1477-3155-9-55 * the whole document * ----- | 1-13 | |
| A | PANYAM J ET AL: "Biodegradable nanoparticles for drug and gene delivery to cells and tissue", ADVANCED DRUG DELIVERY REVIEWS, ELSEVIER BV, AMSTERDAM, NL, vol. 55, no. 3, 24 February 2003 (2003-02-24), pages 329-347, XP008096954, ISSN: 0169-409X, DOI: 10.1016/S0169-409X(02)00228-4 * the whole document * ----- | 1-13 | TECHNICAL FIELDS SEARCHED (IPC) |
| A,D | FILIP VAN HAUWERMEIREN ET AL: "Treatment of TNF mediated diseases by selective inhibition of soluble TNF or TNFR1", CYTOKINE AND GROWTH FACTOR REVIEWS, vol. 22, no. 5, 1 October 2011 (2011-10-01), pages 311-319, XP028337283, ISSN: 1359-6101, DOI: 10.1016/J.CYTOGFR.2011.09.004 [retrieved on 2011-09-14] * page 313 - page 314; table 2 * ----- | 1-13 | |

~~The present search report has been drawn up for all claims~~

2

| Place of search | Date of completion of the search | Examiner |
|---|---|---|
| The Hague | 6 May 2013 | Bucka, Alexander |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 12 07 5141

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  1-13(partially)

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 12 07 5141

The Search Division considers that the present European patent application does not comply with the requirements of unity of invention and relates to several inventions or groups of inventions, namely:

1. claims: 1-13(partially)

Pharmaceutical composition for modulation of T cell and B cell responses made of one or more preparations and comprising a therapeutically effective dose of at least one inhibitor of tumor necrosis factor receptor 1 (TNFR1)-mediated functions and at least one antigen or allergen, wherein the inhibitor of TNFR1-mediated functions is selected from TNFR1-specific antisense oligonucleotides, antagonistic TNF mutants with specificity for TNFR1, anti-TNFR1 aptamers, anti-TNFR1 antibodies and fragments thereof, and allow specific inhibition of TNFR1 while leaving TNFR2 signaling intact.

---

2. claims: 1-13(partially)

Pharmaceutical composition for modulation of T cell and B cell responses made of one or more preparations and comprising a therapeutically effective dose of at least one inhibitor of tumor necrosis factor receptor 1 (TNFR1)-mediated functions and at least one antigen or allergen, wherein the inhibitor of TNFR1-mediated functions is selected from glutathione precursors such as N-acetyl-L-cysteine, glutathione and derivatives thereof such as S-methylglutathione, and salicylates such as acetylsalicylic acid and sodium salicylate, and allow selective inhibition of TNFR1- mediated functions.

---

3. claims: 1-5, 7-13(all partially)

Pharmaceutical composition for modulation of T cell and B cell responses made of one or more preparations and comprising a therapeutically effective dose of at least one inhibitor of tumor necrosis factor receptor 1 (TNFR1)-mediated functions and at least one antigen or allergen, wherein the inhibitor of TNFR1-mediated functions is selected from dominant-negative sTNF mutants and allow specific inhibition of sTNF while leaving the mTNF-TNFR1 and mTNF-TNFR2 interactions intact.

---

## ANNEX TO THE EUROPEAN SEARCH REPORT ON EUROPEAN PATENT APPLICATION NO. EP 12 07 5141

This annex lists the patent family members relating to the patent documents cited in the above-mentioned European search report. The members are as contained in the European Patent Office EDP file on
The European Patent Office is in no way liable for these particulars which are merely given for the purpose of information.

06-05-2013

| Patent document cited in search report | Publication date | Patent family member(s) | Publication date |
|---|---|---|---|
| WO 0248168 A1 | 20-06-2002 | AU 4342902 A | 24-06-2002 |
| | | WO 0248168 A1 | 20-06-2002 |
| WO 2007137129 A2 | 29-11-2007 | AR 061064 A1 | 30-07-2008 |
| | | AU 2007253776 A1 | 29-11-2007 |
| | | CA 2650416 A1 | 29-11-2007 |
| | | CL 14342007 A1 | 22-02-2008 |
| | | CN 101448943 A | 03-06-2009 |
| | | EP 2018426 A2 | 28-01-2009 |
| | | ES 2413804 T3 | 17-07-2013 |
| | | JP 2009537566 A | 29-10-2009 |
| | | JP 2013049727 A | 14-03-2013 |
| | | JP 2013049728 A | 14-03-2013 |
| | | KR 20090010234 A | 29-01-2009 |
| | | KR 20120093306 A | 22-08-2012 |
| | | RU 2008150324 A | 27-06-2010 |
| | | TW 200812623 A | 16-03-2008 |
| | | US 2007281901 A1 | 06-12-2007 |
| | | US 2010166676 A1 | 01-07-2010 |
| | | US 2011142767 A1 | 16-06-2011 |
| | | UY 30361 A1 | 31-08-2007 |
| | | WO 2007137129 A2 | 29-11-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 12075059 A
- EP 11075261 A
- WO 2005066206 A1
- EP 1354893 B1
- EP 1785490 B1
- WO 9918142 A
- WO 02102309 A
- US 7553931 B


### Non-patent literature cited in the description


- *The Lenercept Multiple Sclerosis Study Group and The University of British Columbia MS/MRI Analysis Group,* 1999
- **ABBOTT et al.** *BMC Immunol.,* 2011, vol. 12, 72
- **ABDULAMIR et al.** *J. Investig. Allergol. Clin. Immunol.,* 2009, vol. 19, 218-224
- **ABE et al.** *J. Immunol.,* 2001, vol. 167, 4651-4660
- **ABRAMSON et al.** *Cochrane Database Syst. Rev.,* 2003
- **ADAMY et al.** *J. Mol. Cell. Cardiology,* 2007, vol. 43, 344-353
- **AKBAR et al.** *Immunology,* 2003, vol. 109, 319-325
- **AKDIS ; AKDIS.** *J. Allergy Clin. Immunol.,* 2011, vol. 127, 18-27
- **ALEXOPOULOU et al.** *Eur. J. Immunol.,* 2006, vol. 36, 2768-2780
- **ALHALAWEH et al.** *Eur. J. Pharm. Sci.,* 2009, vol. 38, 206-214
- **AMADOU et al.** *Am. J. Physiol.,* 2002, vol. 282, C1339-C1347
- **ANDERSEN.** *Nat. med.,* 2004, vol. 10, S18-S25
- **ARNTZ et al.** *Arthritis Res. Therapy,* 2010, vol. 12, 61
- **BARNES.** *Trends Pharmacol. Sci.,* 2010, vol. 31, 335-343
- **BAUTSCH et al.** *J. Immunol.,* 2000, vol. 165, 5401-5405
- **BITON et al.** *Joint Bone Spine,* 2012, vol. 79, 119-123
- **BLUESTONE et al.** *Nature,* vol. 464, 1293, 2010
- **BOURRAINDELOUP et al.** *Circulation,* 2004, vol. 110, 2003-2009
- **CAILLERET et al.** *Circulation,* 2004, vol. 109, 406-411
- **CHANG et al.** *Vaccine,* 2001, vol. 19, 2884-2889
- TNF pathophysiology. Molecular and cellular mechanisms. **CHEN ; OPPENHEIM.** Curr. Dir. Autoimmun. Basel, 2010, 119-134
- **CHEN et al.** *J. Immunl.,* 2007, vol. 179, 154-161
- **CHEN et al.** *J. Immunol.,* 2010, vol. 185, 174-182
- **CHOI et al.** *Pharmaceut. Res,* 2003, vol. 20, 2008-2010
- **CIANFERONI et al.** *Blood,* 2001, vol. 97, 1742-1749
- **CLAPP et al.** *J. Pharm. Sci.,* 2011, vol. 100, 388-401
- **COPPIETERS et al.** *J. Exp. Med.,* 2012, vol. 209, 51-60
- **DEFER et al.** *J. Biol. Chem.,* 2007, vol. 282, 35564-35573
- **DEMEDTS et al.** *N. Engl. J. Med.,* 2005, vol. 353, 2229-2242
- **DONG et al.** *J. Biol. Chem.,* 1997, vol. 272, 9962-9970
- **DROUIN et al.** *J. Immunol.,* 2001, vol. 167, 4141-4145
- **DROUIN et al.** *Am. J. Respir. Crit. Care Med.,* 2006, vol. 173, 852-857
- **EHRENSTEIN et al.** *J. Exp. Med.,* 2004, vol. 200, 277-285
- **EYLAR et al.** *Int. Immunol.,* 1993, vol. 1, 97-101
- **FAMULOK et al.** *Curr. Top. Microbiol. Immunol.,* 1999, vol. 243, 123-136
- **FARIA ; WEINER.** *Immunol. Rev.,* 2005, vol. 206, 232-259
- **FELDMANN et al.** *Nat. Med.,* 2003, vol. 9, 1245-1250
- **FURST et al.** *J. Rheumatol.,* 1987, vol. 14, 342-347

- **GARREN et al.** *Ann. Neurol.,* 2008, vol. 63, 611-620
- **GILBERT et al.** *J. Control. Release,* 1987, vol. 5, 113-118
- **GODEBU et al.** *J. Immunol.,* 2008, vol. 181, 1798-1805
- **GOHIL et al.** *Global J. Pharmacol.,* 2010, vol. 4, 19-30
- **GONG et al.** *Int. J. Pharm.,* 2009, vol. 365, 89-99
- **GONG et al.** *BMC Biotechnol.,* 2009, vol. 9, 8
- **GOVERMAN.** *Nature Rev. immunol.,* 2009, vol. 9, 393-407
- **GRINBERG-BLEYER et al.** *J. Clin. Invest.,* 2010, vol. 120, 4558-4568
- **HAGENAARS et al.** *J. Control. Release,* 2010, vol. 144, 17-24
- **HAMANO et al.** *Eur. J. Immunol.,* 2011, vol. 41, 2010-2020
- **HARTL et al.** *J. Allergy Clin. Immunol.,* 2007, vol. 119, 1258-1266
- **HAYAKAWA et al.** *EMBO J.,* 2003, vol. 22, 3356-3366
- **HEEGER ; KEMPER.** *Immunobiology,* 2012, vol. 217, 216-224
- **HELBLING-LECLERC et al.** *Biochim. Biophys. Acta,* 1999, vol. 1418, 165-175
- **HERING et al.** *Am. J. Transplant.,* 2004, vol. 4, 390-401
- **HEROLD et al.** *N. Engl. J. Med.,* 2002, vol. 346, 1692-1698
- **HIGUCHI et al.** *J. Rheumatol.,* 2000, vol. 27, 1038-1044
- **HOLGATE ; POLOSA.** *Nature Rev. Immunol,* 2008, vol. 8, 218-230
- **HOMANN ; VON HERRATH.** *Clin. Immunol.,* 2004, vol. 112, 202-209
- **HOUSLEY et al.** *J. Immunol.,* 2011, vol. 186, 6779-6787
- **HUANG et al.** *Clin. Cancer Res.,* 2006, vol. 12, 2849-2855
- **HUMBLES et al.** *Nature,* 2000, vol. 406, 998-1001
- **HYUN et al.** *Biomacromolecules,* 2007, vol. 8, 1093-1100
- **ICHIM et al.** *Expert Opin. Biol. Ther.,* 2008, vol. 8, 191-199
- **JACOBSEN et al.** *Allergy,* 2007, vol. 62, 943-948
- **JAECKEL et al.** *Horm. Metab. Res.,* 2008, vol. 40, 126-136
- **JEANNIN et al.** *J. Exp. Med.,* 1995, vol. 182, 1785-1792
- **JEONG et al.** *Nature,* 1997, vol. 388, 860-862
- **JUEDES ; VON HERRATH.** *Res. Rev.,* 2004, vol. 20, 446-451
- **JURYNCZYK et al.** *Ann. Neurol.,* 2010, vol. 68, 593-601
- **KAMPHUIS et al.** *Lancet,* 2005, vol. 366, 50-56
- **KANG et al.** *Biomaterials,* 2010, vol. 31, 2453-2460
- **KARAGIANNIDIS et al.** *J. Allergy Clin. Immunol.,* 2004, vol. 114, 1425-1433
- **KASSIOTIS ; KOLLIAS.** *J. Exp. Med.,* 2001, vol. 1993, 427-434
- **KAWAMOTO et al.** *J. Clin. Invest.,* 2004, vol. 114, 399-407
- **KEARLEY et al.** *J. Exp. Med.,* 2005, vol. 202, 1539-1547
- **KEARLEY et al.** *J. Allergy Clin. Immunol.,* 2008, vol. 122, 617-624
- **KELLY.** *Altern. Med. Rev.,* 1998, vol. 3, 114-127
- **KEYMEULEN.** *N. Engl. J. Med.,* 2005, vol. 352, 2598-2608
- **KIM et al.** *Biomaterials,* 2004, vol. 25, 305-313
- **KIM et al.** *J. Gene Med.,* 2009, vol. 11, 26-37
- **KLEIJWEGT et al.** *J. Immunol.,* 2010, vol. 185, 1412-1418
- **KNIP et al.** *Diabetes,* 2005, vol. 54
- **KÖHL et al.** *J. Clin. Invest,* 2006, vol. 116, 783-796
- **KOOP ; GHOSH.** *Science,* 1994, vol. 265, 956-959
- **KORN et al.** *Nature Med,* 2007, vol. 13, 423-431
- **KUKREJA et al.** *J. Clin. Invest,* 2002, vol. 109, 131-140
- **KWAN et al.** *Immunol. Res.,* 2012
- **LANDEWE et al.** *Ann. Rheum. Dis.,* 2010, vol. 69, 1655-1659
- **LANGIER et al.** *Israel Med. Assoc. J.,* 2012, vol. 14, 180-183
- **LARCHE et al.** *Nat. Rev. Immunol.,* 2006, vol. 6, 761-771
- **LEE et al.** *Clin. Exp. Immunol.,* 2007, vol. 148, 53-63
- **LEROUX-ROELS.** *Vaccine,* 2010, vol. 285, C25-C36
- **LI et al.** *Blood,* 2008, vol. 112, 5084-5094
- **LI et al.** *J. Mol. Cell Biol.,* 2012, vol. 4, 38-47
- **LIANG et al.** *J. Biol. Chem.,* 1989, vol. 264, 13519-13523
- **LIANG et al.** *J. Immunol.,* 1991, vol. 146, 1909-1913
- **LIN et al.** *Diabetes,* 2010, vol. 59, 2247-2252
- **LIU ; HANNUN.** *Biol. Chem.,* 1997, vol. 272, 16281-16287
- **LIU et al.** *J. Immunol.,* 2008, vol. 180, 5882-5889
- **LUDVIGSSON et al.** *N. Engl. J. Med.,* 2008, vol. 359, 676-781
- *MacroGenics,* 20 October 2011, http://www.macrogenics.com/press_releases-284.html

- **MANTEL et al.** *PLoS biology,* 2007, vol. 5, 2847-2861
- **MAZZEO et al.** *Eur. J. Immunol.,* 1998, vol. 28, 3205-3213
- **MCGEACHY et al.** *J. Immunol.,* 2005, vol. 175, 3025-3032
- **MCTIERNAN et al.** *Gene Ther.,* 2007, vol. 14 (23), 1613-22
- **MOLLER et al.** *J. Allergy Clin. Immunol,* 2002, vol. 109, 251-256
- **MUNEGOWDA et al.** *J. Clin. Immunol.,* 2011, vol. 31, 811-826
- **NADKARNI et al.** *J. Exp. Med.,* 2007, vol. 204, 33-39
- **NICHOLLS et al.** *Ann. N.Y. Acad. Sci.,* 2010, vol. 1213, 46-61
- **NIE et al.** *Internatl. J. Nanomed.,* 2011, vol. 6, 151-166
- **NOMURA et al.** *J. Control. Release,* 2011, vol. 149, 8-14
- **NOTLEY et al.** *J. Exp. Med.,* 2008, vol. 205, 2491-2497
- **O'CONNOR et al.** *J. Immunol.,* 2007, vol. 179, 958-966
- **ORAL et al.** *J. Biol. Chem.,* 1997, vol. 272, 4836-4842
- **PAI et al.** *Am. Assoc. Pharmac. Sci. J.,* 2009, vol. 11, 88-98
- **PENDYALA ; CREAVEN.** *Cancer Epidemiol. Biomarkers Prevention,* 1995, vol. 4, 245-251
- **PENG et al.** *J. Immunol.,* 2006, vol. 176, 3330-3341
- **PENG et al.** *Blood,* 2008, vol. 111, 2452-2461
- **PENG et al.** *Biomaterials,* 2010, vol. 31, 5227-5236
- **QIAO et al.** *Int. J. Pharm.,* 2005, vol. 294, 103-112
- **RAEDLER et al.** *Am. J. Transplant.,* 2009, vol. 9, 1784-1795
- **ROLLAND et al.** *Pharmacol. Ther.,* 2009, vol. 121, 273-284
- **ROSS et al.** *J. Liposome Res.,* 1998, vol. 8, 499-520
- **RUEL-GARIEPY ; LEROUX.** *Eur. J. Pharmaceutics Biopharmaceutics,* 2004, vol. 58, 409-426
- **SANDSTROM et al.** *J. Leukocyte Biol.,* 1994, vol. 55, 221-226
- **SCHMIDT-WEBER ; BLASER.** *Curr. Opin. Allergy Clin. Immunol.,* 2005, vol. 5, 525-530
- **SCHMIDT-WEBER ; BLASER.** *Inflamm. Allergy Drug Targets,* 2006, vol. 5, 15-21
- **SEN et al.** *Free Radical Biol. Med,* 1997, vol. 22, 1241-1257
- **SEN.** *Nutritional Biochem.,* 1997, vol. 8, 660-672
- **SHIBATA et al.** *J. Biol. Chem.,* 2008, vol. 283, 998-1007
- **SHIBATA et al.** *Biomaterials,* 2009, vol. 30, 6638-6647
- **SINHA et al.** *Int. J. Pharm.,* 2004, vol. 278, 1-23
- **SKYLER et al.** *Diabetes Care,* 2005, vol. 28, 1068-1076
- **SMYTH et al.** *Chest,* 2010, vol. 138, 905-912
- **STAAL et al.** *Proc. Natl. Acad. Sci. USA,* 1990, vol. 87, 9943-9947
- **STÄGER et al.** *Nat. Med.,* 2003, vol. 9, 1287-1292
- **STEED et al.** *Science,* 2003, vol. 301, 1895-1898
- **STEINMAN ; ZAMVIL.** *Ann. Neurol.,* 2010, vol. 68, 567-569
- **STRAINIC et al.** *Immunity,* 2008, vol. 28, 425-435
- **STRICKLAND et al.** *J. Exp. Med.,* 2006, vol. 203, 2649
- **SURESH et al.** *J. Immunol.,* 2003, vol. 170, 788-794
- **SUVANNAVEJH et al.** *Cell. Immunol,* 2000, vol. 205, 24-33
- The Lenercept Multiple Sclerosis Study Group and The University of British Columbia MS/MRI Analysis Group. *Neurology,* 1999, vol. 53, 457-465
- **THOMMESEN ; LAEGREID.** *J. Biochem. Mol. Biol.,* 2005, vol. 38, 281-289
- **THORBURN ; HANSBRO.** *Am. J. Respir. Cell Mol. Biol.,* 2010, vol. 43, 511-519
- **TIROUVANZIAM et al.** *Proc. Natl. Acad. Sci. USA,* 2006, vol. 103, 4628-4633
- **TSUBOI et al.** *Cytokine,* 1995, vol. 7, 372-379
- **VALENCIA et al.** *Blood,* 2006, vol. 108, 253-261
- **VAN HAUWERMEIREN et al.** *Cytokine Growth Factors Rev.,* 2011, vol. 22, 311-319
- **VAN MIERLO et al.** *J. Immunol.,* 2008, vol. 180, 2747-2751
- **VIEYRA et al.** *Am. J. Pathol.,* 2011, vol. 179, 766-774
- **VIGLIETTA et al.** *J. Exp. Med.,* 2004, vol. 199, 971-979
- **VON HAEHLING et al.** *Basic Res. Cardiol.,* 2004, vol. 99, 18-28
- **WALKER et al.** *J. Clin. Invest.,* 2003, vol. 112, 1437-1443
- **WARREN et al.** *Eur. J. Neurol.,* 2006, vol. 13, 887-895
- **WENZEL et al.** *Lancet,* 2007, vol. 370, 1422-1431
- **WENZEL et al.** *Am. J. Respir. Crit. Care Med.,* 2009, vol. 179, 549-558
- **WU et al.** *Biomaterials,* 2012, vol. 33, 2351-3260
- **YALCINDAG et al.** *J. Allergy Clin. Immunol.,* 2006, vol. 117, 1455-1461

- **YAN et al.** *Frontiers Biosci,* 2005, vol. 10, 1802-1827
- **YU ; MALEK.** *J. Immunol.,* 2006, vol. 177, 5115-5121
- **ZAHAROFF et al.** *Vaccine,* 2007, vol. 25, 2085-2094
- **ZALEVSKY et al.** *J. Immunol.,* 2007, vol. 179, 1872-1883
- **ZETTLITZ et al.** *MAbs,* 2010, vol. 2, 639-647
- **ZHANG et al.** *Biomacromolecules,* 2006, vol. 7, 2492-2500
- **ZHOU.** *Immunobiology,* 2012, vol. 217, 225-234
- **ZOZULYA ; WIENDL.** *Hum. Immunol.,* 2008, vol. 69, 797-804
- **BIDDISON.** *Curr. Protcols Cell Biol.,* 1998
- **EYLAR et al.** *Intern. Immunol.,* 1993, vol. 5, 97-101
- **IYER et al.** *J. Org. Chem.,* 1990, vol. 55, 4699-4704
- **OJWANG et al.** *Biochemistry,* 1997, vol. 36, 6033-6045
- **OJWANG et al.** *Antisense Nucleic Acid Drug Dev.,* 1997, vol. 7, 447-459
- **OJWANG ; RANDO.** *Methods : a companion to Meth Enzymol.,* 1999, vol. 18, 244-251
- **ROBINSON et al.** *Curr. Protcols Cytometry,* 1998
- **GRASSETTI ; MURRAY.** *Arch. Biochem. Biophys,* 1967, vol. 119, 41-49
- **KUKOC-MODUN ; RADIC.** *Intern. J. Analyt. Chem.,* 2011
- **WEI et al.** *Pharmaceutical Res.,* 2006, vol. 23, 1251-1264, www.jenway.com
- **OJWANG ; RANDO.** *Methods: a companion to Meth Enzymol.,* 1999, vol. 18, 244-251
- **YIAMOUYIANNIS et al.** *Am. J. Pathol.,* 1999, vol. 154, 1911-1921
- **AGUILAR-PIMENTEL et al.** *Am. J. Respir. Crit. Care Med.,* 2010, vol. 181, 7-16